# EUROPEAN PATENT SPECIFICATION

(11) **EP 4 001 298 B1**
(45) Date of publication and mention of the grant of the patent: **05.03.2025**
(21) Application number: 20843624.6
(22) Date of filing: 17.07.2020
(51) Int. Cl.: C07K 14/025, C12N 15/37, A61K 39/12, A61P 31/20, A61P 35/00, A61P 15/00

(54) **POLYVALENT IMMUNOGENICITY COMPOSITION FOR HUMAN PAPILLOMAVIRUS**
POLYVALENTE IMMUNOGENITÄTSZUSAMMENSETZUNG FÜR HUMANES PAPILLOMAVIRUS
COMPOSITION POLYVALENTE INDUISANT L'IMMUNOGÉNICITÉ POUR PAPILLOMAVIRUS HUMAIN

(30) Priority: 19.07.2019 CN 201910657255
(43) Date of publication of application: 25.05.2022
(73) Proprietor: Sinocelltech Ltd, Economic and Technological Development Zone Beijing 100176 (CN)
(72) Inventor: XIE, Liangzhi, Beijing 100176 (CN); LUO, Chunxia, Beijing 100176 (CN); ZHANG, Wei, Beijing 100176 (CN); SUO, Xiaoyan, Beijing 100176 (CN); PANG, Lin, Beijing 100176 (CN); HU, Ping, Beijing 100176 (CN)
(74) Representative: Dolphin, Kirsty Mairi
(86) International application number: PCT/CN2020/102601
(87) International publication number: WO 2021/013071

(56) References cited:
- WO-A1-01/97840
- CN-A- 101 518 647
- CN-A- 102 268 076
- CN-A- 102 747 047
- CN-A- 106 795 518
- US-A- 5 821 087
- LUXEMBOURG ALAIN ET AL: "Phase II studies to select the formulation of a multivalent HPV L1 virus-like particle (VLP) vaccine", HUMAN VACCINES & IMMUNOTHERAPEUTICS, vol. 11, no. 6, 27 April 2015 (2015-04-27), US, pages 1313 - 1322, XP093007309, ISSN: 2164-5515, DOI: 10.1080/21645515.2015.1012010
- ZHANG T ET AL: "Trivalent Human Papillomavirus (HPV) VLP vaccine covering HPV type 58 can elicit high level of humoral immunity but also induce immune interference among component types", VACCINE, ELSEVIER, AMSTERDAM, NL, vol. 28, no. 19, 26 April 2010 (2010-04-26), pages 3479 - 3487, XP026997130, ISSN: 0264-410X, [retrieved on 20100306]
- NEIL D CHRISTENSEN ET AL: "Hybrid papillomavirus L1 molecules assemble into virus-like particles that reconstitute conformational epitopes and induce neutralizing antibodies to distinct HPV types", VIROLOGY, ELSEVIER, AMSTERDAM, NL, vol. 291, no. 2, 20 December 2001 (2001-12-20), pages 324 - 334, XP002280750, ISSN: 0042-6822, DOI: 10.1006/VIRO.2001.1220
- MATHIEU BOXUS ET AL: "Broad Cross-Protection Is Induced in Preclinical Models by a Human Papillomavirus Vaccine Composed of Ll/L2 Chimeric Virus-Like Particles", JOURNAL OF VIROLOGY, vol. 90, no. 14, 15 July 2016 (2016-07-15), US, pages 6314 - 6325, XP055422243, ISSN: 0022-538X, DOI: 10.1128/JVI.00449-16
- WANG DANING ET AL: "Rational design of a multi-valent human papillomavirus vaccine by capsomere-hybrid co-assembly of virus-like particles", NATURE COMMUNICATIONS, vol. 11, no. 1, 5 June 2020 (2020-06-05), XP055919924, Retrieved from the Internet <URL:http://www.nature.com/articles/s41467-020-16639-1> DOI: 10.1038/s41467-020-16639-1

## Description

### TECHNICAL FIELD

The present invention relates to a multivalent immunogenic composition for preventing human papillomavirus (HPV) related diseases or infections and uses thereof.

### BACKGROUND

Papilloma virus (PV) belongs to the *Papillomaviridae* Family and causes papillomas in humans, cattle, dogs, and rabbits. One of its members, human papilloma virus (HPV), is a non-enveloped DNA virus. The genome of this virus is a double-stranded closed circular DNA, about 7.2-8kb in size, with 8 open reading frames, which can be divided into three regions according to their functions: (1) early region (E), about 4.5kb, encoding E1, E2, E4-E7, a total of 6 non-structural proteins related to viral replication, transcription and transformation; (2) late region (L), about 2.5kb, encoding the major capsid protein L1 and the minor capsid protein L2; (3) long regulatory region (LCR), which is located between the end of the L region and the beginning of the E region, is about 800-900 bp long and does not encode any protein, serving as DNA replication and expression regulatory elements.

The L1 proteins and the L2 proteins are synthesized late in the HPV infection cycle. The L1 protein is the major capsid protein and has a molecular weight of 55-60 kDa. The L2 protein is the minor capsid protein. 72 L1 protein pentamers form the outer shell of the icosahedral HPV particle (45-55 nm in diameter), which encloses closed circular double-stranded DNA. The L2 protein is located on the inner side of the L1 protein (Structure of Small Virus-like Particles Assembled from the L1 Protein of Human Papillomavirus 16 Chen, X.S., R. L.Garcea, Mol.Cell.5(3):557-567, 2000).

The ORF of the L1 protein, the most conserved gene in the PV genome, can be used to identify new PV types. A new PV type is identified if its complete genome is cloned and its L1 ORF DNA sequence differs from the closest known PV type by more than 10%. Homologies with differences between 2% and 10% are defined as different subtypes, and differences of less than 2% are defined as different variants of the same subtype (E.-M. de Villiers et al. / Virology 324 (2004) 17-27).

At late stages of HPV infection, newly synthesized L1 proteins in the cytoplasm are transported to the nucleus of terminally differentiated keratin where, together with L2 proteins, package the replicated HPV genomic DNA to form infectious viruses (Nelson, L.M, et al. 2002. Nuclear import strategies of high risk HPV16 L1 major capsid protein. J. Biol. Chem. 277: 23958-23964). This suggests that nuclear import of the L1 protein plays a very important role in HPV infection and production. The ability of the virus to enter the nucleus is determined by the nuclear localization signal (NLS) at the C-terminus of the HPV L1 protein, the NLS is characterized by its abundance of basic amino acids (Garcia-Bustos, J., et al. 1991. Nuclear protein localization. Biochimica et Biophysica Acta 1071: 83-101).

15 high-risk (HR) HPV types can lead to cancers of cervix, anus, penis, vagina, vulva and oropharynx, among which HPV-16 and HPV-18 are by far the most common causes of cancers, accounting for approximately 70% of cervical cancers, and the other HR-HPV types (Types 31, 33, 35, 39, 45, 51, 52, 56, 58, 59, 68, 73, and 82) cause the rest. HPV-16 accounts for approximately 95% of HPV-positive oropharyngeal cancers (OPCs). The persistent low-risk genotypes HPV-6 and HPV-11 cause most anogenital warts and respiratory papillomas, but are rarely associated with cancers (Human Papillomavirus in Cervical Cancer and Oropharyngeal Cancer: One Cause, Two Diseases Tara A. Bermanand John T. Schiller, PhD2 Cancer 2017;123:2219-29).

The L1 protein can be recombinantly expressed by poxvirus, baculovirus, or yeast systems and then self-assembles to form virus-like particles (VLP) containing approximately 72 L1 proteins, similar to the virus capsid. VLP has no indication. VLP induces neutralizing antibodies in inoculated animals and protects experimental animals from subsequent attack by infectious viruses. Thus, VLP appears to be an excellent candidate for papilloma virus vaccines (Structure of Small Virus-like Particles Assembled from the L1 Protein of Human Papillomavirus 16 Chen, X.S. , R. L. Garcea, Mol. Cell. 5(3):557-567, 2000).

Glaxo's CERVARIX^{®}, a bivalent recombinant HPV vaccine, contains HPV Type 16 recombinant L1 protein and HPV Type 18 recombinant L1 protein. The L1 protein is obtained by expression of a recombinant baculovirus expression vector system in insect cells of the nocturnal moth (*Trichoplusia ni*)*.* The L1 protein self-assembles into virus-like particles for the prevention of cervical cancer, Grade 2 or 3 cervical intraepithelial neoplasia and adenocarcinoma in situ caused by HPV Types 16 and 18, and Grade 1 cervical intraepithelial neoplasia (oncogenic) in women aged 9-25 years (https://www.fda.gov/downloads/BiologicsBloodVaccines/Vaccines/ApprovedP roducts/UCM186981.pdf).

GARDASIL^{®} is a human papilloma virus quadrivalent (Types 6, 11, 16 and 18) recombinant vaccine from Merck for the prevention of cervical cancer, genital warts (condyloma acuminata) and precancerous or proliferative abnormal lesions caused by HPV Types 6, 11, 16 and 18 in girls and women aged 9-26 years; and for the prevention of anal cancer, genital warts (condyloma acuminatum) and pre-cancerous or abnormal developmental lesions caused by HPV Types 6, 11, 16, and 18 in boys and men aged 9-26 (https://www.fda.gov/vaccines-blood-biologics/vaccines/gardasil).

GARDASIL^{®}9 is a nine-valent recombinant human papilloma virus vaccine from Merck that contains virus-like particles of L1 proteins of HPV Types 6, 11, 16, 18, 31, 33, 45, 52 and 58, the L1 protein is produced by fermentation of *Saccharomyces cerevisiae* and self-assembles into VLP. It is used in girls and women aged 9-45 years for the prevention of cervical cancer, vulvar cancer, vaginal cancer and anal cancer caused by HPV Types 16, 18, 31, 33, 45, 52 and 58, genital warts (condyloma acuminata) caused by HPV Types 6 and 11, and precancerous or proliferative abnormalities caused by HPV Types 6, 11, 16, 18, 31, 33, 45, 52 and 58; and in boys and men aged 9-45 years for the prevention of anal cancer caused by HPV Types 16, 18, 31, 33, 45, 52 and 58, genital warts (condyloma acuminatum) caused by HPV Types 6 and 11 and pre-cancerous or developmentally abnormal lesions caused by HPV Types 6, 11, 16, 18, 31, 33, 45, 52 and 58 (https://www.fda.gov/vaccines-blood-biologics/vaccines /gardasil-9).

The instruction for GARDASIL^{®}9 announced that HPV Types 16 and 18 are the cause of about 70% of cervical cancers, with the remaining 20% of cases attributed to Types 31, 33, 45, 52 and 58, therefor GARDASIL^{®}9 prevents 90% of cervical cancers (https://www.fda.gov/ BiologicsBloodVaccines/Vaccines/ApprovedProducts/ucm426445.htm).

Industrial production of virus-like particles is critical to HPV vaccine development. The common systems for producing virus-like particles are mainly classified into the eukaryotic expression system and the prokaryotic expression system.

Commonly used eukaryotic expression systems include poxvirus expression systems, insect baculovirus expression systems, and yeast expression systems. The HPV L1 protein expressed in eukaryotic expression systems can be spontaneously assembled to virus-like particles as its natural conformation is less disrupted, but the yield thereof is low. The HPV L1 protein expressed in prokaryotic expression systems, mainly E. coli expression systems, is of high yields but mostly in the form of inclusion bodies, this form of protein cannot be easily purified thus makes the production process complicated.

Therefore, there is still a need to obtain high yields of HPV virus-like particles, so as to obtain HPV multivalent vaccines for broad-spectrum prevention of HPV-related diseases or infections, including those caused by the HPV types that are not currently covered by commercial available vaccines. Luxembourg, A. et al. (2015) Human Vaccines & immunotherapies, vol.11, no. 6, 1313-1322 and WO 01/97840 disclose HPV immunogenic compositions.

### SUMMARY

The present invention provides an HPV immunogenic composition for use in preventing HPV-related diseases or infections, comprising chimeric HPV L1 protein of HPV types 6, 11, 16, 18, 31, 35, 39, 45, 51, 52 , 56 and 58, HPV Type 33 L1 protein and HPV Type 59 L1 protein, wherein said chimeric HPV L1 protein of HPV Types 6, 11, 16, 18, 31, 35, 39, 45, 51, 52, 56 and 58 is shown in SEQ ID No: 3, SEQ ID No: 16, SEQ ID No: 29, SEQ ID No: 42, SEQ ID No: 55, SEQ ID No: 71, SEQ ID No: 84, SEQ ID No: 97, SEQ ID No: 110, SEQ ID No: 123, SEQ ID No: 136 and SEQ ID No: 149, respectively, and the HPV Type 33 L1 protein and HPV Type 59 L1 protein is shown in SEQ ID No: 66 and SEQ ID No: 160, respectively.

### BRIEF DESCRIPTION OF THE DRAWINGS

**Figure 1A****:** Expression of L1 protein of HPV 6 L1: 33C. M: Marker; L: cell lysate; E-S: supernatant collected after centrifugation of the lysate.
**Figure 1B****:** Expression of L1 protein of HPV 11 L1: 33C. M: Marker; L: cell lysate; E-S: supernatant collected after centrifugation of the lysate.
**Figure 1C****:** Expression of L1 protein of HPV 16 L1: 33C. M: Marker; L: cell lysate; E-S: supernatant collected after centrifugation of the lysate.
**Figure 1D****:** Expression of L1 protein of HPV 18 L1: 33C. M: Marker; L: cell lysate; E-S: supernatant collected after centrifugation of the lysate.
**Figure 1E****:** Expression of L1 protein of HPV 31 L1: 33C. M: Marker; L: cell lysate; E-S: supernatant collected after centrifugation of the lysate.
**Figure 1F****:** Expression of L1 protein of HPV 33 L1. M: Marker; L: cell lysate; E-S: supernatant collected after centrifugation of the lysate.
**Figure 1G****:** Expression of L1 protein of HPV 35 L1: 33C. M: Marker; L: cell lysate; E-S: supernatant collected after centrifugation of the lysate.
**Figure 1H****:** Expression of L1 protein of HPV 39 L1: 59C. M: Marker; L: cell lysate; E-S: supernatant collected after centrifugation of the lysate.
**Figure 1I****:** Expression of L1 protein of HPV 45 L1: 33C. M: Marker; L: cell lysate; E-S: supernatant collected after centrifugation of the lysate.
**Figure 1J****:** Expression of L1 protein of HPV 51 L1: 33C. M: Marker; L: cell lysate; E-S: supernatant collected after centrifugation of the lysate.
**Figure 1K****:** Expression of L1 protein of HPV 52 L1: 33C. M: Marker; L: cell lysate; E-S: supernatant collected after centrifugation of the lysate.
**Figure 1L****:** Expression of L1 protein of HPV 56 L1: 33C. M: Marker; L: cell lysate; E-S: supernatant collected after centrifugation of the lysate.
**Figure 1M****:** Expression of L1 protein of HPV 58 L1: 33C. M: Marker; L: cell lysate; E-S: supernatant collected after centrifugation of the lysate.
**Figure 1N****:** Expression of L1 protein of HPV 59 L1. M: Marker; L: cell lysate; E-S: supernatant collected after centrifugation of the lysate.
**Figure 2A****:** Transmission electron microscopy of HPV 6 L1: 33C virus-like particles.
**Figure 2B****:** Transmission electron microscopy of HPV 11 L1: 33C virus-like particles.
**Figure 2C****:** Transmission electron microscopy of HPV 16 L1: 33C virus-like particles.
**Figure 2D****:** Transmission electron microscopy of HPV 18 L1: 33C virus-like particles.
**Figure 2E****:** Transmission electron microscopy of HPV 31 L1: 33C virus-like particles.
**Figure 2F****:** Transmission electron microscopy of HPV 33 L1 virus-like particles.
**Figure 2G****:** Transmission electron microscopy of HPV 35 L1: 33C virus-like particles.
**Figure 2H****:** Transmission electron microscopy of HPV 39 L1: 59C virus-like particles.
**Figure 2I****:** Transmission electron microscopy of HPV 45 L1: 33C virus-like particles.
**Figure 2J****:** Transmission electron microscopy of HPV 51 L1: 33C virus-like particles.
**Figure 2K****:** Transmission electron microscopy of HPV 52 L1: 33C virus-like particles.
**Figure 2L****:** Transmission electron microscopy of HPV 56 L1: 33C virus-like particles.
**Figure 2M****:** Transmission electron microscopy of HPV 58 L1: 33C virus-like particles.
**Figure 2N****:** Transmission electron microscopy of HPV 59 L1 virus-like particles.
**Figure 3****:** The pseudovirus neutralizing titers in mice immunized with compositions of 14 HPV types of virus-like particles. Number of mice, N=10. GMT: Geometric Mean Titer.
**Figure 4****:** Expression of C-terminal truncated HPV 16L1 (1-474). M: Marker; L: cell lysates; E-S: supernatant collected after centrifugation of the lysates.

**DETAILED DESCRIPTION** The invention is defined by the appended claims.

In one aspect, the present disclosure provides a multivalent immunogenic composition for preventing papillomavirus-related diseases or infections. In one aspect of the disclosure, the papillomavirus may be human papillomavirus. In another aspect of the disclosure, the papillomavirus may be canine papillomavirus or rabbit papillomavirus.

In one aspect of the disclosure, there is provided a multivalent HPV immunogenic composition for use in preventing HPV-related diseases or infections, comprising: HPV virus-like particles assembled from L1 proteins of HPV Types 6, 11, 16, 18, 31, 33, 45, 52, and 58; and one or more HPV virus-like particles assembled from L1 proteins of other pathogenic HPV types.

In one aspect of the disclosure, said L1 protein of each HPV type can be a naturally occurring L1 protein, or a non-naturally occurring L1 protein, or a chimeric HPV L1 protein. In one embodiment, said HPV virus-like particles can be assembled from a single type of HPV L1 protein to form monovalent HPV virus-like particles, or can be assembled from multiple types of HPV L1 proteins to form multivalent HPV virus-like particles.

In one aspect of the disclosure, said one or more other pathogenic HPV types are selected from HPV Types 35, 39, 51, 56, and 59.

In one aspect of the disclosure, at least one of said HPV virus-like particle is a chimeric HPV virus-like particle, said chimeric HPV virus-like particle comprises one or more chimeric HPV L1 proteins; said chimeric HPV L1 protein comprises, from its N-terminus to C-terminus orientation: a. an N-terminal fragment derived from L1 protein of the first papilloma virus type, wherein said L1 protein of the first papilloma virus type is selected from HPV Types 6, 11, 16, 18, 31, 35, 39, 45, 51, 52, 56, or 58, said N-terminal fragment maintains the immunogenicity of the L1 protein of the corresponding HPV type; and b. a C-terminal fragment derived from the L1 protein of the second papilloma virus type, said L1 protein of the second papilloma virus type has a better expression level and solubility compared to L1 proteins of other types; wherein the chimeric L1 protein of HPV Types 6, 11, 16, 18, 31, 35, 39, 45, 51, 52, 56, or 58 has the immunogenicity of the L1 protein of the corresponding HPV type.

In one aspect of the disclosure, said chimeric HPV virus-like particle may be assembled from a single type of chimeric HPV L1 proteins to form monovalent HPV virus-like particles, or may be assembled from multiple types of chimeric HPV L1 proteins to form multivalent HPV virus-like particles.

In one aspect of the disclosure, said C-terminal fragment and N-terminal fragment may be freely combined as needed. In one embodiment, the chimeric HPV L1 protein may comprise one or plurality of C-terminal fragments. Said plurality of C-terminal fragments may be the same or different.

In one aspect of the disclosure, said N-terminal fragment is a fragment obtained by truncating the C-terminus of the natural sequence of said L1 protein of the first papilloma virus type at any amino acid position within its α5 region, and a fragment having at least 98% identity therewith; said C-terminal fragment is a fragment obtained by truncating the N-terminus of the natural sequence of the L1 protein of the second papilloma virus type at any amino acid position within its α5 region and functional variants resulting from further mutations, deletions and/or additions to the fragment.

In another aspect of the disclosure, said N-terminal fragment has at least 98.5%, 99%, 99.5% or 100% identity to a fragment obtained by truncating the C-terminus of the natural sequence of said L1 protein of the first papilloma virus type at any amino acid position within its α5 region.

In one aspect of the disclosure, said C-terminal fragment comprises one or plurality of nuclear localization sequences.

In one aspect of the disclosure, said L1 protein of the second papilloma virus type is selected from L1 proteins of HPV Types 1, 2, 3, 4, 6, 7, 10, 11, 13, 16, 18, 22, 26, 28, 31, 32, 33, 35, 39, 42, 44, 45, 51, 52, 53, 56, 58, 59, 60, 63, 66, 68, 73 or 82.

Preferably, said L1 protein of the second papilloma virus type is selected from L1 proteins of HPV Types 16, 28, 33, 59, or 68.

More preferably, said L1 protein of the second papilloma virus type is selected from L1 protein of HPV Type 33 or HPV Type 59.

In one aspect of the disclosure, said L1 protein of the second papilloma virus type is an HPV Type 33 L1 protein, said C-terminal fragment is SEQ ID No: 2; or a fragment having a length of m1 amino acids, preferably a fragment covering amino acids 1-m1 position of SEQ ID No: 2; wherein m1 is an integer of 8-26; or said C-terminal fragment is SEQ ID No: 135; or a fragment having a length of m2 amino acids, preferably a fragment covering amino acids 1-m2 position of SEQ ID No: 135; wherein m2 is an integer of 13-31.

The C-terminal fragment of the HPV Type 33 L1 protein has a nuclear localization sequence or the C-terminal fragment of the HPV Type 33 L1 protein has two nuclear localization sequences. The amino acid sequences with amino acid No: 7-8 (KR) and the amino acid sequences amino with amino acid No: 20-23 (KRKK) of SEQ ID No: 2 are nuclear localization sequences of the C-terminal fragments of the HPV Type 33 L1 protein.

In one aspect of the disclosure, said L1 protein of the second papilloma virus type is the HPV Type 59 L1 protein, said C-terminal fragment is SEQ ID No: 13; or a fragment having a length of n amino acids, preferably covering amino acids 1-n position of SEQ ID No: 13; wherein n is an integer of 16-38.

The C-terminal fragment of the HPV Type 59 L1 protein has a nuclear localization sequence or the C-terminal fragment of the HPV Type 59 L1 protein has two nuclear localization sequences. The chimeric HPV L1 protein comprises one or plurality of C-terminal fragments of the HPV Type 59 L1 protein. Said plurality of C-terminal fragments of the HPV Type 59 L1 protein may be the same or different. The amino acid sequence (RKR) of the 14^{th} to 16^{th} position of SEQ ID No: 13 and the amino acid sequence (KRVKRRK) of the 28^{th} to 34^{th} position of SEQ ID No: 13 are the nuclear localization sequences of the C-terminal fragment of the HPV Type 59 L1 protein.

In one aspect of the disclosure, said chimeric HPV L1 protein comprises both a C-terminal fragment of the HPV Type 33 L1 protein and a C-terminal fragment of the HPV Type 59 L1 protein.

In one aspect of the disclosure, said N-terminal fragment of said HPV Type 6 L1 protein has 98%, 98.5%, 99%, 99.5%, 99%, or 100% identity to the fragment obtained by truncating the C-terminus of the sequence shown in SEQ ID No: 1 at any amino acid site within its alpha 5 region.

In one aspect of the disclosure said N-terminal fragment of said HPV Type 11 L1 protein has 98%, 98.5%, 99%, 99.5%, 99% or 100% identity to the fragment obtained by truncating the C-terminus of the sequence shown in SEQ ID No: 14 at any amino acid site within its alpha 5 region.

In one aspect of the disclosure said N-terminal fragment of said HPV Type 16 L1 protein has 98%, 98.5%, 99%, 99.5%, 99% or 100% identity to the fragment obtained by truncating the C-terminus of the sequence shown in SEQ ID No: 27 at any amino acid site within its alpha 5 region.

In one aspect of the disclosure said N-terminal fragment of said HPV Type 18 L1 protein has 98%, 98.5%, 99%, 99.5%, 99% or 100% identity to the fragment obtained by truncating the C-terminus of the sequence shown in SEQ ID No: 40 at any amino acid site within its alpha 5 region.

In one aspect of the disclosure said N-terminal fragment of said HPV Type 31 L1 protein has 98%, 98.5%, 99%, 99.5%, 99% or 100% identity to the fragment obtained by truncating the C-terminus of the sequence shown in SEQ ID No: 53 at any amino acid site within its alpha 5 region.

In one aspect of the disclosure said N-terminal fragment of said HPV Type 35 L1 protein has 98%, 98.5%, 99%, 99.5%, 99% or 100% identity to the fragment obtained by truncating the C-terminus of the sequence shown in SEQ ID No: 69 at any amino acid site within its alpha 5 region.

In one aspect of the disclosure said N-terminal fragment of said HPV Type 39 L1 protein has 98%, 98.5%, 99%, 99.5%, 99% or 100% identity to the fragment obtained by truncating the C-terminus of the sequence shown in SEQ ID No: 82 at any amino acid site within its alpha 5 region.

In one aspect of the disclosure said N-terminal fragment of said HPV Type 45 L1 protein has 98%, 98.5%, 99%, 99.5%, 99% or 100% identity to the fragment obtained by truncating the C-terminus of the sequence shown in SEQ ID No: 95 at any amino acid site within its alpha 5 region.

In one aspect of the disclosure said N-terminal fragment of said HPV Type 51 L1 protein has 98%, 98.5%, 99%, 99.5%, 99% or 100% identity to the fragment obtained by truncating the C-terminus of the sequence shown in SEQ ID No: 108 at any amino acid site within its alpha 5 region.

In one aspect of the disclosure said N-terminal fragment of said HPV Type 52 L1 protein has 98%, 98.5%, 99%, 99.5%, 99% or 100% identity to the fragment obtained by truncating the C-terminus of the sequence shown in SEQ ID No: 121 at any amino acid site within its alpha 5 region.

In one aspect of the disclosure said N-terminal fragment of said HPV Type 56 L1 protein has 98%, 98.5%, 99%, 99.5%, 99% or 100% identity to the fragment obtained by truncating the C-terminus of the sequence shown in SEQ ID No: 134 at any amino acid site within its alpha 5 region; and

In one aspect of the disclosure said N-terminal fragment of said HPV Type 58 L1 protein has 98%, 98.5%, 99%, 99.5%, 99% or 100% identity to the fragment obtained by truncating the C-terminus of the sequence shown in SEQ ID No: 147 at any amino acid site within its alpha 5 region.

The C-terminus of said N-terminal fragment can be connected to the N-terminus of said C-terminal fragment directly or via a linker.

The linker does not affect the immunogenicity of said N-terminal fragment and does not affect the expression level or solubility of the protein. In one embodiment, said N-terminal fragment and said C-terminal fragment are connected via a linker comprising 1, 2, 3, 4, 5, 6, 7, 8, 9 or 10 amino acids. In one embodiment, the linker is an artificial sequence or, the linker is a naturally occurring sequence in the HPV L1 protein. The linker may be a partial sequence of the HPV Type 33 L1 protein or, the linker may be a partial sequence of the HPV Type 59 L1 protein.

In one aspect, within the range of plus or minus 4 amino acid positions of the connection point when the C-terminus of said N-terminal fragment is connected to the N-terminus of said C-terminal fragment, there presents the following continuous amino acid sequence: RKFL; preferably, within the range of plus or minus 6 amino acid positions of the connection point, there presents the following continuous amino acid sequence: LGRKFL.

In the context of the invention, chimeric HPV L1 proteins of chimeric HPV Types 6, 11, 16, 18, 31, 35, 39, 45, 51, 52, 56, and 58 as shown in SEQ ID No: 3, SEQ ID No: 16, SEQ ID No: 29, SEQ ID No: 42, SEQ ID No: 55, SEQ ID No: 71, SEQ ID No: 84, SEQ ID No: 97, SEQ ID No: 110, SEQ ID No: 123, SEQ ID No: 136 and SEQ ID No: 149, respectively; and the HPV Type 33 L1 protein and the HPV Type 59 L1 protein as shown in SEQ ID No: 66 and SEQ ID No: 160, respectively are for use in preventing HPV-related diseases or infections.

In one aspect at least one of said HPV virus-like particles is composed of a single type of chimeric HPV L1 protein, preferably is composed of said single type of chimeric HPV L1 protein having the same amino acid sequence.

In one aspect the chimeric HPV virus-like particle is the icosahedron comprising 72 pentamers of said chimeric HPV L1 protein. In one embodiment, the HPV virus-like particle has correctly formed disulfide bonds and thus has a good natural conformation. In one aspect, the chimeric HPV virus-like particles self-assemble in an *in vivo* expression system.

In one embodiment, said multivalent HPV immunogenic composition further comprises a physiologically acceptable carrier and, optionally, an adjuvant. In one embodiment, the adjuvant comprises one or more of aluminum salt, lipid A derivative, and ISCOM.

In one embodiment, said adjuvant is an aluminum phosphate adjuvant.

In one embodiment, the present invention provides the multivalent HPV immunogenic composition as defined in the claims for use in preventing HPV-related diseases or infections.

Papillomavirus L1 proteins expressed by eukaryotic expression systems can spontaneously assemble to virus-like particles, but the low expression level makes them not suitable for mass production.

The sequences of the L1 proteins of each HPV type can be easily obtained from https://www.uniprot.org. For a given HPV type, the L1 protein can be derived from different strains, thus the amino acid sequence thereof may have multiple versions, any version of the natural sequence can be used in the present invention. It is possible that the sequence of the HPV L1 protein of a given type used during the conception and design of the present invention may differ from the sequence used in the following examples, but such differences do not affect the decisions and conclusions of the inventors.

It is generally accepted by those of skill in the art that the C-terminus of the L1 protein does not contain major neutralizing antigenic epitope and therefore attempts have been made to increase expression by truncating the C-terminus of the HPV L1 protein, for example Glaxo's US patent US6361778B1, in which the C-terminus of the HPV16 L1 protein is truncated by 1-34 amino acids, preferably 26 amino acids, states that the yield of VLP is increased by many-fold, preferably at least by 10 folds, and in particular by about 10 to 100 folds. Inspired by this, the inventors attempted to truncate the C-terminus of HPV 16 L1 protein by 31 amino acids and named the truncated protein as HPV 16 L1 (1-474). The protein is in high expression level but poor solubility, and is difficult to be extract and purified (see Comparative Example).

The poor solubility of the protein due to this truncation may be caused by the deletion of the nuclear localization sequence located at the C-terminus, but the present invention is not binding to this speculation. During research and production, the inventor discovered that HPV Type 16 L1 protein, HPV Type 28 L1 protein, HPV Type 33 L1 protein, HPV Type 59 L1 protein and HPV Type 68 L1 protein have better expression levels and solubility than other HPV types L1 proteins. Inspired by the discovery, the inventors replaced the C-terminus of specific HPV types L1 proteins that are less extractable or less soluble with the C-terminus of specific HPV types L1 protein having better expression levels and solubility. That is, the inventors have constructed a chimeric protein: comprising, in the N-terminus to C-terminus orientation, an N-terminal fragment derived from the L1 protein of the first papilloma virus type (e.g. HPV L1 protein), which provides the immunogenicity of the first papilloma virus type (e.g. HPV), and a C-terminal fragment derived from the L1 protein of the second papilloma virus type (e.g. HPV L1 protein), which provides the features of better expression levels and solubility. These two fragments can be connected directly or via a linker.

The length of the N-terminal fragment of the HPV L1 protein appropriate for maintaining the immunogenicity of the L1 protein of the first type and ensuring the formation of VLP is determined. The following reports relate to epitope studies of common HPV types.

Sunanda Baidya et al. reported that the epitopes of the L1 protein 48EEYDLQFIFQLCKITL T A65, 45RHGEEYDLQFIFQLCKITLTA65, 63LPDPNKF69, 79PETQRLVWAC88, 36PVPGQYDA43, 77YNPETQRLVWAC88, 188DTGYGAMD195, 36PVPGQYDATK45, 45KQDIPKVSAYQYRVFRV61, 130RDNVSVDYKQTQLCI144 and 49YSRHVEEYDLQFIF62 can be used as tools for designing HPV 16 and 18 vaccines (see Epitope design of L1 protein for vaccine production against Human Papilloma Virus types 16 and 18, Bioinformation 13(3): 86-93 March 2017).

Katharina Slupetzky et al. reported that the regions near aa 282-286 and 351-355 of HPV-16 contribute to the neutralization epitopes and that the latter is the immunodominant site (see Chimeric papilloma virus-like particles expressing a foreign epitope on capsid surface loops, Journal of General Virology (2001), 82, 2799-2804).

Brooke Bishop et al. prepared the following three variants of the HPV 11, 16, 18 and 35 L1 proteins: deletion of 9 amino acids at its N terminus, deletion of α4 (corresponding to amino acid residues 404-436 of HPV 16), and deletion of 31 amino acids at its C terminus respectively, and reported that the former two could not be assembled into VLP, but this phenomenon has not been reported in the latter one (Crystal Structures of Four Types of Human Papillomavirus L1 Capsid Proteins UNDERSTANDING THE SPECIFICITY OF NEUTRALIZING MONOCLONAL ANTIBODIES, The Journal of Biological Chemistry, 282, 31803-31811). Each of the α-helix, β-fold sheets and Loop Region of each type of HPV L1 protein can be conveniently determined by sequence analysis software commonly used in the field. Wherein the α-helix regions contains the α1 Region, α2 Region, α3 Region, α4 Region and α5 Region.

β-B1 β-B2 BC-loop β-C
HPV16-L1 msLWlpseaTVYlPP-vpVskVVsTDeYVarTnIyYHAgtSRLLaVGhPYfpikkpnnnKilvPKVSglQYRVFrihLPDPNKFgfPDTS 89
HPV35-L1 msLWrsneaTVYlPP-vsVskVVsTDeYVtrTnIyYHAgsSRLLaVGhPYyaikkqdsnKiavPKVSglQYRVFrvkLPDPNKFgfPDTS 89
HPV11-L1 --mWrpsdsTVYvPPpnpVskVVaTDaYVkrTnIfYHAssSRLLaVGhPYysikkvn--KtvvPKVSgyQYRVFkvvLPDPNKFalPDSS 86
HPV18-L1 malWrpsdnTVYlPP-psVarVVnTDdYVtpTsIfYHAgsSRLLtVGnPYfrvpagggnKqdiPKVSayQYRVFrvqLPDPNKFglPDTS 89

β-D DE-loop β-E EF-
HPV16-L1 fynPdtQRLVWACvGvEvGRGQPLGVGiSGHPllNKlDDtEnasayaaNagvDnRecismDYKQTQLCliGCkPpiGEHWgKGspCtnva 179
HPV35-L1 fydPasQRLVWACtGvEvGRGQPLGVGiSGHPllNKlDDtEnsnkyvgNsgtDnRecismDYKQTQLCliGCrPpiGEHWgKGtpCnanq 179
HPV11-L1 lfdPttQRLVWACtGlEvGRGQPLGVGvSGHPllNKyDDvEnsggyggNpgqDnRvnvgmDYKQTQLCmvGCaPplGEHWgKGtqCsnts 176
HPV18-L1 iynPetQRLVWACaGvEiGRGQPLGVGlSGHPfyNKlDDtEsshaatsNvseDvRdnvsvDYKQTQLCilGCaPaiGEHWaKGtaCksrp 179

loop α1 β-F FG-
HPV16-L1 vnpGdCPPLELintVIqDGDMVDTGFGAMdFttLQaNKseVPLDICtsiCKYPDYIkMvsePYGDslFFyLRrEQmFvRHlfNRAGavGe 269
HPV35-L1 vkaGeCPPLELlntVIqDGDMVDTGFGAMdFttLQaNKsdVPLDICssiCKYPDYLkMvsePYGDmlFFyLRrEQmFvRHlfNRAGtvGe 269
HPV11-L1 vqnGdCPPLELitsVIqDGDMVDTGFGAMnFadLQtNKsdVPLDICgtvCKYPDYLqMaadPYGDrlFFyLRkEQmFaRHffNRAGtvGe 266
HPV18-L1 lsqGdCPPLELkntVleDGDMVDTGYGAMdFstLQdTKceVPLDICqsiCKYPDYLqMsadPYGDsmFFcLRrEQlFaRHfwNRAGtmGd 269

loop G1 G2 β-H1 β-H2 HI-loop
HPV16-L1 nVPddLyiKGsgstanlaSsnYfptPSGSmVtSdaQiFNKPYWLqrAQGHNNGiCWgNqLFVTVVDTTrSTNmslCaaistse-ttYknt 358
HPV35-L1 tVPadLyiKG--ttgtlpStsYfptPSGSmVtSdaQiFNKPYWLqrAQGHNNGiCWsNqLFVTVVDTTrSTNmsvCsavsssd-stYknd 356
HPV11-L1 pVPddLlvKGgnnrssvaSsiYvhtPSGSlVsSeaQlFNKPYWLqkAQGHNNGiCWgNhLFVTVVDTTrSTNmtlCasvsks--atYtns 354
HPV18-L1 tVPqsLyiKGtgmpaspgScvYspsPSGSiVtSdsQlFNKPYWLhkAQGHNNGvCWhNqLFVTVVDTTpSTNltiCastqspvPgqYdat 359

β-I α2 α3 α4
HPV16-L1 nfKeYlRHgEEyDLQFIFQLCkITLtAdVMtYIHsMNstiLEDWNFGlqPPPggtLeDTYRfVtSqAiaCQKhtppapkeDPlkkytFWe 448
HPV35-L1 nfKeYlRHgEEyDLQFIFQLCkITLtAdVMtYIHsMNpsiLEDWNFGltPPPsgtLeDTYRyVtSqAvtCQKpsapkpkdDPlknytFWe 446
HPV11-L1 dyKeYmRHvEEfDLQFIFQLCsITLsAeVMaYIHtMNpsvLEDWNFGlsPPPngtLeDTYRyVqSqAitCQKptpekekqDPykdmsFWe 444
HPV18-L1 kfKqYsRHvEEyDLQFIFQLCtITLtAdVMsYIHsMNssiLEDWNFGvpPPPttsLvDTYRfVqSvAitCQKdaapaenkDPydklkFWn 449

β-J α5
HPV16-L1 VnLKEKFSadLDQfPLGRKFLlQaGlkakpkftlgkr-katpttsststtakRkkrkl 505
HPV35-L1 VdLKEKFSadLDQfPLGRKFLlQaGlkarpnfrlgkr-aapastskksstkrRkvks 502
HPV11-L1 VnLKEKFSseLDQfPLGRKFLlQsGyrgrtsartgik-rpavskpstapkrkRtktkk 501
HPV18-L1 VdLKEKFSldLDQyPLGRKFLvQaGlrrkptigprkrsapsattsskpakrvRvrark 507

The inventors performed sequence alignments of L1 proteins of 14 HPV types (Types 6, 11, 16, 18, 31, 33, 35, 39, 45, 51, 52, 56, 58, and 59) and then performed secondary structure prediction according to the literature cited above (Crystal Structures of Four Types of Human Papillomavirus L1 Capsid Proteins UNDERSTANDING THE SPECIFICITY OF NEUTRALIZING MONOCLONAL ANTIBODIES, The Journal of Biological Chemistry, 282,31803-31811), the results of which are shown below, where the part between the downward-pointing arrows corresponds to the regions which had been deleted for preparing the variants in that literature.

▼ ▼ β-B1 β-B2 BC-
HPV6-L1 --MWRPSDSTVYVPPPNPVSKVVATDAYVTRTNIFYHASSSRLLAVGHPYFSIKRA----- 54
HPV11-L1 --MWRPSDSTVYVPPPNPVSKVVATDAYVKRTNIFYHASSSRLLAVGHPYYSIKKV----- 54
HPV52-L1 MSVWRPSEATVYLP-PVPVSKVVSTDEYVSRTSIYYYAGSSRLLTVGHPYFSIKNTSSGN 59
HPV58-L1 MSVWRPSEATVYLP-PVPVSKVVSTDEYVSRTSIYYYAGSSRLLAVGNPYFSIKSP--NN 57
HPV33-L1 MSVWRPSEATVYLP-PVPVSKVVSTDEYVSRTSIYYYAGSSRLLAVGHPYFSIKNP--TN 57
HPV31-L1 MSLWRPSEATVYLP-PVPVSKVVSTDEYVTRTNIYYHAGSARLLTVGHPYYSIPKS--DN 57
HPV16-L1 MSLWLPSEATVYLP-PVPVSKVVSTDEYVARTNIYYHAGTSRLLAVGHPYFPIKKP--NN 57
HPV35-L1 MSLWRSNEATVYLP-PVSVSKVVSTDEYVTRTNIYYHAGSSRLLAVGHPYYAIKKQ--DS 57
HPV51-L1 MALWRTNDSKVYLP-PAPVSRIVNTEEYITRTGIYYYAGSSRLITLGHPYFPIPK----T 55
HPV56-L1 MATWRPSENKVYLP-PTPVSKVVATDSYVKRTSIFYHAGSSRLLAVGHPYYSVTK----D 55
HPV39-L1 MAMWRSSDSMVYLP-PPSVAKVVNTDDYVTRTGIYYYAGSSRLLTVGHPYFKV-GM--NG 56
HPV59-L1 MALWRSSDNKVYLP-PPSVAKVVSTDEYVTRTSIFYHAGSSRLLTVGHPYFKV-PK--GG 56
HPV18-L1 MALWRPSDNTVYLP-PPSVARVVNTDDYVTRTSIFYHAGSSRLLTVGNPYFRV-PA--GG 56
HPV45-L1 MALWRPSDSTVYLP-PPSVARVVNTDDYVSRTSIFYHAGSSRLLTVGNPYFRVVPS--GA 57

loop β-C β-D
HPV6-L1 -NKTVVPKVSGYQYRVFKVVLPDPNKFALPDSSLFDPTTQRLVWACTGLEVGRGQPLGVG 113
HPV11-L1 -NKTVVPKVSGYQYRVFKVVLPDPNKFALPDSSLFDPTTQRLVWACTGLEVGRGQPLGVG 113
HPV52-L1 GKKVLVPKVSGLQYRVFRIKLPDPNKFGFPDTSFYNPETQRLVWACTGLEIGRGQPLGVG 119
HPV58-L1 NKKVLVPKVSGLQYRVFRVRLPDPNKFGFPDTSFYNPDTQRLVWACVGLEIGRGQPLGVG 117
HPV33-L1 AKKLLVPKVSGLQYRVFRVRLPDPNKFGFPDTSFYNPDTQRLVWACVGLEIGRGQPLGVG 117
HPV31-L1 PKKIVVPKVSGLQYRVFRVRLPDPNKFGFPDTSFYNPETQRLVWACVGLEVGRGQPLGVG 117
HPV16-L1 -NKILVPKVSGLQYRVFRIHLPDPNKFGFPDTSFYNPDTQRLVWACVGVEVGRGQPLGVG 116
HPV35-L1 -NKIAVPKVSGLQYRVFRVKLPDPNKFGFPDTSFYDPASQRLVWACTGVEVGRGQPLGVG 116
HPV51-L1 STRAAIPKVSAFQYRVFRVQLPDPNKFGLPDPNLYNPDTDRLVWGCVGVEVGRGQPLGVG 115
HPV56-L1 NTKTNIPKVSAYQYRVFRVRLPDPNKFGLPDTNIYNPDQERLVWACVGLEVGRGQPLGAG 115
HPV39-L1 GRKQDIPKVSAYQYRVFRVTLPDPNKFSIPDASLYNPETQRLVWACVGVEVGRGQPLGVG 116
HPV59-L1 NGRQDVPKVSAYQYRVFRVKLPDPNKFGLPDNTVYDPNSQRLVWACVGVEIGRGQPLGVG 116
HPV18-L1 GNKQDIPKVSAYQYRVFRVQLPDPNKFGLPDTSIYNPETQRLVWACAGVEIGRGQPLGVG 116
HPV45-L1 GNKQAVPKVSAYQYRVFRVALPDPNKFGLPDSTIYNPETQRLVWACVGMEIGRGQPLGIG 117

DE-loop β-E EF-
HPV6-L1 VSGHPFLNKYDDVENSG-SGGNPGQDNRVNVGMDYKQTQLCMVGCAPPLGEHWGKGKQCT 172
HPV11-L1 VSGHPLLNKYDDVENSGGYGGNPGQDNRVNVGMDYKQTQLCMVGCAPPLGEHWGKGTQCS 173
HPV52-L1 ISGHPLLNKFDDTETSNKYAGKPGIDNRECLSMDYKQTQLCILGCKPPIGEHWGKGTPCN 179
HPV58-L1 VSGHPYLNKFDDTETSNRYPAQPGSDNRECLSMDYKQTQLCLIGCKPPTGEHWGKGVACN 177
HPV33-L1 ISGHPLLNKFDDTETGNKYPGQPGADNRECLSMDYKQTQLCLLGCKPPTGEHWGKGVACT 177
HPV31-L1 ISGHPLLNKFDDTENSNRYAGGPGTDNRECISMDYKQTQLCLLGCKPPIGEHWGKGSPCS 177
HPV16-L1 ISGHPLLNKLDDTENASAYAANAGVDNRECISMDYKQTQLCLIGCKPPIGEHWGKGSPCT 176
HPV35-L1 ISGHPLLNKLDDTENSNKYVGNSGTDNRECISMDYKQTQLCLIGCRPPIGEHWGKGTPCN 176
HPV51-L1 LSGHPLFNKYDDTENSRIANGNAQQDVRDNTSVDNKQTQLCIIGCAPPIGEHWGIGTTCK 175
HPV56-L1 LSGHPLFNRLDDTESSNLANNNVIEDSRDNISVDGKQTQLCIVGCTPAMGEHWTKGAVCK 175
HPV39-L1 ISGHPLYNRQDDTENSPFS-STTNKDSRDNVSVDYKQTQLCIIGCVPAIGEHWGKGKACK 175
HPV59-L1 LSGHPLYNKLDDTENSHVASAVDTKDTRDNVSVDYKQTQLCIIGCVPAIGEHWTKGTACK 176
HPV18-L1 LSGHPFYNKLDDTESSHAATSNVSEDVRDNVSVDYKQTQLCILGCAPAIGEHWAKGTACK 176
HPV45-L1 LSGHPFYNKLDDTESAHAATAVITQDVRDNVSVDYKQTQLCILGCVPAIGEHWAKGTLCK 177

loop
HPV6-L1 NTPVQAGDCPPLELITSVIQDGDMVDTGFGAMNFADLQTNKSDVPIDICGTTCKYPDYLQ 232
HPV11-L1 NTSVQNGDCPPLELITSVIQDGDMVDTGFGAMNFADLQTNKSDVPLDICGTVCKYPDYLQ 233
HPV52-L1 NNSGNPGDCPPLQLINSVIQDGDMVDTGFGCMDFNTLQASKSDVPIDICSSVCKYPDYLQ 239
HPV58-L1 NNA-AATDCPPLELFNSIIEDGDMVDTGFGCMDFGTLQANKSDVPIDICNSTCKYPDYLK 236
HPV33-L1 NAA-PANDCPPLELINTIIEDGDMVDTGFGCMDFKTLQANKSDVPIDICGSTCKYPDYLK 236
HPV31-L1 NNAITPGDCPPLELKNSVIQDGDMVDTGFGAMDFTALQDTKSNVPLDICNSICKYPDYLK 237
HPV16-L1 NVAVNPGDCPPLELINTVIQDGDMVDTGFGAMDFTTLQANKSEVPLDICTSICKYPDYIK 236
HPV35-L1 ANQVKAGECPPLELLNTVLQDGDMVDTGFGAMDFTTLQANKSDVPLDICSSICKYPDYLK 236
HPV51-L1 NTPVPPGDCPPLELVSSVIQDGDMIDTGFGAMDFAALQATKSDVPLDISQSVCKYPDYLK 235
HPV56-L1 STQVTTGDCPPLALINTPIEDGDMIDTGFGAMDFKVLQESKAEVPLDIVQSTCKYPDYLK 235
HPV39-L1 PNNVSTGDCPPLELVNTPIEDGDMIDTGYGAMDFGALQETKSEVPLDICQSICKYPDYLQ 235
HPV59-L1 PTTVVQGDCPPLELINTPIEDGDMVDTGYGAMDFKLLQDNKSEVPLDICQSICKYPDYLQ 236
HPV18-L1 SRPLSQGDCPPLELKNTVLEDGDMVDTGYGAMDFSTLQDTKCEVPLDICQSICKYPDYLQ 236
HPV45-L1 PAQLQPGDCPPLELKNTIIEDGDMVDTGYGAMDFSTLQDTKCEVPLDICQSICKYPDYLQ 237

α1 β-F FG-loop
HPV6-L1 MAADPYGDRLFFFLRKEQMFARHFFNRAGEVGEPVPDTLIIKGS--GNRTSVGSSIYVNT 290
HPV11-L1 MAADPYGDRLFFYLRKEQMFARHFFNRAGTVGEPVPDDLLVKGG--NNRSSVASSIYVHT 291
HPV52-L1 MASEPYGDSLFFFLRREQMFVRHFFNRAGTLGDPVPGDLYIQGSNSGNTATVQSSAFFPT 299
HPV58-L1 MASEPYGDSLFFFLRREQMFVRHFFNRAGKLGEAVPDDLYIKGS--GNTAVIQSSAFFPT 294
HPV33-L1 MTSEPYGDSLFFFLRREQMFVRHFFNRAGTLGEAVPDDLYIKGS--GTTASIQSSAFFPT 294
HPV31-L1 MVAEPYGDTLFFYLRREQMFVRHFFNRSGTVGESVPTDLYIKGS--GSTATLANSTYFPT 295

HPV16-L1 MVSEPYGDSLFFYLRREQMFVRHLFNRAGAVGENVPDDLYIKGS--GSTANLASSNYFPT 294
HPV35-L1 MVSEPYGDMLFFYLRREQMFVRHLFNRAGTVGETVPADLYIKGT--T--GTLPSTSYFPT 292
HPV51-L1 MSADTYGNSMFFHLRREQIFARHYYNKLVGVGEDIPNDYYIKGSG-NGRDPIESYIYSAT 294
HPV56-L1 MSADAYGDSMWFYLRREQLFARHYFNRAGKVGETIPAELYLKGS--NGREPPPSSVYVAT 293
HPV39-L1 MSADVYGDSMFFCLRREQLFARHFWNRGGMVGDAIPAQLYIKGT--DIRANPGSSVYCPS 293
HPV59-L1 MSADAYGDSMFFCLRREQVFARHFWNRSGTMGDQLPESLYIKGT--DIRANPGSYLYSPS 294
HPV18-L1 MSADPYGDSMFFCLRREQLFARHFWNRAGTMGDTVPQSLYIKGT--GMRASPGSCVYSPS 294
HPV45-L1 MSADPYGDSMFFCLRREQLFARHFWNRAGVMGDTVPTDLYIKGTSANMRETPGSCVYSPS 297

G1 G2 β-H1 β-H2 HI-

HPV6-L1 PSGSLVSSEAQLFNKPYWLQKAQGHNNGICWGNQLFVTVVDTTRSTNMTLCASVT--TSS 348
HPV11-L1 PSGSLVSSEAQLFNKPYWLQKAQGHNNGICWGNHLFVTVVDTTRSTNMTLCASVS--KSA 349
HPV52-L1 PSGSMVTSESQLFNKPYWLQRAQGHNNGICWGNQLFVTVVDTTRSTNMTLCAEVK--KES 357
HPV58-L1 PSGSIVTSESQLFNKPYWLQRAQGHNNGICWGNQLFVTVVDTTRSTNMTLCTEVT--KEG 352
HPV33-L1 PSGSMVTSESQLFNKPYWLQRAQGHNNGICWGNQVFVTVVDTTRSTNMTLCTQVT--SDS 352
HPV31-L1 PSGSMVTSDAQIFNKPYWMQRAQGHNNGICWGNQLFVTVVDTTRSTNMSVCAAIAN-SDT 354
HPV16-L1 PSGSMVTSDAQIFNKPYWLQRAQGHNNGICWGNQLFVTVVDTTRSTNMSLCAAIST-SET 353
HPV35-L1 PSGSMVTSDAQIFNKPYWLQRAQGHNNGICWSNQLFVTVVDTTRSTNMSVCSAVSS-SDS 351
HPV51-L1 PSGSMITSDSQIFNKPYWLHRAQGHNNGICWNNQLFITCVDTTRSTNLTISTATAA-VSP 353
HPV56-L1 PSGSMITSEAQLFNKPYWLQRAQGHNNGICWGNQLFVTVVDTTRSTNMTISTATE--QLS 351
HPV39-L1 PSGSMVTSDSQLFNKPYWLHKAQGHNNGICWHNQLFLTVVDTTRSTNFTLSTSIESSIPS 353
HPV59-L1 PSGSVVTSDSQLFNKPYWLHKAQGLNNGICWHNQLFLTVVDTTRSTNLSVCASTTSSIPN 354
HPV18-L1 PSGSIVTSDSQLFNKPYWLHKAQGHNNGVCWHNQLFVTVVDTTRSTNLTICASTQSPVPG 354
HPV45-L1 PSGSITTSDSQLFNKPYWLHKAQGHNNGICWHNQLFVTVVDTTRSTNLTLCASTQNPVPN 357

loop β-I α2 α3 ▼ ▪

HPV6-L1 TYTNSDYKEYMRHVEEYDLQFIFQLCSITLSAEVMAYIHTMNPSVLEDWNFGLSPPPNGT 408
HPV11-L1 TYTNSDYKEYMRHVEEFDLQFIFQLCSITLSAEVMAYIHTMNPSVLEDWNFGLSPPPNGT 409
HPV52-L1 TYKNENFKEYLRHGEEFDLQFIFQLCKITLTADVMTYIHKMDATILEDWQFGLTPPPSAS 417
HPV58-L1 TYKNDNFKEYVRHVEEYDLQFVFQLCKITLTAEIMTYIHTMDSNILEDWQFGLTPPPSAS 412
HPV33-L1 TYKNENFKEYIRHVEEYDLQFVFQLCKVTLTAEVMTYIHAMNPDILEDWQFGLTPPPSAS 412
HPV31-L1 TFKSSNFKEYLRHGEEFDLQFIFQLCKITLSADIMTYIHSMNPAILEDWNFGLTTPPSGS 414
HPV16-L1 TYKNTNFKEYLRHGEEYDLQFIFQLCKITLTADVMTYIHSMNSTILEDWNFGLQPPPGGT 413
HPV35-L1 TYKNDNFKEYLRHGEEYDLQFIFQLCKITLTADVMTYIHSMNPSILEDWNFGLTPPPSGT 411
HPV51-L1 TFTPSNFKQYIRHGEEYELQFIFQLCKITLTTEVMAYLHTMDPTILEQWNFGLTLPPSAS 413
HPV56-L1 KYDARKINQYLRHVEEYELQFVFQLCKITLSAEVMAYLHNMNANLLEDWNIGLSPPVATS 411
HPV39-L1 TYDPSKFKEYTRHVEEYDLQFIFQLCTVTLTTDVMSYIHTMNSSILDNWNFAVAPPPSAS 413
HPV59-L1 VYTPTSFKEYARHVEEFDLQFIFQLCKITLTTEVMSYIHNMNTTILEDWNFGVTPPPTAS 414
HPV18-L1 QYDATKFKQYSRHVEEYDLQFIFQLCTITLTADVMSYIHSMNSSILEDWNFGVPPPPTTS 414
HPV45-L1 TYDPTKFKHYSRHVEEYDLQFIFQLCTITLTAEVMSYIHSMNSSILENWNFGVPPPPTTS 417

α4 ▼ β-J α5

HPV6-L1 LEDTYRYVQSQAITCQKPTPEKEKPDPYKNLSFWEVNLKEKFSSELDQYPLGRKFLLQSG 468
HPV11-L1 LEDTYRYVQSQAITCQKPTPEKEKQDPYKDMSFWEVNLKEKFSSELDQFPLGRKFLLQSG 469
HPV52-L1 LEDTYRFVTSTAITCQKNTPPKGKEDPLKDYMFWEVDLKEKFSADLDQFPLGRKFLLQAG 477
HPV58-L1 LQDTYRFVTSQAITCQKTAPPKEKEDPLNKYTFWEVNLKEKFSADLDQFPLGRKFLLQSG 472
HPV33-L1 LQDTYRFVTSQAITCQKTVPPKEKEDPLGKYTFWEVDLKEKFSADLDQFPLGRKFLLQAG 472
HPV31-L1 LEDTYRFVTSQAITCQKSAPQKPKEDPFKDYVFWEVNLKEKFSADLDQFPLGRKFLLQAG 474
HPV16-L1 LEDTYRFVTSQAIACQKHTPPAPKEDPLKKYTFWEVNLKEKFSADLDQFPLGRKFLLQAG 473
HPV35-L1 LEDTYRYVTSQAVTCQKPSAPKPKDDPLKNYTFWEVDLKEKFSADLDQFPLGRKFLLQAG 471
HPV51-L1 LEDAYRFVRNAATSCQKDTPPQAKPDPLAKYKFWDVDLKERFSLDLDQFALGRKFLLQVG 473
HPV56-L1 LEDKYRYVRSTAITCQREQPPTEKQDPLAKYKFWDVNLQDSFSTDLDQFPLGRKFLMQLG 471
HPV39-L1 LVDTYRYLQSAAITCQKDAPAPEKKDPYDGLKFWNVDLREKFSLELDQFPLGRKFLLQAR 473
HPV59-L1 LVDTYRFVQSAAVTCQKDTAPPVKQDPYDKLKFWPVDLKERFSADLDQFPLGRKFLLQLG 474
HPV18-L1 LVDTYRFVQSVAITCQKDAAPAENKDPYDKLKFWNVDLKEKFSLDLDQYPLGRKFLVQAG 474
HPV45-L1 LVDTYRFVQSVAVTCQKDTTPPEKQDPYDKLKFWTVDLKEKFSSDLDQYPLGRKFLVQAG 477

▼ ▼

HPV6-L1 YRGRSSIRTGVKRPAVSKASAAPKRKRAKTKR-- 500
HPV11-L1 YRGRTSARTGIKRPAVSKPSTAPKRKRTKTKK-- 501
HPV52-L1 LQARPKLKRPASSAPRTST-----KKK-KVKR--- 503
HPV58-L1 LKAKPRLKRSAPTTRAP-ST----KRK-KVKK--- 498
HPV33-L1 LKAKPKLKRAAPTSTRTSSA----KRK-KVKK--- 499
HPV31-L1 YRARPKFKAGKRSAPSASTTTPAKRKKTKK---- 504
HPV16-L1 LKAKPKFTLGKRKATPTTSSTSTTAKRKKRKL-- 505
HPV35-L1 LKARPNFRLGKRAAPASTSKKSSTKRRKVKS--- 502
HPV51-L1 VQRKPRPGLKRPASSASSSSSSSAKRKRVKK--- 504
HPV56-L1 TRSKPAVATSKKRSAPTSTS-TPAKRKRR----- 499
HPV39-L1 VRRRPTIGPRKRPAASTSSSSATKHKRKRVSK-- 505
HPV59-L1 ARPKPTIGPRKRAAPAPTSTPSPKRVKRRKSSRK 508
HPV18-L1 LRRKPTIGPRKRSAPSATTSSKPAKRVRVRARK- 507
HPV45-L1 LRRRPTIGPRKRPAASTSTASRPAKRVRIRSKK- 510

In addition to the methods used by the inventors for sequence alignments, protein secondary structure prediction software that can be used for prediction includes, but is not limited to:
1. JPred: http://www.compbio.dundee.ac.uk/jpred/index.html
2. ProtPredicct: http://predictprotein.org
3. PsiPred: http://bioinf.cs.ucl.ac.uk/psipred
4. SCRATCH-1D: http://download.igb.uci.edu
5. Nnpredict: http://www.cmpharm.uesf.edu/~nomi/nnpredict
6. Predictprotein: http://www.emblheidelberg.de/predictprotein/SOPMA http://www.ibcp.fr/predict.html
7. SSPRED: http://www.embl-heidelberg.de/sspred/ssprd_info.html.

In one aspect of the present disclosure, the inventors determined the length of the N-terminal fragment derived from the L 1 protein of the first HPV type in the following manner: the natural sequence of the L1 protein is truncated within its α5 region and nearby regions thereof, with the sequence from its N-terminus to the newly generated C-terminus within the α5 region retained. Such a truncated sequence ensures that it has the immunogenicity of the first type and is capable of forming VLP.

The N-terminal fragment of the HPV L1 protein derived from the first type can be further modified as long as it retains the immunogenicity of the first type and is capable of forming VLP.

The length of the C-terminal fragment of the HPV L1 protein derived from the second type is determined in the following manner: The natural sequence of the L1 protein is truncated within its α5 region and nearby regions thereof, then the sequence from the newly generated N-terminus within its α5 region to the C-terminus is retained. Such a truncated sequence does not have the major neutralizing antigenic epitope so that does not interfere with the immunogenicity of the resulting chimeric protein.

The C-terminal fragment of the HPV L1 protein derived from the second type can be further mutated, deleted and/or added, preferably retaining at least one of its nuclear localization sequences. Yang et al. predicted the nuclear localization sequences of 107 HPV subtypes (Yang et al. Predicting the nuclear localization signals of 107 types of HPV L1 proteins by bioinformatic analysis. Geno. Prot. Bioinfo. Vol. 4 No. 1 2006). The nuclear localization sequences of each type of HPV L1 protein can be conveniently determined by sequence analysis software commonly used in the field.

The above-described connection of the N-terminal fragment to the C-terminal fragment occurs at the newly generated C-terminus of the former and at the newly generated N-terminus of the latter. This can be a direct connection or a connection via a linker. The site where the connection occurs is defined as the origin coordinate, the N-terminal side of the origin is regarded as minus while the C-terminal side as plus.

The following shows the sequence of amino acids 453-469 of the HPV6 L1 protein and the corresponding sequences of L1 proteins of other HPV types. These sequences overlap with their α5 region respectively. It can be seen these sequences are highly similar. The numbers in brackets indicate the positions of the last amino acid residue of the listed sequences, where for HPV Type 45, an additional 26 amino acids are present at the N-terminus of the L1 protein of some HPV Type 45 strains, while at the N-terminus of the L1 protein of other HPV Type 45 strains, the said additional 26 amino acids are not present, therefore the number is indicated as (478) + 26.
HPV6 ELDQYPLGRKFLLQSGY(469)
HPV11 ELDQFPLGRKFLLQSGY(470)
HPV16 DLDQFPLGRKFLLQAGL(474)
HPV18 DLDQYPLGRKFLVQAGL(475)
HPV31 DLDQFPLGRKFLLQAGY(475)
HPV35 DLDQFPLGRKFLLQAGL(472)
HPV39 ELDQFPLGRKFLLQARV (474)
HPV45 DLDQYPLGRKFLVQAGL(478)+26
HPV51 DLDQFALGRKFLLQVGV(474)
HPV52 DLDQFPLGRKFLLQAGL(478)
HPV56 DLDQFPLGRKFLMQLGTRS(474)
HPV58 DLDQFPLGRKFLLQSGL(473)
HPV33 DLDQFPLGRKFLLQAGL(473)KAKPKLKRAAPTSTRTSSAKRKKVKK wherein KR at positions 480-481 and KRKK at positions 493-496 are nuclear localization sequences.
HPV59 DLDQFPLGRKFLLQLGA(475)RPKPTIGPRKRAAPAPTSTPSPKRVKRRK SSRK, wherein RKR at positions 484-486 and KRVKRRK at positions 498-504 are nuclear localization sequences.

In one aspect of the present disclosure, the inventors have completed C-terminus substitutions of L1 proteins between different HPV types by taking advantage of sequence similarity of the α5 region and nearby regions between the HPV types.

Most preferably, the inventors have noted that L1 protein of each HPV type has a tetrapeptide RKFL or, more advantageously, a hexapeptide LGRKFL, at a similar position. The inventors have skillfully exploited this highly conserved sequence to locate the chimeric protein connection point at any amino acid position within this oligopeptide. On one hand, the sequence starting from the N-terminus of the chimeric protein to RKFL or LGRKFL is identical to the sequence of the N-terminal fragment derived from the L1 protein of the first type, while on the other hand, the sequence starting from RKFL or LGRKFL to the C-terminus of the chimeric protein is identical to the sequence of the C-terminal fragment derived from the L1 protein of the second type.

The chimeric protein thus produced maintains a high degree of similarity to the natural HPV L1 protein, and it can be expected to perform well in production and even in medical or prophylactic procedures thereafter.

It will be understandable for those skilled in the art that there are different strains with different natural sequences of a given HPV type.

It will be understandable for those skilled in the art that because of the high degree of similarity between L1 protein of different HPV types, if, during the construction of the chimeric proteins, the N-terminal fragment derived from the L1 protein of the first type is extended by more amino acid residues towards the C-terminus, or the C-terminal fragment derived from L1 protein of the second HPV virus type is extended by more amino acid residues towards the N-terminus, it is also possible to form chimeric proteins that are structurally identical to the present invention due to identical or similar amino acids at the corresponding sites.

Variants of the chimeric proteins may be formed by mutations, deletions and/or additions of amino acid residues. These variants are likely to have the immunogenicity of the L1 protein of the first type, can form VLP, and have a good yield and solubility.

### The beneficial effects of the invention

The expression systems commonly used for producing virus-like particles are classified into eukaryotic expression systems and prokaryotic expression systems. The papilloma virus L proteins expressed by the eukaryotic expression systems can spontaneously assemble into viral-like particles, but have the disadvantage of low expression level thus are not suitable for mass production. The papilloma virus L protein expressed by the prokaryotic expression system requires *in vitro* processing to obtain virus-like particles because of often destroyed natural conformation, and has low yield, being difficult to be used in industrialization.

The present invention modifies the C-terminus of the L protein of the papilloma virus (e.g. human papilloma virus), for example by replacing it with the C-terminal fragment of HPV Type 16 L1 protein, HPV Type 28 L1 protein, HPV Type 33 L1 protein, HPV Type 59 L1 protein or HPV Type 68 L1 protein, thus can be used in expression systems (e.g. host cells, e.g. insect cells) to improve the expression level and the solubility of the papilloma virus L protein in expression systems (for example, host cells, e.g. insect cells). This can be used for the mass production of vaccines such as HPV vaccines.

The inventors found that the HPV Type 16 L1 protein, the HPV Type 28 L1 protein, the HPV Type 33 L1 protein, the HPV Type 59 L1 protein and the HPV Type 68 L1 protein have increased expression level and increased solubility compared to L1 proteins of other HPV types, and that said increased protein expression level and increased solubility was found to be depend on the C-terminal sequence of said HPV L1 protein. Among 107 HPV Type L1 proteins, most of them have nuclear localization sequences at the C-termini, and the C-terminal sequences have some similarities.

For papilloma virus L proteins that are currently inexpressible, very low in expression level or insoluble after expression, replacement of their C-terminal fragments with C-terminal fragments of the HPV Type 16 L1 protein, HPV Type 28 L1 protein, HPV Type 33 L1 protein, HPV Type 59 L1 protein or HPV Type 68 L1 protein makes it possible for soluble expression and subsequent purification. This strategy can be used for the mass production of polyvalent vaccines (e.g. HPV vaccines), making it possible to provide more comprehensive protection against a wide range of papilloma virus infections, especially HPV.

There needs to increase the expression level and the solubility of the HPV L1 protein in insect cells for mass production purpose. In addition, the virus-like particles assembled by the HPV L protein lack good conformation in yeast cells due to failure to form correct disulphide bonds .

For the HPV L1 proteins that are poorly expressed and insoluble in insect cells, a significant increase in expression level and solubility can be resulted after the modification of its C-terminal fragment into the C-terminal fragment of the HPV Type 33 or 59 L1 protein, thus they could be used for mass production of HPV vaccines.

For HPV L1 proteins that are better expressed and better soluble in insect cells compared to L1 proteins of other HPV types, such as HPV Type 16 protein, HPV Type 28 L1 protein, HPV Type 68 L1 protein, etc., there are needs to further improve the expression level and the solubility in order to achieve mass production of vaccines. In the present invention, for example, after modifying the C-terminal fragment of the HPV Type 16 L1 protein to the C-terminal fragment of the HPV Type 33 L1 protein, the expression level and the solubility of the modified chimeric HPV Type 16 protein are improved, which is conducive to the mass production of HPV vaccines.

To sum up, the chimeric HPV L1 proteins showed much higher expression level and solubility in insect cells compared to the unmodified HPV L1 protein. It can be used for the mass production of HPV vaccines. In addition, the chimeric HPV L1 proteins can correctly form disulfide bonds thus be assembled into HPV virus-like particles with good conformations in insect cells. This can improve the immunogenicity of HPV virus-like particles and trigger better immune responses.

The multivalent vaccine of the present invention can be used to prevent diseases or infections related with a wide range of HPV, including those HPV types that are not currently preventable.

### Definition

Unless otherwise stated, all technical and scientific terms used herein have the meanings normally understood by a person of ordinary skill in the art to which the invention belongs. For the convenience of understanding the present invention, the following terms are cited below for their ordinary meaning.

When used herein and in the attached claims, the singular forms "a/an", "another" and "said/the" include the plural designations of the objects unless the context clearly indicates otherwise. Unless otherwise expressly stated, the terms "include/comprise/have", "for example", etc. are intended to convey inclusion rather than limitation.

The term "immunogenicity" refers to the ability of a substance, for example a protein or a peptide, to trigger an immune response, i.e. the ability to trigger the production of antibodies, in particular the ability to trigger humoral- or cell-mediated response.

The term "antibody" refers to an immunoglobulin molecule that binds an antigen. Antibodies may be polyclonal mixtures or monoclonal. Antibodies may be intact immunoglobulins of natural origin or of recombinant origin or may be immunoreactive portions of intact immunoglobulins. Antibodies may be present in a variety of forms including, for example, Fv, Fab', F(ab')2 and as single chains.

The term "antigenicity" refers to the ability of a substance, for example a protein or a peptide, to trigger the production of antibodies that bind specifically to it.

The term "epitope" includes any protein determinant cluster that specifically binds to an antibody or T-cell receptor. Epitope determinants typically consist of chemically active surface groups (e.g. amino acids or sugar side chains, or combinations thereof) of the molecule and typically have specific three-dimensional structural characteristics as well as specific charge characteristics.

The terms "subtype" or "type" are used interchangeably herein to refer to genetic variant of virus that allows it can be recognized by the immune system as distinct antigen from type to type. For example, HPV 16 is immunologically distinguishable from HPV 33.

The term "HPV L1 protein", as used herein, the term "HPV" and "human papilloma virus" refer to envelope-free double-stranded DNA viruses of the Papillomavirus family. Their genomes are circular and approximately 8 kilobase pairs in size. Most HPVs encode eight major proteins, six in the "early" region (E1-E2) and two in the "late" region (L1 (major capsid protein) and L2 (minor capsid protein)). Over 120 HPV types have been identified and they are labelled by numbers (e.g. HPV-16, HPV-18, etc.).

The term "HPV" or "HPV virus" refers to papilloma viruses of the *Papillomaviridae* Family, which are envelope-free DNA viruses with a double-stranded closed circular DNA genome of approximately 8 kb in size that is usually classified into three regions: (i) the early region (E ), which contains six open reading frames E1, E2, E4-E7, encoding non-structural proteins related to viral replication, transcription and transformation, as well as open reading frames E3 and E8; (ii) the late region (L), which contains reading frames encoding the major capsid protein L1 and the minor capsid protein L2; and (iii) the long regulatory region (LCR), which does not encode any proteins but has the origin of replication and multiple transcription factor binding sites.

The terms "HPV L1 protein" and "HPV L2 protein" refer to proteins encoded by the late region (L) of the HPV gene and synthesized late in the HPV infection cycle. The L2 protein is the minor capsid protein. 72 L 1 pentamers form the outer shell of the icosahedral HPV particles, which encloses the closed circular double-stranded DNA microchromosome.

The term "virus-like particle" refers to a hollow particle containing one or plurality of structural proteins of a virus, without viral nucleic acids.

"HPV pseudovirus" is an ideal model for *in vitro* neutralization of HPV, by taking advantages of the non-specific nucleic acid encapsulation property of HPV VLP, HPV pseudovirus is formed by wrapping free DNA or introducing an exogenous plasmid into a VLP composed of intracellularly-expressed HPV L1 and L2.

The "pseudovirus neutralization assay" is a method for evaluating the neutralizing activity of antibodies. After incubation of immunized animal serum with a certain amount of pseudovirus and then infection of the cells, the amount of the cells decreases when serum neutralizing antibodies increases, showing a linear negative correlation in a certain range. The neutralizing activity of antibodies in serum can therefore be assessed by measuring changes in the amounts of cells.

The term "fragment thereof" or "variant thereof" refers to a deletion, insertion and/or substitution of nucleotides or amino acids sequence of the present invention. Preferably, the fragment or variant of the polypeptide provided by the present invention is capable of triggering the humoral- and/or the cellular-immune response in animals or humans.

The term "chimeric" means that sequences of polypeptides or nucleotides derived from different parental molecules are connected together by -CO-NH- or 3', 5'-phosphodiester bonds, respectively. Preferably, they are not spaced by additional linker sequences, but are directly adjacent to each other.

The term "truncation" refers to the removal of one or plurality of amino acids from the N- and/or C-terminus of a polypeptide or the deletion of one or plurality of amino acids from the interior of a polypeptide.

The term "nuclear localization sequence" refers to an amino acid sequence that directs the protein into the nucleus. In some HPV L1 proteins, two tight clusters of basic residues (i.e. nuclear localization sequences) (e.g. one is KRKR, KRKK, KRKRK, KRKKRK, KRVKRRK, etc. and the other is KR, RKR, KRK, etc.) have a spacer region of 10-14 amino acids between them. The above clusters of basic residues belong to nuclear localization sequences. In some other HPV L1 proteins, the nuclear localization sequence is a tight cluster of basic residues formed by arginines and/or lysines. Nuclear localization sequences include, but are not limited to, examples of clusters of basic residues as described above. See Jun Yang et al, Predicting the Nuclear Localization Signals of 107 Types of HPV L1 Proteins by Bioinformatic Analysis, Genomics, Proteomics & Bioinformatics Volume 4, Issue 1, 2006, Pages 34-41.

The term "functional variant" refers to a version of a polypeptide or a protein that retains the desired activities or characteristics after truncation, mutation, deletion and/or addition.

"Sequence identity" between two sequences of polypeptides or nucleic acids indicates the number of identical residues between said sequences as a percentage of the total number of residues, and is calculated based on the size of the smaller one of the compared molecules. When calculating the percentage identity, the sequences being compared are matched in such a way as to produce the maximum match between the sequences, with the vacant positions (if present) in the match being resolved by a specific algorithm. Preferred computer program methods for determining identity between two sequences include, but are not limited to, GCG program packages including GAP, BLASTP, BLASTN and FASTA (Altschul et al., 1990, J. Mol. Biol. 215: 403-410). The above programs are publicly available from the National Center of Biotechnology Information (NCBI) and other sources. The well-known Smith Waterman Algorithm can also be used to determine the identity.

Non-critical amino acids can be conservatively substituted without affecting the normal function of the protein. Conservative substitution means replacing amino acids with chemically or functionally similar amino acids. Tables for conservative substitutions that provide similar amino acids are well known in the art. By way of example, in some embodiments, the groups of amino acids provided in **Tables 1-3** are considered to be conservative substitutions for each other.

**Table 1 In some embodiments, the selected groups of amino acids that are considered to be conservative substitutions for each other**

| | |
|---|---|
| Acid residues | D and E |
| Basic residues | K, R and H |
| Hydrophilic uncharged residues | S, T, N and Q |
| Aliphatic uncharged residues | G, A, V, L and I |
| Non-polar uncharged residues | C, M and P |
| Aromatic residues | F, Y and W |

**Table 2 In some embodiments, other selected groups of amino acids considered to be conservative substitutions for each other**

| | |
|---|---|
| Group 1 | A, S and T |
| Group 2 | D and E |
| Group 3 | N and Q |
| Group 4 | R and K |
| Group 5 | I, L and M |
| Group 6 | F, Y and W |

**Table 3 In some embodiments, other selected groups of amino acids considered to be conservative substitutions for each other**

| | |
|---|---|
| Group A | A and G |
| Group B | D and E |
| Group C | N and Q |
| Group D | R, K and H |
| Group E | I, L, M and V |
| Group F | F, Y and W |
| Group G | S and T |
| Group H | C and M |

The term "amino acids" refers to the twenty common naturally occurring amino acids. Naturally occurring amino acids include alanine (Ala; A), arginine (Arg; R), asparagine (Asn; N), aspartic acid (Asp; D), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y) and valine (Val; V).

As used herein, a "physiologically acceptable carrier" is non-toxic to cells or mammals at the dose and concentration used. Usually it is a pH buffered aqueous solution, non-limiting examples of which include buffers; antioxidants; oligopeptides; proteins; hydrophilic polymers; amino acids; monosaccharides, disaccharides and other carbohydrates; chelating agents; sugar alcohols; salt-forming counterions such as sodium; and/or nonionic surface active agents.

The term "adjuvant" refers to a compound or mixture that enhances immune responses. In particular, a vaccine may comprise an adjuvant. Adjuvants for use in the present invention may include, but are not limited to, one or plurality of the following: mineral-containing adjuvant compositions, oil-emulsion adjuvants, saponin adjuvant formulations, derivatives of bacteria or microbes.

The term "vector" refers to a nucleic acid molecule capable of proliferating another nucleic acid connected to it. The term includes vectors as self-replicating nucleic acid structures and as vectors integrated into the genome of host cells into which the vector has been introduced. Certain vectors are capable of directing the expression of nucleic acids to which such vectors are operatively connected.

The term "host cell" refers to a cell into which an exogenous nucleic acid has been introduced, as well as to the progeny of such a cell. Host cells include "transformants" (or "transformed cells"), "transfectants" (or "transfected cells") or "infectants" (or "infected cells"), each of which includes primary transformed, transfected or infected cells and the progeny derived from them. Such progeny may not be identical to the parental cells in terms of nucleic acid content and may contain mutations.

The administration amount is preferably a "prophylactically effective amount" (herein the prophylaxis may be considered as treatment and the two may be used interchangeably), which is sufficient to show benefit to the individual.

### Examples

### Example 1 Construction of chimeric genes

Example 1.1: Construction of a chimeric gene with the C-terminus of HPV6 L1 substituted with the C-terminus of HPV33 L1

### 1.1.1 Construction of pFB-HPV6 L1 as the template

The HPV6 L1 gene with the KpnI and XbaI cleavage sites at both ends of the synthesized sequences was synthesized by Thermo Fisher [formerly Invitrogen (Shanghai) Trading Co.], its sequence is shown in SEQ ID NO: 5. The plasmid pcDNA3-HPV6-L1 comprising the nucleotide sequence encoding amino acid 1-500 of HPV6 L1 was obtained by ligating the synthesized gene fragment with pcDNA3 vector (distributor: Thermo Fisher) at KpnI and XbaI cleavage sites.

The obtained pcDNA3-HPV6-L1 plasmid was subjected to double enzyme digestion with KpnI and XbaI to obtain a gene fragment of HPV6 L1 (1-500). The fragment was then ligated with the KpnI / XbaI double digested pFastBac^{™}1 vector (distributor: Thermo Fisher) to obtain a rod vector containing the HPV6 L1 (1-500) gene fragment, named as pFB-HPV6 L1.

### 1.1.2 Construction of pFB-HPV33 L1 as the template

The HPV33 L1 gene with the KpnI and XbaI cleavage sites at both ends of the synthesized sequences was synthesized by Thermo Fisher [formerly Invitrogen (Shanghai) Trading Co.), its sequence is shown as SEQ ID NO: 6. The plasmid pcDNA3-HPV33-L1 comprising the nucleotide sequence encoding amino acids 1-499 of HPV33 LL1 was obtained by ligating the synthesized gene fragment with pcDNA3 vector (distributor: Thermo Fisher) at Kpn I and XbaI cleavage sites.

The pcDNA3-HPV33-L1 plasmid was subjected to double enzyme digestion with KpnI and Xbal to obtain a fragment of the HPV33 L1 (1-499) gene. The fragment was then ligated to the KpnI and XbaI double digested pFastBac^{™}1 vector (distributor: Thermo Fisher) to obtain a rod vector containing the HPV33 L1 (1-499) gene fragment, named as pFB-HPV33 L1.

### 1.1.3 Construction of pFB-HPV6 L1: 33C

Chimeric gene with HPV6 L1 C-terminus substituted with HPV33 L1 C-terminus: the constructed recombinant plasmid pFB-HPV6 L1 was used as the gene template to amplify a 1426 bp gene fragment using primers F1 and R1, the primer sequence F1 is shown in SEQ ID No: 7 and R1 is shown in SEQ ID No: 8.

This gene fragment contains a fragment encoding amino acids 1-469 of HPV6 L1, 10 bases overlapping with the gene fragment encoding amino acids 474-499 of HPV33 L1, and a fragment of the KpnI digest site (GGTAC^C), the amplified sequence is shown in SEQ ID No: 9.

PCR amplification parameters: pre-denaturation at 94°C for 5min; denaturation at 98°C for 10 s, annealing at 69°C for 15 s, 1 kb/1 min at 72°C, for 30 cycles; extension at 72°C for 5 min; end at 16°C.

The recombinant plasmid pFB-HPV33 L1 was used as the gene template to amplify a gene fragment of 101 bp in length using primers F2 and R2, the primer sequence of F2 is shown in SEQ ID No: 10 and the primer sequence of R2 is shown in SEQ ID No: 11.

This gene fragment contains a gene fragment encoding 26 C-terminal amino acids (474-499) of HPV33 L1, a 10 bp base overlapping with the gene fragment encoding the C-terminus of amino acids 1-469 of HPV6 L1 and the XbaI (T^CTAGA) digest site, the amplified sequence is shown in SEQ ID No: 12.

PCR amplification parameters: pre-denaturation at 94°C for 5min; denaturation at 98°C for 10 s, annealing at 69°C for 15 s, 1 kb/1 min at 72°C, for 30 cycles; extension at 72°C for 5min; end at 16°C.

### PCR ligating sequences:

The ligating primers were F1 and R2, and the fragments amplified using the above primers (amplified fragments of F1 and R1, amplified fragments of F2 and R2) were used as templates.

PCR ligating parameters: pre-denaturation at 94°C for 5 min; denaturation at 98°C for 10 s, annealing at 52°C for 15 s, 72°C for 1 kb/1 min, for 5 cycles; denaturation at 98°C for 10 s, annealing at 68°C for 15 s, 72°C for 1 kb/1 min, for 25 cycles; extension at 72°C for 5 min; end at 16°C.

The final result was SEQ ID NO: 4, a nucleotide sequence encoding amino acids 1-469 of HPV6 L1 and 26 C-terminal amino acids of HPV33 L1(aa 474-499), with KpnI and XbaI cleavage sites at both ends (hereafter referred to as the ligating sequence).

The recombinant plasmid pFB-HPV6 L1: 33C was obtained by double digesting the pFastBac^{™}1 vector and the ligating sequence fragment with KpnI+XbaI enzymes and cloning the ligating sequence into the pFastBac^{™}1 vector to obtain pFB-HPV6 L1: 33C, which is a chimeric gene with the C-terminus of HPV6 L1 substituted by the C-terminus of HPV33 L1.

### Example 1.2 Construction of chimeric gene with the C-terminus of HPV11 L1 substituted by the C-terminus of HPV33 L1

The experimental methods and procedures were the same as in Example 1.1, see Appendix 2 for relevant sequences.

### Example 1.3 Construction of a chimeric gene with the C-terminus of HPV16 L1 substituted by the C-terminus of HPV33 L1

The experimental method and procedure were the same as in Example 1.1, see Appendix 3 for relevant sequences.

### Example 1.4 Construction of a chimeric gene with the C-terminus of HPV18 L1 substituted by the C-terminus of HPV33 L1

The experimental method and procedure were the same as in Example 1.1, see Appendix 4 for relevant sequences.

### Example 1.5 Construction of a chimeric gene with the C-terminus of HPV31 L1 substituted by the C-terminus of HPV33 L1

The experimental method and procedure were the same as in Example 1.1, see Appendix 5 for relevant sequences.

### Example 1.6 Construction of HPV33 L1 gene

### 1.6.1 Preparation of PFB-HPV33L1 gene

The HPV33L1 gene with the KpnI and XbaI cleavage sites at both ends of the synthesized sequences was synthesized by Thermo Fisher [formerly Invitrogen (Shanghai) Trading Co.], its sequence is shown in SEQ ID No: 68. The plasmid pcDNA3-HPV33-L1 comprising the nucleotide sequence encoding amino acid 1-499 of HPV33 L1 was obtained by ligating the synthesized gene fragment with pcDNA3 vector (distributor: Thermo Fisher) at KpnI and XbaI cleavage sites.

The obtained pcDNA3-HPV33-L1 plasmid was subjected to double enzyme digestion with KpnI and XbaI to obtain a gene fragment of HPV33 L1 (1-499). The fragment was then ligated with the KpnI / XbaI double digested pFastBac^{™}1 vector (distributor: Thermo Fisher) to obtain a rod vector containing the HPV33 L1 (1-499) gene fragment, named as pFB-HPV33 L1.

### Example 1.7 Construction of a chimeric gene with the C-terminus of HPV35 L1 substituted by the C-terminus of HPV33 L1

The experimental methods and procedures were the same as in Example 1.1, see Appendix 7 for relevant sequences.

### Example 1.8 Construction of chimeric gene with the C-terminus of HPV39 L1 substituted by the C-terminus of HPV59 L1

### 1.8.1 Construction of pFB-HPV39 L1 used as the template

The HPV39 L1 gene with the KpnI and XbaI cleavage sites at both ends of the synthesized sequences was synthesized by Thermo Fisher [formerly Invitrogen (Shanghai) Trading Co.), its sequence is shown as SEQ ID NO: 86. The plasmid pcDNA3-HPV39-L1 containing a nucleotide sequence encoding amino acids 1-505 of HPV39 L1 was obtained by ligating the synthesized gene fragment with pcDNA3 vector (distributor: Thermo Fisher) at KpnI and XbaI cleavage sites.

The pcDNA3-HPV39-L1 plasmid was subjected to double digestion with KpnI and XbaI to obtain a fragment of the HPV39 L1 (1-505) gene. The fragment was then ligated to the KpnI and XbaI double digested pFastBacTM1 vector (distributor: Thermo Fisher) to obtain a rod vector containing the HPV39 L1 (1-505) gene fragment, named as pFB-HPV39 L1.

### 1.8.2 Construction of pFB-HPV59 L1 as the template

The HPV59 L1 gene with the KpnI and XbaI cleavage sites at both ends of the synthesized sequences was synthesized by Thermo Fisher [formerly Invitrogen (Shanghai) Trading Co.), its sequence is shown as SEQ ID NO: 87. The plasmid pcDNA3-HPV59-L1 containing a nucleotide sequence encoding amino acids 1-508 of HPV59 L1 was obtained by ligating the synthesized gene fragment with pcDNA3 vector (distributor: Thermo Fisher) at KpnI and XbaI cleavage sites.

The pcDNA3-HPV59-L1 plasmid was subjected to double digestion with KpnI and XbaI to obtain a fragment of the HPV59 L1 (1-508) gene. The fragment was then ligated to the KpnI and XbaI double digested pFastBacTM1 vector (distributor: Thermo Fisher) to obtain a rod vector containing the HPV59 L1 (1-508) gene fragment, named as pFB-HPV59 L1.

### 1.8.3 Construction of pFB-HPV39 L1: 59C

Chimeric gene with HPV39 L1 C-terminus substituted with HPV59 L1 C-terminus: The constructed recombinant plasmid pFB-HPV39 L1 was used as the gene template to amplify a 1428 bp gene fragment using primers F1 and R1, the primer sequence F1 is shown in SEQ ID No: 88 and the primer sequence R1 is shown in SEQ ID No: 89.

This fragment contains a fragment encoding amino acids 1-469 of HPV39 L1, a 12-base overlapping with a fragment encoding amino acids 471-508 of HPV59 L1 and a segment of the KpnI digest site (GGTAC^C), the amplified sequence is shown in SEQ ID No: 90.

PCR amplification parameters: pre-denaturation at 94°C for 5min; denaturation at 98°C for 10 s, annealing at 69°C for 15 s, 1 kb/1 min at 72°C, for 30 cycles; extension at 72°C for 5min; end at 16°C.

The recombinant plasmid pFB-HPV59 L1 was used as the gene template to amplify a gene fragment of 139 bp in length using primers F2 and R2. The primer sequence F2 is shown in SEQ ID No: 91 and R2 is shown in SEQ ID No: 92.

This gene fragment contains a gene fragment encoding 38 C-terminal amino acids of the HPV59 L1(aa471-508), a 12 bp base overlapping with the gene fragment encoding amino acids 1-469 of HPV39 L1 and the XbaI (T^CTAGA) digest site, and the amplified sequence is shown in SEQ ID No: 93.

PCR amplification parameters: pre-denaturation at 94°C for 5min; denaturation at 98°C for 10 s, annealing at 69°C for 15 s, 1 kb/1 min at 72°C for 30 cycles; extension at 72°C for 5min; end at 16°C.

### PCR ligating sequence

The ligating primers were F1 and R2, and the fragments amplified by using the above primers (F1 and R1 amplified fragments, F2 and R2 amplified fragments) were used as templates.

PCR ligating parameters: pre-denaturation at 94°C for 5 min; denaturation at 98°C for 10 s, annealing at 52°C for 15 s, 72°C for 1 kb/1 min, for 5 cycles; denaturation at 98°C for 10 s, annealing at 68°C for 15 s, 72°C for 1 kb/1 min, for 25 cycles; extension at 72°C for 5 min; end at 16°C.

The final result was SEQ ID NO: 85, a nucleotide sequence encoding amino acids 1-469 of HPV39 L1 and 38 C-terminal amino acids of HPV59 L1(471-508) with KpnI and XbaI enzyme cleavage sites at both ends (hereafter referred to as the ligating sequence).

The recombinant plasmid pFB-HPV39 L1: 59C was obtained by double digesting the pFastBac^{™}1 vector and the ligating sequence fragment with KpnI+XbaI enzymes and the ligating sequence was cloned into the pFastBac^{™}1 vector o obtain pFB-HPV39 L1: 59C, which is a chimeric gene with the C-terminus of HPV39 L1 substituted by the C-terminus of HPV59 L1.

### Example 1.9 Construction of chimeric gene with the C-terminus of HPV45 L1 substituted by the C-terminus of HPV33 L1

The experimental methods and procedures were the same as in Example 1.1, see Appendix 9 for relevant sequences.

### Example 1.10 Construction of a chimeric gene with the C-terminus of HPV51 L1 substituted by the C-terminus of HPV33 L1

The experimental methods and procedures were the same as in Example 1.1, see Appendix 10 for relevant sequences.

### Example 1.11 Construction of a chimeric gene with the C-terminus of HPV52 L1 substituted by the C-terminus of HPV33 L1

The experimental methods and procedures were the same as in Example 1.1, see Appendix 11 for relevant sequences.

### Example 1.12 Construction of a chimeric gene with the C-terminus of HPV56 L1 substituted by the C-terminus of HPV33 L1

The experimental methods and procedures were the same as in Example 1.1, see Appendix 12 for relevant sequences.

### Example 1.13 Construction of a chimeric gene with the C-terminus of HPV58 L1 substituted by the C-terminus of HPV33 L1

The experimental methods and procedures were the same as in Example 1.1, see Appendix 13 for relevant sequences.

### Example 1.14 Construction of HPV59 L1 gene

### Example 1.14.1 Preparation of pFB-HPV59 L1 gene

The HPV59 L1 gene with the KpnI and XbaI cleavage sites at both ends of the synthesized sequences was synthesized by Thermo Fisher [formerly Invitrogen (Shanghai) Trading Co.], its sequence is shown in SEQ ID No: 162. The plasmid pcDNA3-HPV59-L1 comprising the nucleotide sequence encoding amino acid 1-508 of HPV59 L1 was obtained by ligating the synthesized gene fragment with pcDNA3 vector (distributor: Thermo Fisher) at KpnI and XbaI cleavage sites.

The obtained pcDNA3-HPV59-L1 plasmid was subjected to double enzyme digestion with KpnI and XbaI to obtain a gene fragment of HPV59 L1 (1-508). The fragment was then ligated with the KpnI / XbaI double digested pFastBac^{™}1 vector (distributor: Thermo Fisher) to obtain a rod vector containing the HPV59 L1 (1-508) gene fragment, named as pFB-HPV59 L1.

### Example 2 Recombinant baculovirus packaging

### Example 2.1: HPV6 L1: 33C recombinant baculovirus packaging

The recombinant plasmid of pFB-HPV6 L1: 33C constructed in Example 1 was identified and sequenced to be correct, and was transformed into DH10Bac bacteria competent cells (Bac-to-Bac^{®} kit, purchased from Thermo Fisher), incubated at 37°C for proliferation, and incubated in a flat dish for streak culture. White colonies was selected, incubated overnight. The bacterial culture was collected and the recombinant baculovirus DNA was extracted using alkaline lysis method.

The recombinant baculovirus DNA was transfected into insect cells SF9 using a cationic transfection reagent (purchased from Sino Biological) to package the recombinant baculovirus virulent strains. The procedure was as follows:
a. SF9 cells at log phase were inoculated in dishes at a density of 0.6×10⁶ cell/dish. The dish inoculated with SF9 cells were left at room temperature for 2 hrs. for the cell's adhering to the dish wall.
b. 20 µL of extracted plasmid Bacmid DNA was added to 200 µL Grace's Medium (no serum, no additives, purchased from Gibico) and mixed and inverted 5 times.
c. 25 µL of 0.2x TF1 (transfection reagent, purchased from Sino Biological) was added dropwise to 200 µL of Grace's Meduim and mixed gently.
d. Mixed b and c. Incubated at room temperature for 15-45 min.
e. During the incubation of DNA with cellfectin (purchased from Sino Biological), the cell supernatant was discarded and 0.8 mL of Grace Medium( serum additive free) was added into the dish.
f. The incubated mixture of DNA and transfection reagent of d was added to the dish dropwise.
g. Incubated at 27°C for 2 hr.
h. Cell culture medium was discarded and 2.5mL complete growth media /dish (SCD6 SF + 10% FBS) (SCD6 SF was purchased from Sino Biological, FBS was purchased from Gibico) was added.
i. Culture was performed at 27°C for 7 days and whether the viral infection occurred was observed.

Virus supernatant was collected after visible lesions were observed in the transfected cells, typically after 7-11 days of culturing. The viral supernatant, i.e., the P1 generation virus strain of HPV6 L1: 33C is collected aseptically with a pipette. SF9 cells, at a density of 2 × 10⁶ cells/mL, were infected using P1 generation virus strain of HPV6 L1: 33C at a ratio of 1: 50 (V/V), cultured at 27°C for 3 days, and centrifuged at 1000g ± 200g for 10 min at room temperature. The collected virus supernatant was the P2 generation virus and could be used for infecting the host cells and production.

### Example 2.2: Packaging of HPV11 L1: 33C recombinant baculovirus

The experimental methods and procedures were the same as in Example 2.1.

### Example 2.3: Packaging of HPV 16L1: 33C recombinant baculovirus

The experimental methods and procedures are the same as those of Example 2.1.

### Example 2.4: Packaging of HPV18 L1: 33C recombinant baculovirus

The experimental methods and procedures are the same as those of Example 2.1.

### Example 2.5: Packaging of HPV31 L1: 33C recombinant baculovirus

The experimental methods and procedures are the same as those of Example 2.1.

### Example 2.6: Packaging of HPV33 L1 recombinant baculovirus

The experimental methods and procedures are the same as those of Example 2.1.

### Example 2.7: Packaging of HPV35 L1: 33C recombinant baculovirus

The experimental methods and procedures are the same as those of Example 2.1.

### Example 2.8: Packaging of HPV39 L1: 59C recombinant baculovirus

The experimental methods and procedures are the same as those of Example 2.1.

### Example 2.9: Packaging of HPV45 L1: 33C recombinant baculovirus

The experimental methods and procedures are the same as those of Example 2.1.

### Example 2.10: Packaging of HPV51 L1: 33C recombinant baculovirus

The experimental methods and procedures are the same as those of Example 2.1.

### Example 2.11: Packaging of HPV52 L1: 33C recombinant baculovirus

The experimental methods and procedures are the same as those of Example 2.1.

### Example 2.12: Packaging of HPV56 L1: 33C recombinant baculovirus

The experimental methods and procedures are the same as those of Example 2.1.

### Example 2.13: Packaging of HPV58 L1: 33C recombinant baculovirus

The experimental methods and procedures were the same as those of Example 2.1.

### Example 2.14: Packaging of HPV59 L1 recombinant baculovirus

The experimental methods and procedures were the same as those of Example 2.1.

### Example 3 Expression of chimeric proteins or proteins

### Example 3.1: Expression of HPV6 L1: 33C

High Five cells were infected with baculovirus containing the HPV6 L1: 33C recombinant gene obtained in Example 2 at a ratio of 1: 200 (V/V), and the cell precipitate was collected by centrifugation at 1000g ± 100g at room temperature. The cells were broken up by sonication at low temperature for 3 min, centrifuged at >10,000g for 10 min and the supernatant was collected for SDS-PAGE. Lane 1: Marker (The marker is a mixture of 7 purified proteins with molecular weights ranging from 14.4 kDa to 116 kDa, produced by Thermo Scientific); Lane 2: cell lysate; Lane 3: supernatant of the lysate collected by centrifugation.

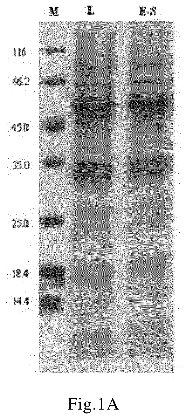

The result is shown in **Figure 1A****.** The HPV6 L1: 33C L1 protein prepared by this method has a yield of >100 mg/L and a protein size of approximate 56 KD, which can be used for mass production.

### Example 3.2: Expression and production of HPV11 L1: 33C

The experimental methods and procedures were the same as in Example 3.1.

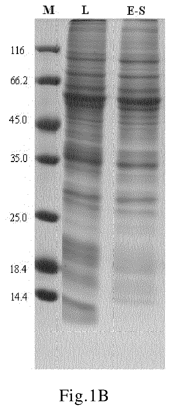

The result is shown in **Figure 1B****.** The HPV11 L1: 33C L1 protein prepared by this method has a yield of >100 mg/L and a protein size of approximate 56 KD, which can be used for mass production.

### Example 3.3: Expression and production of HPV 16L1: 33C

The experimental methods and procedures were the same as in Example 3.1.

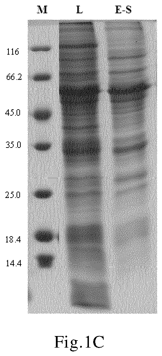

The result is shown in **Figure 1C****.** The HPV 16L1: 33C L1 protein prepared by this method has a yield of > 100 mg/L and a protein size of approximate 56 KD, which can be used for mass production.

### Example 3.4: Expression and production of HPV18 L1: 33C

The experimental methods and procedures were the same as in Example 3.1.

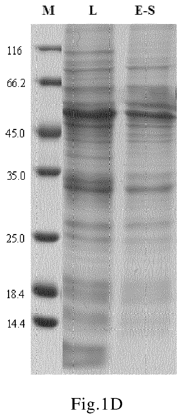

The result is shown in **Figure 1D****.** The HPV18 L1: 33C L1 protein prepared method has a yield of > 100 mg/L and a protein size of approximate 56 KD, which can be used for mass production.

### Example 3.5: Expression and production of HPV31 L1: 33C

The experimental methods and procedures were the same as in Example 3.1.

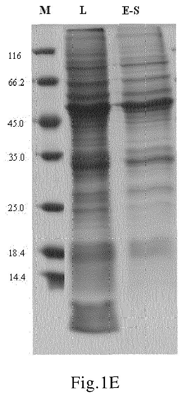

The result is shown in **Figure 1E****.** The HPV31 L1: 33C L1 protein prepared by this method has a yield of >100 mg/L and a protein size of approximate 56 KD, which can be used for mass production.

### Example 3.6: Expression and production of HPV33 L1

The experimental methods and procedures were the same as in Example 3.1.

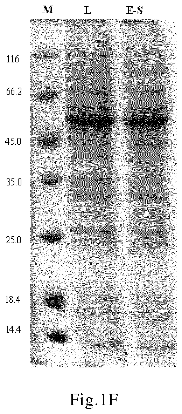

The result is shown in **Figure 1F****.** The HPV33 L1 L1 protein prepared by this method has a yield of > 100 mg/L and a protein size of approximate 56 KD, which can be used for mass production.

### Example 3.7: Expression and production of HPV35 L1: 33C

The experimental methods and procedures were the same as in Example 3.1.

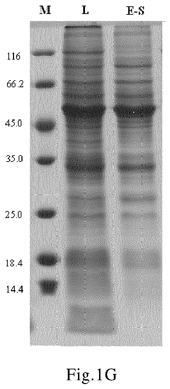

The result is shown in **Figure 1G****.** The HPV35 L1: 33C L1 protein prepared by this method has a yield of >100 mg/L and a protein size of approximate 56 KD, which can be used for mass production.

### Example 3.8: Expression and production of HPV39 L1: 59C

The experimental methods and procedures were the same as in Example 3.1.

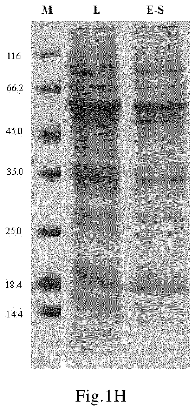

The result is shown in **Figure 1H****.** The HPV39 L1: 59C L1 protein prepared by this method has a yield of >100 mg/L and a protein size of approximate 56 KD, which can be used for mass production.

### Example 3.9: Expression and production of HPV45 L1: 33C

The experimental methods and procedures were the same as in Example 3.1.

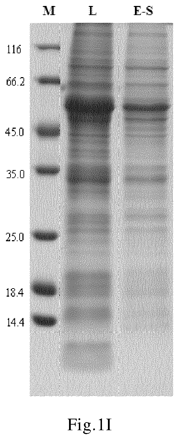

The result is shown in **Figure 1I****.** The HPV45 L1: 33C L1 protein prepared by this method has a yield of >100 mg/L and a protein size of approximate 56 KD, which can be used for mass production.

### Example 3.10: Expression and production of HPV51 L1: 33C

The experimental methods and procedures were the same as in Example 3.1.

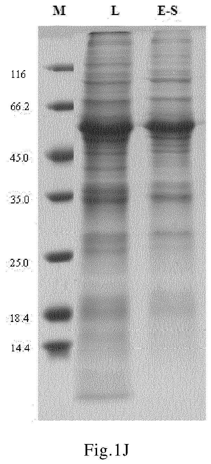

The result is shown in **Figure 1J****.** The HPV51 L1: 33C L1 protein prepared by this method has a yield of >100 mg/L and a protein size of approximate 56 KD, which can be used for mass production.

### Example 3.11: Expression and production of HPV52 L1: 33C

The experimental methods and procedures were the same as in Example 3.1.

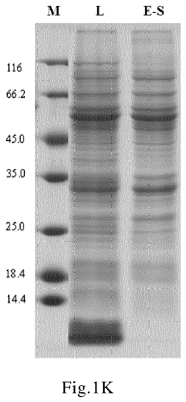

The result is shown in **Figure 1K****.** The HPV52 L1: 33C L1 protein prepared by this method has a yield of >100 mg/L and a protein size of approximate 56 KD, which can be used for mass production.

### Example 3.12: Expression and production of HPV56 L1: 33C

The experimental methods and procedures were the same as in Example 3.1.

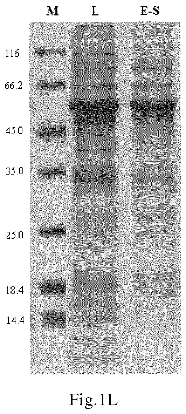

The result is shown in **Figure 1L****.** The HPV56 L1: 33C L1 protein prepared by this method has a yield of >100 mg/L and a protein size of approximate 56 KD, which can be used for mass production.

### Example 3.13: Expression and production of HPV58 L1: 33C

The experimental methods and procedures were the same as in Example 3.1.

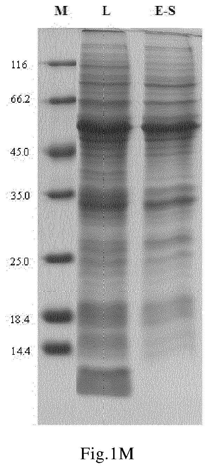

The result is shown in **Figure 1M****.** The HPV58 L1: 33C L1 protein prepared by this method has a yield of >100 mg/L and a protein size of approximate 56 KD, which can be used for mass production.

### Example 3.14: Expression and production of HPV59 L1

The experimental methods and procedures were the same as in Example 3.1.

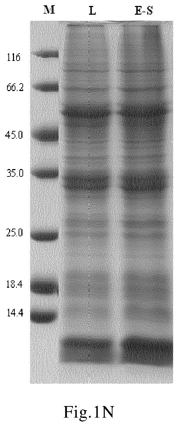

The result is shown in **Figure 1N****.** The HPV59 L1 L1 protein prepared by this method has a yield of > 100 mg/L and a protein size of approximate 56 KD, which can be used for mass production.

### Example 4 Preparation of purified virus-like particles

### Example 4.1: Preparation of purified HPV6 L1: 33C virus-like particles

The HPV6 L1: 33C virus-like particles were purified by a two-step chromatography method, i.e. HS-MMA method, the supernatant collected in Example 3 was purified, and finally, high purity virus-like particles were obtained.

### First step chromatography:

Medium: POROS^{®} 50 HS strong cation exchange media produced by Thermo Fisher was used.

Medium volume: 150 mL of media volume, 30 mL/min of linear flow rate.

Chromatography conditions: equilibration buffer (pH 6.2, the salt concentration is 50 mM phosphate, 0.5 M NaCl); wash buffer (the salt concentration is 50 mM phosphate, 0.75 M NaCl, pH 6.2).

The chromatography column was first equilibrated with 5 CV of equilibration buffer and then the sample was loaded. After loading, the column was then eluted with 5 CV of equilibration buffer and wash buffer, respectively, to remove the protein impurities.

Elution conditions: a 50 mM phosphate buffer containing 50 mM arginine hydrochloride, pH 6.2, with an elution salt concentration being of 1.25 M NaCl, was used.

Second step chromatography.

Medium: MMA ion exchange media produced by Bestchrom (Shanghai) Biosciences Co., Ltd was used.

Medium volume: media volume is150 mL, while linear flow rate is 30 mL/min.

Chromatography conditions: equilibration buffer: 50 mM PB, 1.25 M NaCl, pH 6.2. The chromatography column was first equilibrated with 4 CV equilibration buffer and then the sample was loaded. After loading, protein impurities were rinsed off with 5 CV equilibration buffer and then the target protein was eluted with elution buffer and collected.

Elution conditions: 100 mM NaAC, 150 mM NaCl, 0.01% Tween 80, pH 4.5.

### Example 4.2: Preparation of purified HPV11 L1: 33C virus-like particles

The experimental methods and procedures were the same as in Example 4.1.

### Example 4.3: Purification and preparation of HPV 16L1: 33C virus-like particles

The experimental methods and procedures were the same as in Example 4.1.

### Example 4.4: Purification and preparation of HPV18 L1: 33C virus-like particles

The experimental methods and procedures were the same as in Example 4.1.

### Example 4.5: Purification and preparation of HPV31 L1: 33C virus-like particles

The experimental methods and procedures were the same as in Example 4.1.

### Example 4.6: Preparation of purified HPV33 L1 virus-like particles

The experimental methods and procedures were the same as in Example 4.1.

### Example 4.7: Preparation of purified HPV35 L1: 33C virus-like particles

The experimental methods and procedures were the same as in Example 4.1.

### Example 4.8: Preparation of purified HPV39 L1: 59C virus-like particles

The experimental methods and procedures were the same as in Example 4.1.

### Example 4.9: Preparation of purified HPV45 L1: 33C virus-like particles

The experimental methods and procedures were the same as in Example 4.1.

### Example 4.10: Preparation of purified HPV51 L1: 33C virus-like particles

The experimental methods and procedures were the same as in Example 4.1.

### Example 4.11: Preparation of purified HPV52 L1: 33C virus-like particles

The experimental methods and procedures were the same as in Example 4.1.

### Example 4.12: Preparation of purified HPV56 L1: 33C virus-like particles

The experimental methods and procedures were the same as in Example 4.1.

### Example 4.13: Preparation of purified HPV58 L1: 33C virus-like particles

The experimental methods and procedures were the same as in Example 4.1.

### Example 4.14: Preparation of purified HPV59 L1 virus-like particles

The experimental methods and procedures were the same as in Example 4.1.

### Example 5 Morphological observation of virus-like particles

### Example 5.1: Morphological observation of HPV6 L1: 33C virus-like particles

10 µL sample was taken for transmission electron microscopy. The sample was fixed onto a carbon coated copper grid for 2 min, the rest liquid was absorbed off with filter paper, and then stained twice with phosphotungstic acid (Beijing Electron Microscopy China Technology Co., Ltd., concentration 2%, pH 6.5) for 30 seconds each time, the rest staining solution was absorbed off with filter paper, left the sample for drying, then performed transmission electron microscopy observation. The transmission electron microscope (Brand: Hitachi, Model No.: H-7650) was 80KV with a magnification of 80,000x.

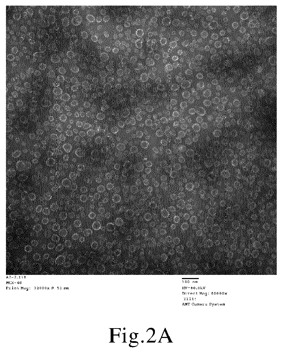

The electron microscopy observation is shown in **Figure 2A****.** As can be seen in **Figure 2A****,** the C-terminal-modified HPV6 L1: 33C can form uniform-sized virus-like particles with an average diameter of approximate 60 nm.

### Example 5.2: Morphological observation of HPV11 L1: 33C virus-like particles

The experimental methods and procedures were the same as in Example 5.1.

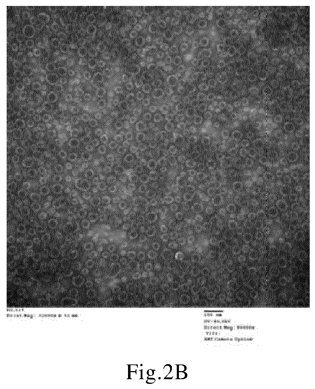

The electron microscopy observation is shown in **Figure 2B****.** As can be seen in **Figure 2B****,** the C-terminal-modified HPV11 L1: 33C can form uniform-sized virus-like particles, with an average diameter of approximate 60 nm.

### Example 5.3: Morphological observation of HPV 16L1: 33C virus-like particles

The experimental methods and procedures were the same as in Example 5.1.

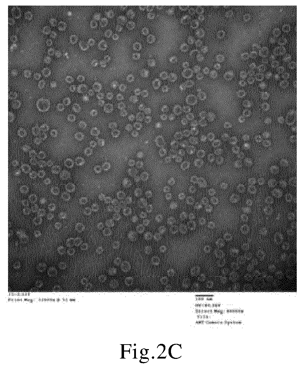

The electron microscopy observation is shown in **Figure 2C****.** As can be seen in **Figure 2C****,** the C-terminal-modified HPV 16L1: 33C can form uniform-sized virus-like particles with an average diameter of approximate 60 nm.

### Example 5.4: Morphological observation of HPV18 L1: 33C virus-like particles

The experimental methods and procedures were the same as in Example 5.1.

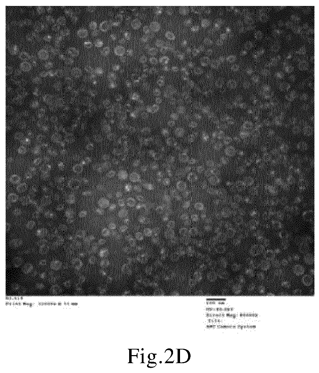

The electron microscopy observation is shown in **Figure 2D****.** As can be seen in **Figure 2D****,** the C-terminal-modified HPV18 L1: 33C can form uniform-sized virus-like particles with an average diameter of approximate 60 nm.

### Example 5.5: Morphological observation of HPV31 L1: 33C virus-like particles

The experimental methods and procedures were the same as in Example 5.1.

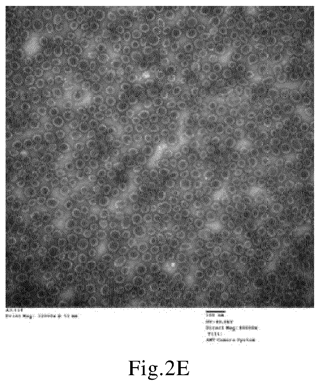

The electron microscopy observation is shown in **Figure 2E****.** As can be seen in **Figure 2E****,** the C-terminal-modified HPV31 L1: 33C can form uniform-sized virus-like particles with an average diameter of approximate 60 nm.

### Example 5.6: Morphological observation of HPV33 L1 virus-like particles

The experimental methods and procedures were the same as in Example 5.1.

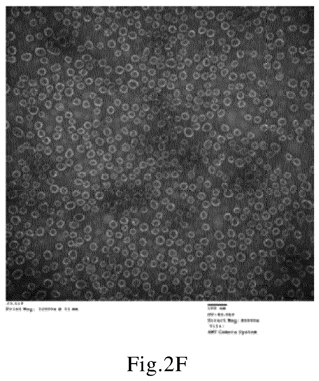

The electron microscopy observation is shown in **Figure 2F****.** As can be seen in **Figure 2F****,** the C-terminally modified HPV33 L1 can form uniform-sized virus-like particles with an average diameter of approximate 60 nm.

### Example 5.7: Morphological observation of HPV35 L1: 33C virus-like particles

The experimental methods and procedures were the same as in Example 5.1.

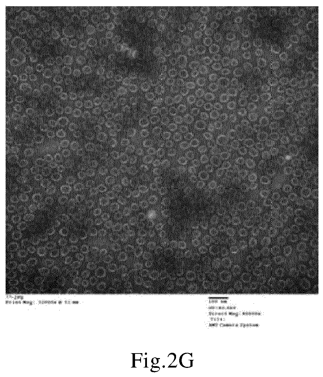

The electron microscopy observation is shown in **Figure 2G****.** As can be seen in **Figure 2G****,** the C-terminally modified HPV35 L1: 33C can form uniform-sized virus-like particles with an average diameter of approximate 60 nm.

### Example 5.8: Morphological observation of HPV39 L1: 59C virus-like particles

The experimental methods and procedures were the same as in Example 5.1.

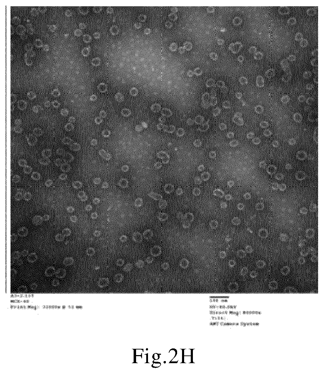

The electron microscopy observation is shown in **Figure 2H****.** As can be seen in **Figure 2H****,** the C-terminal-modified HPV39 L1: 59C can form uniform-sized virus-like particles with an average diameter of approximate 60 nm.

### Example 5.9: Morphological observation of HPV45 L1: 33C virus-like particles

The experimental methods and procedures were the same as in Example 5.1.

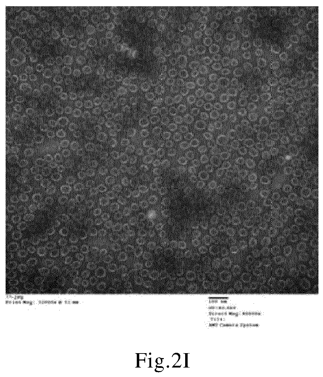

The electron microscopy observation is shown in **Figure 2I**. As can be seen in **Figure 2I**, the C-terminal-modified HPV45 L1: 33C can form uniform-sized virus-like particles with an average diameter of approximate 60 nm.

### Example 5.10: Morphological observation of HPV51 L1: 33C virus-like particles

The experimental methods and procedures were the same as in Example 5.1.

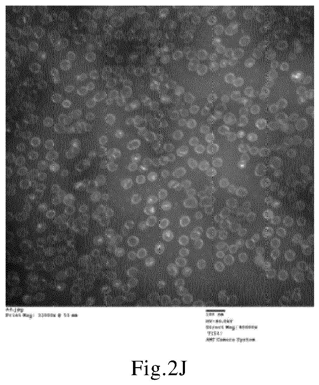

The electron microscopy observation is shown in **Figure 2J****.** As can be seen in **Figure 2J****,** the C-terminal-modified HPV51 L1: 33C can form uniform-sized virus-like particles with an average diameter of approximate 60 nm.

### Example 5.11: Morphological observation ofHPV52 L1: 33C virus-like particles

The experimental methods and procedures were the same as in Example 5.1.

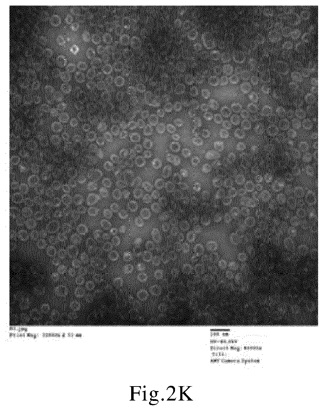

The electron microscopy observation is shown in **Figure 2K****.** As can be seen in **Figure 2K****,** the C-terminal-modified HPV52 L1: 33C can form uniform-sized virus-like particles with an average diameter of approximate 60 nm.

### Example 5.12: Morphological observation of HPV56 L1: 33C virus-like particles

The experimental methods and procedures were the same as in Example 5.1.

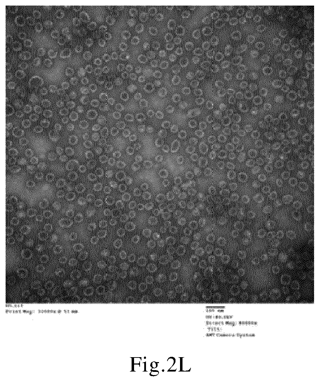

The electron microscopy observation is shown in **Figure 2L**. As can be seen in **Figure 2L**, the C-terminal-modified HPV56 L1: 33C can form uniform-sized virus-like particles with an average diameter of approximate 60 nm.

### Example 5.13: Morphological observation of HPV58 L1: 33C virus-like particles

### The experimental methods and procedures were the same as in Example 5.1.

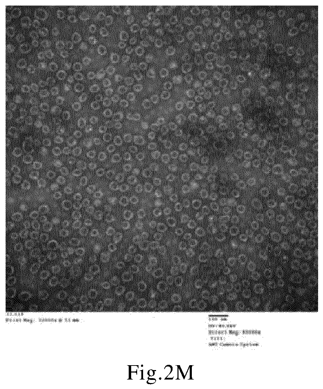

The electron microscopy observation is shown in **Figure 2M****.** As can be seen in **Figure 2M****,** the C-terminal-modified HPV58 L1: 33C can form uniform-sized virus-like particles with an average diameter of approximate 60 nm.

### Example 5.14: Morphological observation of HPV59 L1 virus-like particles

The experimental methods and procedures were the same as in Example 5.1.

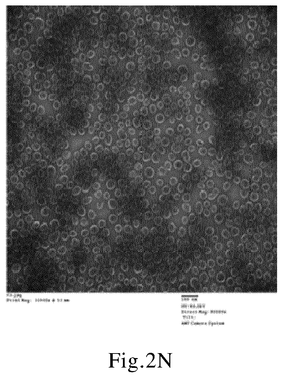

The electron microscopy observation is shown in **Figure 2N****.** As can be seen in **Figure 2N****,** the C-terminal-modified HPV59 L1 : 33C
can form uniform-sized virus-like particles with an average diameter of approximate 60 nm.

### Example 6 Immunogenicity evaluation of virus-like particles in animals

### Example 6.1: Immunogenicity evaluation of HPV6 L1: 33C virus-like particles in animals

### 6.1.1 Modeling of pseudovirus-neutralizing cells

As HPV is difficult to be cultured *in vitro* and has a strong host specificity, it is difficult to be reproduced in organisms other than humans, thus there is a lack of suitable animal models. Therefore, there is a need to establish suitable and effective *in vitro* neutralization experimental models for the evaluation of vaccine immunoprotectivity.

HPV pseudovirus is an ideal model for HPV *in vitro* neutralization: Thanks to the HPV VLP's characteristic of non-specifically encapsulating nucleic acids, HPV pseudovirus can be formed from the VLPs, composed of HPV L1 and L2 expressed in cells, by encapsulating free DNA or introducing exogenous plasmid.

The immunogenicity of immunized animal serum samples was analyzed by pseudovirus neutralization assay. The animal immunized with HPV6 virus-like particles can produce neutralizing antibodies against HPV6 which can neutralize HPV6 pseudovirus. When the immunized animal serum is incubated with a certain amount of pseudovirus and then infects cells, the number of cells capable of expressing GFP fluorescence decreases when neutralization antibodies in the serum increases, showing a linear negative correlation in a certain range, so the neutralizing activity of antibodies in the serum can be evaluated by detecting the change in the number of cells expressing GFP.

Construction method of pseudovirus: The HPV6 pCMV3-3-HPV6 L1+L2 (L1 sequence was from Uniprot P69898, L2 sequence was from Uniprot Q84297) plasmid (purchased from Sino Biological) and the fluorescent plasmid (PSEU-GFP Spark, purchased from Sino Biological) were co-transfected into 293FT adherent cells (purchased from Thermo Fisher). The specific methods refer to the published literature (Pastrana D V, Buck C B, Pang Y S, Thompson C D, Castle P E, FitzGerald P C, Kjaer S K, Lowy D R, Schiller J T. Reactivity of human sera in a sensitive, high-throughput pseudovirus-based papilloma virus neutralization assay for HPV16 and HPV18. [J] Virology 2004,321:205-216.). The pseudovirus supernatant was collected and aliquoted, and stored in a -80°C refrigerator for stock.

### 6.1.2 Immunoprotective evaluation of HPV6 L1: 33C virus-like particles in animals

### Immunization procedures in mice:

HPV6 L1: 33C virus-like particles were absorbed onto aluminum phosphate adjuvant, mixed, and used to immunize mice at a dose of 0.15 µg/200 µL per mouse, 10 mice in total. The mice were immunized with the diluted samples on Days 0, 7 and 21, with control mice immunized with blank serum. Blood was collected from the eyes of the mice on Day 28 and the sera were isolated for pseudovirus neutralization titers assay.

### EC50 assay of mice:

The murine serum was inactivated at 56°C for 30 minutes, centrifuged at 6000g, 5 mins, and the supernatant was collected for assaying. 4-8 hours prior to the assay, 293FT cells were inoculated at a density of 15,000 cells/well into 96-well plates and incubated at 37°C in a CO₂ incubator with 5% CO₂. The post-immune murine serum and blank control serum were serial diluted with neutralizing media respectively, then mixed with the HPV6 pseudovirus prepared in 6.1 at a volume ratio of 1:1, incubated at 2-8°C for 1 hrs. , then 100 µL/well of the mixture were added to 293FT cells, which had been inoculated for 4-8 hrs in advance. Each sample was in replicate, and blank serum control group, pseudovirus positive control group and pseudovirus negative control group were used. The cells infected by pseudovirus were incubated at 37°C in a CO₂ incubator with 5% CO₂ for 62-96 hrs , fluorescence scanning photographed and counted in an ELISPOT analyser (Model No.: S6 Universal-V Analyzer, Manufacturer: CTL). On the basis of the neutralization inhibition of each murine serum sample, the maximum dilution of the serum at 50% neutralization inhibition was calculated for each murine serum sample according to the Reed-Muench method, i.e. the half efficacy dilution EC50.

The results of the HPV6 serum pseudovirus neutralization titer assay are detailed in **Table 4.**

**Table 4 EC50 of Serum Neutralization Titer Assay in Mice (GMT±SEM)**

| HPV Type | EC₅₀ Value |
|---|---|
| Type 6 | 4948±1831 |

| | |
|---|---|
| Notes: 1. number of animals, N = 10. 2. GMT (Geometric Mean Titer): geometric mean titer. 3. SEM (Standard Error of Mean): standard error. | |

The above evaluation results show that the HPV6 L1: 33C virus-like particles have good immunogenicity and can produce neutralizing antibodies with high titers in animals, which can be used to prepare into a vaccine for preventing HPV infections.

### Example 6.2: Evaluation of immunogenicity of HPV11 L1: 33C virus-like particles in animals

The experimental methods and procedures were the same as in Example 6.1. The L1 sequence was from Uniprot P04012 and the L2 sequence was from Uniprot P04013.

The results of HPV11 serum pseudovirus neutralization titer assay are detailed in **Table 5.**

**Table 5 EC50 of Serum Neutralization Titer Assay in Mice (GMT±SEM)**

| HPV Type | EC₅₀ Value |
|---|---|
| Type 11 | 15024±8400 |

The above evaluation results show that the HPV11 L1: 33C virus-like particles have good immunogenicity, can produce high titers of neutralizing antibodies in animals, and can be used to prepare into a vaccine for the prevention of HPV infection.

### Example 6.3: Evaluation of immunogenicity of HPV 16L1: 33C virus-like particles in animals

The experimental methods and procedures were the same as in Example 6.1. The L1 sequence was from Uniprot P03101 and the L2 sequence was from Uniprot P03107.

The results of HPV16 serum pseudovirus neutralization titer assay are detailed in **Table 6.**

**Table 6 EC50 of Serum Neutralization Titer Assay in Mice (GMT±SEM)**

| HPV Type | EC₅₀ Value |
|---|---|
| Type 16 | 32449±7224 |

The above evaluation results show that the HPV 16L1: 33C virus-like particles have good immunogenicity and can produce high titers of neutralizing antibodies in animals, which can be used to prepare into a vaccine for the prevention of HPV infection.

### Example 6.4: Evaluation of immunogenicity of HPV18 L1: 33C virus-like particles in animals

The experimental methods and procedures were the same as in Example 6.1. The L1 sequence was from Uniprot Q80B70 and the L2 sequence was from Uniprot P06793.

The results of HPV18 serum pseudovirus neutralization titer assay are detailed in **Table 7.**

**Table 7 EC50 of Serum Neutralization Titer Assay in Mice (GMT±SEM)**

| HPV Type | EC₅₀ Value |
|---|---|
| Type 18 | 18480±4051 |

The above evaluation results show that the HPV18 L1: 33C virus-like particles have good immunogenicity, can produce high titers of neutralizing antibodies in animals, and can be used to prepare into a vaccine for preventing HPV infection.

### Example 6.5: Evaluation of immunogenicity of HPV31 L1: 33C virus-like particles in animals

The experimental methods and procedures were the same as in Example 6.1. The L1 sequence was from Uniprot P17388 and the L2 sequence was from Uniprot P17389.

The results of HPV31 serum pseudovirus neutralization titer assay are detailed in **Table 8.**

**Table 8 EC50 of Serum Neutralization Titer Assay in Mice (GMT±SEM)**

| HPV Type | EC₅₀ Value |
|---|---|
| Type 31 | 5210±1147 |

The above evaluation results show that the HPV31 L1: 33C virus-like particles have good immunogenicity, can produce high titers of neutralizing antibodies in animals, and can be used to prepare into a vaccine for preventing HPV infection.

### Example 6.6: Evaluation of immunogenicity of HPV33 L1 virus-like particles in animals

The experimental methods and procedures were the same as in Example 6.1. The L1 sequence was from Uniprot P06416 and the L2 sequence was from Uniprot P06418.

The results of HPV33 serum pseudovirus neutralization titer assay are detailed in **Table 9.**

**Table 9 EC50 of Serum Neutralization Titer Assay in Mice (GMT±SEM)**

| HPV Type | EC₅₀ Value |
|---|---|
| Type 33 | 4666±1555 |

The above evaluation results show that the HPV33 L1 virus-like particles have good immunogenicity, can produce high titers of neutralizing antibodies in animals, and can be used to prepare into a vaccine for preventing HPV infection.

### Example 6.7: Evaluation of immunogenicity of HPV35 L1: 33C virus-like particles in animals

The experimental methods and procedures were the same as in Example 6.1. The L1 sequence was from Uniprot P27232 and the L2 sequence was from Uniprot P27234.

The results of HPV35 serum pseudovirus neutralization titer assay are detailed in **Table 10.**

**Table 10 EC50 of Serum Neutralization Titer Assay in Mice (GMT±SEM)**

| HPV Type | EC₅₀ Value |
|---|---|
| Type 35 | 2293±1448 |

The above evaluation results show that the HPV35 L1: 33C virus-like particles have good immunogenicity, can produce high titers of neutralizing antibodies in animals, and can be used to prepare into a vaccine for preventing HPV infection.

### Example 6.8: Immunogenicity evaluation of HPV39 L1: 59C virus-like particles in animals

The experimental methods and procedures were the same as in Example 6.1. The L1 sequence was from Uniprot P24838 and the L2 sequence was from Uniprot P24839.

The results of HPV39 serum pseudovirus neutralization titer assay are detailed in **Table 11.**

**Table 11 EC50 of Serum Neutralization Titer Assay in Mice (GMT±SEM)**

| HPV Type | EC₅₀ Value |
|---|---|
| Type 39 | 25526±5857 |

The above evaluation results show that the HPV39 L1: 59C virus-like particles have good immunogenicity, can produce high titers of neutralizing antibodies in animals, and can be used to prepare into a vaccine for preventing HPV infection.

### Example 6.9: Immunogenicity evaluation of HPV45 L1: 33C virus-like particles in animals

The experimental methods and procedures were the same as in Example 6.1. The L1 sequence was from Uniprot P36741 and the L2 sequence was from Uniprot P36761.

The results of HPV45 serum pseudovirus neutralization titer assay are detailed in **Table 12.**

**Table 12 EC50 of Serum Neutralization Titer Assay in Mice (GMT±SEM)**

| HPV Type | EC₅₀ Value |
|---|---|
| Type 45 | 755±935 |

The above evaluation results show that the HPV45 L1: 33C virus-like particles have good immunogenicity, can produce high titers of neutralizing antibodies in animals, and can be used to prepare into a vaccine for preventing HPV infection.

### Example 6.10: Evaluation of immunogenicity of HPV51 L1: 33C virus-like particles in animals

The experimental methods and procedures were the same as in Example 6.1. The L1 sequence was from Uniprot P26536 and the L2 sequence was from Uniprot P26539.

The results of HPV51 serum pseudovirus neutralization titer assay are detailed in **Table 13.**

**Table 13 EC50 of Serum Neutralization Titer Assay in Mice (GMT±SEM)**

| HPV Type | EC₅₀ Value |
|---|---|
| Type 51 | 5528±1572 |

The above evaluation results show that the HPV51 L1: 33C virus-like particles have good immunogenicity, can produce high titers of neutralizing antibodies in animals, and can be used to prepare into a vaccine for preventing HPV infection.

### Example 6.11: Evaluation of immunogenicity of HPV52 L1: 33C virus-like particles in animals

The experimental methods and procedures were the same as in Example 6.1. The L1 sequence was from Uniprot Q05138 and the L2 sequence was from Uniprot F8S4U2.

The results of HPV52 serum pseudovirus neutralization titer assay are detailed in **Table 14.**

**Table 14 EC50 of Serum Neutralization Titer Assay in Mice (GMT±SEM)**

| HPV Type | EC₅₀ Value |
|---|---|
| Type 52 | 19019±8604 |

The above evaluation results show that the HPV52 L1: 33C virus-like particles have good immunogenicity, can produce high titers of neutralizing antibodies in animals, and can be used to prepare into a vaccine for preventing HPV infection.

### Example 6.12: Evaluation of immunogenicity of HPV56 L1: 33C virus-like particles in animals

The experimental methods and procedures were the same as in Example 6.1. The L1 sequence was from Uniprot P36743 and the L2 sequence was from Uniprot P36765.

The results of HPV56 serum pseudovirus neutralization titer assay are detailed in **Table 15.**

**Table 15 EC50 of Serum Neutralization Titer Assay in Mice (GMT±SEM)**

| HPV Type | EC₅₀ Value |
|---|---|
| Type 56 | 2497±612 |

The above evaluation results show that the HPV56 L1: 33C virus-like particles have good immunogenicity, can produce high titers of neutralizing antibodies in animals, and can be used to prepare into a vaccine for preventing HPV infection.

### Example 6.13: Evaluation of immunogenicity of HPV58 L1: 33C virus-like particles in animals

The experimental methods and procedures were the same as in Example 6.1. The L1 sequence was from Uniprot P26535 and the L2 sequence was from Uniprot B6ZB12.

The results of HPV58 serum pseudovirus neutralization titer assay are detailed in **Table 16.**

**Table 16 EC50 of Serum Neutralization Titer Assay in Mice (GMT±SEM)**

| HPV Type | EC₅₀ Value |
|---|---|
| Type 58 | 19939±8459 |

The above evaluation results show that the HPV58 L1: 33C virus-like particles have good immunogenicity, can produce high titers of neutralizing antibodies in animals, and can be used to prepare into a vaccine for the prevention of HPV infection.

### Example 6.14: Evaluation of immunogenicity of HPV59 L1 virus-like particles in animals

The experimental methods and procedures were the same as in Example 6.1. The L1 sequence was from Uniprot Q81971 and the L2 sequence was from Uniprot Q81970.

The results of HPV59 serum pseudovirus neutralization titer assay are detailed in **Table 17.**

**Table 17 EC50 of Serum Neutralization Titer Assay in Mice (GMT±SEM)**

| HPV Type | EC₅₀ Value |
|---|---|
| Type 59 | 1344±335 |

The above evaluation results show that the HPV59 L1 virus-like particles have good immunogenicity, can produce high titers of neutralizing antibodies in animals, and can be used to prepare into a vaccine for the prevention of HPV infection.

### Example 6.15: Immunogenicity evaluation of 14-valent immunogenic composition of virus-like particles in mice

### Immunization procedures in mice:

A sample consisted of the aforementioned virus-like particles of 14 types (HPV Types 6, 11, 16, 18, 31, 33, 35, 39, 45, 51, 52, 56, 58, and 59) were diluted with aluminum phosphate adjuvant sterilely (for example, the HPV types which were of 20 µg in the sample, were diluted 80-fold to 0.5 µg/mL, the concentration of other HPV types in the samples being of different ration varied accordingly ), 200 µL of the diluted virus-like particle sample was used to immunize each mouse, and the doses are shown in **Table 18.** SPF-grade female Balb/c mice aged 6-8 weeks were divided into groups of 10 mice in each group (experimental group and control group), and were immunized with the diluted samples on Day 0, 7 and 21 respectively, with control mice immunized with blank serum. Blood was collected from the eyes of mice on Day 28, and the sera were isolated for pseudovirus neutralization titers assay.

**Table 18 Doses of Each HPV Types in the Immunogenic Composition for Immunization in Each Mouse (µg)**

| **HPV Type** | **6** | **11** | **16** | **18** | **31** | **33** | **45** | **52** | **58** | **35** | **39** | **51** | **56** | **59** |
|---|---|---|---|---|---|---|---|---|---|---|---|---|---|---|
| Dose | 0.15 | 0.2 | 0.3 | 0.2 | 0.1 | 0.1 | 0.1 | 0.1 | 0.1 | 0.1 | 0.1 | 0.1 | 0.1 | 0.1 |

### Serum neutralization titer assay in immunized mice by pseudovirus neutralization assay:

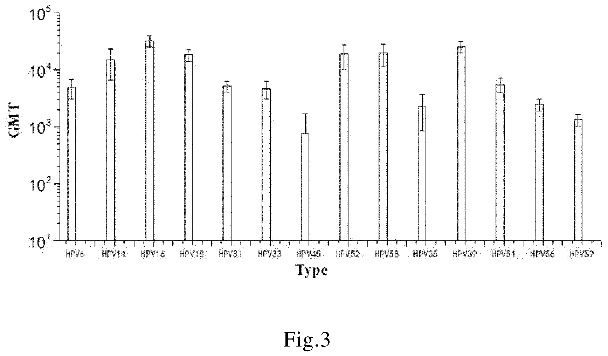

The murine serum was inactivated at 56°C for 30 minutes, centrifuged at 6000g, 5 mins, and the supernatant was collected for assaying. 4-8 hours prior to the assay, 293FT cells were inoculated at a density of 15,000 cells/well into 96-well plates and incubated at 37°C in a CO₂ incubator with 5% CO₂. The immunized murine serum and blank control serum were serial diluted with neutralizing media respectively, then mixed with the diluted 14 types of pseudovirus samples (the 14 types of HPV pseudovirus are those of HPV types 6, 11, 16, 18, 31, 33, 35, 39, 45, 51, 52, 56, 58 and 59, prepared in Examples 6.1-**6.14)** at a volume ratio of 1:1, incubated at 2-8°C for 1 h, then 100 µL/well of the mixture were added to 293FT cells, which had been inoculated for 4-8 h in advance. Each sample was in replicate; and blank serum control group, pseudovirus positive control group and pseudovirus negative control group were used. The cells infected by pseudovirus were incubated at 37°C in a CO₂ incubator with 5% CO₂ for 62-96 h, fluorescence scanning photographed and counted in an ELISPOT analyser (Model No.: S6 Universal-V Analyzer, Manufacturer: CTL). On the basis of the neutralization inhibition of each murine serum sample, the maximum dilution of the serum at 50% neutralization inhibition was calculated for each murine serum sample according to the Reed-Muench method, i.e. the half efficacy dilution EC50. The results are shown in **Figure 3****.**

As demonstrated **Figure 3****,** the 14-valent immunogenic composition of virus-like particles can produce high titers of neutralizing antibodies in animals, and can be used to prepare a vaccine for the prevention of HPV infection.

### Comparative Example 1: Expression of C-terminal truncated HPV16 L1 (aa 1-474)

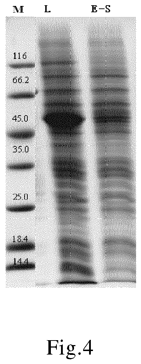

The inventors attempted to truncate the C-terminus of HPV16 L1 by 31 amino acids and named it HPV16 L1 (1-474) (SEQ ID NO: 27). However, it was found in the study that the truncated HPV16 L1 (1-474) protein was highly expressed but has very poor solubility, and is difficult to extract and purify, the detailed results of expression and extraction are shown in **Figure 4****.**

### Appendix 1 : SEQUENCE LISTING -CHIMERIC HUMAN PAPILLOMAVIRUS TYPE 6 L1 PROTEIN

| | |
|---|---|
| SEQ ID No: 1 | 0601The amino acid sequence of HPV type 6 L1 protein aa 1-469 |
| | MWRPSDSTVYVPPPNPVSKVVATDAYVTRTNIFYHASSSRLLAVGHPYFSIKRA NKTVVPKVSGYQYRVFKVVLPDPNKFALPDSSLFDPTTQRLVWACTGLEVGRG QPLGVGVSGHPFLNKYDDVENSGSGGNPGQDNRVNVGMDYKQTQLCMVGCA PPLGEHWGKGKQCTNTPVQAGDCPPLELITSVIQDGDMVDTGFGAMNFADLQT NKSDVPIDICGTTCKYPDYLQMAADPYGDRLFFFLRKEQMFARHFFNRAGEVG EPVPDTLIIKGSGNRTSVGSSIYVNTPSGSLVSSEAQLFNKPYWLQKAQGHNNGI CWGNQLFVTVVDTTRSTNMTLCASVTTSSTYTNSDYKEYMRHVEEYDLQFIFQ LCSITLSAEVMAYIHTMNPSVLEDWNFGLSPPPNGTLEDTYRYVQSQAITCQKP TPEKEKPDPYKNLSFWEVNLKEKFSSELDQYPLGRKFLLQSGY |
| SEQ ID No: 2 | 0602 The amino acid sequence of HPV type 33 L1 protein aa 474-499 |
| | KAKPKLKRAAPTSTRTSSAKRKKVKK |
| SEQ ID No: 3 | 0603 Amino acid sequence of chimeric HPV type 6 L1 protein |
| | MWRPSDSTVYVPPPNPVSKVVATDAYVTRTNIFYHASSSRLLAVGHPYFSIKRA NKTVVPKVSGYQYRVFKVVLPDPNKFALPDSSLFDPTTQRLVWACTGLEVGRG QPLGVGVSGHPFLNKYDDVENSGSGGNPGQDNRVNVGMDYKQTQLCMVGCA PPLGEHWGKGKQCTNTPVQAGDCPPLELITSVIQDGDMVDTGFGAMNFADLQT NKSDVPIDICGTTCKYPDYLQMAADPYGDRLFFFLRKEQMFARHFFNRAGEVG EPVPDTLIIKGSGNRTSVGSSIYVNTPSGSLVSSEAQLFNKPYWLQKAQGHNNGI CWGNQLFVTVVDTTRSTNMTLCASVTTSSTYTNSDYKEYMRHVEEYDLQFIFQ LCSITLSAEVMAYIHTMNPSVLEDWNFGLSPPPNGTLEDTYRYVQSQAITCQKP TPEKEKPDPYKNLSFWEVNLKEKFSSELDQYPLGRKFLLQSGYKAKPKLKRAA |
| | PTSTRTSSAKRKKVKK |
| SEQ ID No: 4 | 0604 Nucleotide sequence of chimeric HPV type 6 L1 protein |
| | ATGTGGAGACCATCTGACAGCACAGTCTATGTGCCTCCTCCAAACCCTGTGAGCAAGGTGGTGGCTACAGA TGCCTATGTGACCAGGACCAACATCTTCTACCATGCCTCCTCCAGCAGACTGCTGGCTGTGGGACACCCATA CTTCAGCATCAAGAGGGCTAACAAGACAGTGGTGCCAAAGGTGTCTGGCTACCAATACAGGGTGTTCAAGG TGGTGCTGCCTGACCCAAACAAGTTTGCCCTGCCTGACTCCTCCCTGTTTGACCCAACCACCCAGAGACTGG TGTGGGCTTGTACTGGATTGGAGGTGGGCAGGGGACAACCACTGGGAGTGGGAGTGTCTGGACACCCATTC CTGAACAAATATGATGATGTGGAGAACTCTGGCTCTGGAGGCAACCCTGGACAAGACAACAGGGTGAATG TGGGGATGGACTACAAGCAGACCCAACTTTGTATGGTGGGCTGTGCCCCTCCACTGGGAGAACACTGGGGC AAGGGCAAGCAGTGTACCAACACACCTGTCCAGGCTGGAGACTGTCCTCCATTGGAACTGATTACCTCTGT GATTCAGGATGGAGATATGGTGGACACAGGCTTTGGAGCTATGAACTTTGCTGACCTCCAAACCAACAAGT CTGATGTGCCAATTGACATCTGTGGCACCACTTGTAAATACCCTGACTACCTCCAAATGGCTGCTGACCCAT ATGGAGACAGACTGTTCTTCTTCCTGAGGAAGGAACAGATGTTTGCCAGACACTTCTTCAACAGGGCTGGA GAGGTGGGAGAACCTGTGCCTGACACCCTGATTATCAAGGGCTCTGGCAACAGGACCTCTGTGGGCTCCAG CATCTATGTGAACACACCATCTGGCTCCCTGGTGTCCTCTGAGGCTCAACTTTTCAACAAGCCATACTGGCT CCAAAAGGCTCAAGGACACAACAATGGCATCTGTTGGGGCAACCAACTTTTTGTGACAGTGGTGGACACCA CCAGGAGCACCAATATGACCCTGTGTGCCTCTGTGACCACCTCCAGCACCTACACCAACTCTGACTACAAG GAATATATGAGGCATGTGGAGGAATATGACCTCCAATTCATCTTCCAACTTTGTAGCATCACCCTGTCTGCT GAGGTGATGGCTTACATCCACACAATGAACCCATCTGTGTTGGAGGACTGGAACTTTGGACTGAGCCCTCC TCCAAATGGCACCTTGGAGGACACCTACAGATATGTCCAGAGCCAGGCTATCACTTGTCAGAAGCCAACAC CTGAGAAGGAGAAGCCTGACCCATACAAGAACCTGTCCTTCTGGGAGGTGAACCTGAAAGAGAAGTTCTCC TCTGAACTGGACCAATACCCACTGGGCAGGAAGTTCCTGCTCCAATCTGGCTACAAAGCCAAGCCAAAACT GAAAAGGGCTGCCCCAACCAGCACCAGGACCTCCTCTGCCAAGAGGAAGAAGGTGAAGAAGTAA |
| SEQ ID No: 5 | 0605 Synthetic HPV6 L1 gene |
| | ctgggtaccATGTGGAGACCATCTGACAGCACAGTCTATGTGCCTCCTCCAAACCCTGTGAGCAAGGTGGTGGC TACAGATGCCTATGTGACCAGGACCAACATCTTCTACCATGCCTCCTCCAGCAGACTGCTGGCTGTGGGAC ACCCATACTTCAGCATCAAGAGGGCTAACAAGACAGTGGTGCCAAAGGTGTCTGGCTACCAATACAGGGTG TTCAAGGTGGTGCTGCCTGACCCAAACAAGTTTGCCCTGCCTGACTCCTCCCTGTTTGACCCAACCACCCAG AGACTGGTGTGGGCTTGTACTGGATTGGAGGTGGGCAGGGGACAACCACTGGGAGTGGGAGTGTCTGGAC ACCCATTCCTGAACAAATATGATGATGTGGAGAACTCTGGCTCTGGAGGCAACCCTGGACAAGACAACAGG GTGAATGTGGGGATGGACTACAAGCAGACCCAACTTTGTATGGTGGGCTGTGCCCCTCCACTGGGAGAACA CTGGGGCAAGGGCAAGCAGTGTACCAACACACCTGTCCAGGCTGGAGACTGTCCTCCATTGGAACTGATTA CCTCTGTGATTCAGGATGGAGATATGGTGGACACAGGCTTTGGAGCTATGAACTTTGCTGACCTCCAAACC AACAAGTCTGATGTGCCAATTGACATCTGTGGCACCACTTGTAAATACCCTGACTACCTCCAAATGGCTGCT GACCCATATGGAGACAGACTGTTCTTCTTCCTGAGGAAGGAACAGATGTTTGCCAGACACTTCTTCAACAG GGCTGGAGAGGTGGGAGAACCTGTGCCTGACACCCTGATTATCAAGGGCTCTGGCAACAGGACCTCTGTGG GCTCCAGCATCTATGTGAACACACCATCTGGCTCCCTGGTGTCCTCTGAGGCTCAACTTTTCAACAAGCCAT ACTGGCTCCAAAAGGCTCAAGGACACAACAATGGCATCTGTTGGGGCAACCAACTTTTTGTGACAGTGGTG GACACCACCAGGAGCACCAATATGACCCTGTGTGCCTCTGTGACCACCTCCAGCACCTACACCAACTCTGA CTACAAGGAATATATGAGGCATGTGGAGGAATATGACCTCCAATTCATCTTCCAACTTTGTAGCATCACCCT GTCTGCTGAGGTGATGGCTTACATCCACACAATGAACCCATCTGTGTTGGAGGACTGGAACTTTGGACTGA GCCCTCCTCCAAATGGCACCTTGGAGGACACCTACAGATATGTCCAGAGCCAGGCTATCACTTGTCAGAAG CCAACACCTGAGAAGGAGAAGCCTGACCCATACAAGAACCTGTCCTTCTGGGAGGTGAACCTGAAAGAGA AGTTCTCCTCTGAACTGGACCAATACCCACTGGGCAGGAAGTTCCTGCTCCAATCTGGCTACAGGGGCAGG TCCAGCATCAGGACAGGAGTGAAGAGACCTGCTGTGAGCAAGGCATCTGCTGCCCCAAAGAGGAAGAGGG CTAAGACCAAGAGGTAAActcgagctc |
| SEQ ID No: 6 | 0606 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG |
| | ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 7 | 0607 HPV6 L1 F1 |
| | CTTGGTACCATGTGGAGACCATCTGACAGCACAGT |
| SEQ ID No: 8 | 0608 HPV6 L1 R1 |
| | GCTTGGCTTTGTAGCCAGATTGGAGCAGGAACTTCC |
| SEQ ID No: 9 | 0609 HPV6 L1 amplified sequence 1 |
| | CTTGGTACC[1] ATGTGGAGACCATCTGACAGCACAGTCTATGTGCCTCCTCCAAACCCTGTGAGCAAGGTG GTGGCTACAGATGCCTATGTGACCAGGACCAACATCTTCTACCATGCCTCCTCCAGCAGA CTGCTGGCTGTGGGACACCCATACTTCAGCATCAAGAGGGCTAACAAGACAGTGGTGCCA AAGGTGTCTGGCTACCAATACAGGGTGTTCAAGGTGGTGCTGCCTGACCCAAACAAGTTT GCCCTGCCTGACTCCTCCCTGTTTGACCCAACCACCCAGAGACTGGTGTGGGCTTGTACT GGATTGGAGGTGGGCAGGGGACAACCACTGGGAGTGGGAGTGTCTGGACACCCATTCCTG AACAAATATGATGATGTGGAGAACTCTGGCTCTGGAGGCAACCCTGGACAAGACAACAGG GTGAATGTGGGGATGGACTACAAGCAGACCCAACTTTGTATGGTGGGCTGTGCCCCTCCA CTGGGAGAACACTGGGGCAAGGGCAAGCAGTGTACCAACACACCTGTCCAGGCTGGAGAC TGTCCTCCATTGGAACTGATTACCTCTGTGATTCAGGATGGAGATATGGTGGACACAGGC TTTGGAGCTATGAACTTTGCTGACCTCCAAACCAACAAGTCTGATGTGCCAATTGACATC TGTGGCACCACTTGTAAATACCCTGACTACCTCCAAATGGCTGCTGACCCATATGGAGAC AGACTGTTCTTCTTCCTGAGGAAGGAACAGATGTTTGCCAGACACTTCTTCAACAGGGCT GGAGAGGTGGGAGAACCTGTGCCTGACACCCTGATTATCAAGGGCTCTGGCAACAGGACC TCTGTGGGCTCCAGCATCTATGTGAACACACCATCTGGCTCCCTGGTGTCCTCTGAGGCT CAACTTTTCAACAAGCCATACTGGCTCCAAAAGGCTCAAGGACACAACAATGGCATCTGT TGGGGCAACCAACTTTTTGTGACAGTGGTGGACACCACCAGGAGCACCAATATGACCCTG TGTGCCTCTGTGACCACCTCCAGCACCTACACCAACTCTGACTACAAGGAATATATGAGG CATGTGGAGGAATATGACCTCCAATTCATCTTCCAACTTTGTAGCATCACCCTGTCTGCT GAGGTGATGGCTTACATCCACACAATGAACCCATCTGTGTTGGAGGACTGGAACTTTGGA CTGAGCCCTCCTCCAAATGGCACCTTGGAGGACACCTACAGATATGTCCAGAGCCAGGCT ATCACTTGTCAGAAGCCAACACCTGAGAAGGAGAAGCCTGACCCATACAAGAACCTGTCC TTCTGGGAGGTGAACCTGAAAGAGAAGTTCTCCTCTGAACTGGACCAATACCCACTGGGC AGGAAGTTCCTGCTCCAATCTGGCTAC[2]AAAGCCAAGC[3] |
| | [1]: Kpnlenzymatic cleavage site; |
| | [2]: the gene fragment encoding HPV6 L1 aa 1-469; |
| | [3]: 10 bases overlapping with the gene fragment encoding HPV33L1 aa 474-499. |
| SEQ ID No: 10 | 0610 HPV6 L1 F2 |
| | ATCTGGCTACAAAGCCAAGCCAAAACTGAAAAGGG |
| SEQ ID No: 11 | 0611 HPV6 L1 R2 |
| | CTGTCTAGATTTACTTCTTCACCTTCTTCCTCTTGGC |
| SEQ ID No: 12 | 0612 HPV6 L1 amplified sequence 2 |
| | ATCTGGCTAC[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV6 L1 aa 1-469; |
| | [2]: the gene fragment encoding the C-terminal 26 amino acids of HPV33L1 aa 474-499; |
| | [3]: Xbal enzymatic cleavage site. |
| SEQ ID No: 13 | 0613The amino acid sequence of HPV type 59 L1 protein aa 471-508 |
| | LQLGARPKPTIGPRKRAAPAPTSTPSPKRVKRRKSSRK |

### Appendix 2 : SEQUENCE LISTING -CHIMERIC HUMAN PAPILLOMAVIRUS TYPE 11 L1 PROTEIN

| | |
|---|---|
| SEQ ID No: 14 | 1101 The amino acid sequence of HPV type 11 L1 protein aa 1-470 |
| | MWRPSDSTVYVPPPNPVSKVVATDAYVKRTNIFYHASSSRLLAVGHPYYSIKK VNKTVVPKVSGYQYRVFKVVLPDPNKFALPDSSLFDPTTQRLVWACTGLEVGR GQPLGVGVSGHPLLNKYDDVENSGGYGGNPGQDNRVNVGMDYKQTQLCMV GCAPPLGEHWGKGTQCSNTSVQNGDCPPLELITSVIQDGDMVDTGFGAMNFAD LQTNKSDVPLDICGTVCKYPDYLQMAADPYGDRLFFYLRKEQMFARHFFNRA GTVGEPVPDDLLVKGGNNRSSVASSIYVHTPSGSLVSSEAQLFNKPYWLQKAQ GHNNGICWGNHLFVTVVDTTRSTNMTLCASVSKSATYTNSDYKEYMRHVEEF DLQFIFQLCSITLSAEVMAYIHTMNPSVLEDWNFGLSPPPNGTLEDTYRYVQSQ AITCQKPTPEKEKQDPYKDMSFWEVNLKEKFSSELDQFPLGRKFLLQSGY |
| SEQ ID No: 15 | 1102 The amino acid sequence of HPV type 33 L1 protein aa 474-499 |
| | KAKPKLKRAAPTSTRTSSAKRKKVKK |
| SEQ ID No: 16 | 1103 Amino acid sequence of chimeric HPV type 11 L1 protein |
| | MWRPSDSTVYVPPPNPVSKVVATDAYVKRTNIFYHASSSRLLAVGHPYYSIKK VNKTVVPKVSGYQYRVFKVVLPDPNKFALPDSSLFDPTTQRLVWACTGLEVGR GQPLGVGVSGHPLLNKYDDVENSGGYGGNPGQDNRVNVGMDYKQTQLCMV GCAPPLGEHWGKGTQCSNTSVQNGDCPPLELITSVIQDGDMVDTGFGAMNFAD LQTNKSDVPLDICGTVCKYPDYLQMAADPYGDRLFFYLRKEQMFARHFFNRA GTVGEPVPDDLLVKGGNNRSSVASSIYVHTPSGSLVSSEAQLFNKPYWLQKAQ GHNNGICWGNHLFVTVVDTTRSTNMTLCASVSKSATYTNSDYKEYMRHVEEF DLQFIFQLCSITLSAEVMAYIHTMNPSVLEDWNFGLSPPPNGTLEDTYRYVQSQ AITCQKPTPEKEKQDPYKDMSFWEVNLKEKFSSELDQFPLGRKFLLQSGYKAKP KLKRAAPTSTRTSSAKRKKVKK |
| SEQ ID No: 17 | 1104 Nucleotide sequence of chimeric HPV type 11 L1 protein |
| | ATGTGGAGACCATCTGACAGCACAGTCTATGTGCCTCCTCCAAACCCTGTGAGCAAGGTGGTGGCTACAGA TGCCTATGTGAAGAGGACCAACATCTTCTACCATGCCTCCTCCAGCAGACTGCTGGCTGTGGGACACCCAT ACTACAGCATCAAGAAGGTGAACAAGACAGTGGTGCCAAAGGTGTCTGGCTACCAATACAGGGTGTTCAA GGTGGTGCTGCCTGACCCAAACAAGTTTGCCCTGCCTGACTCCTCCCTGTTTGACCCAACCACCCAGAGACT GGTGTGGGCTTGTACTGGATTGGAGGTGGGCAGGGGACAACCACTGGGAGTGGGAGTGTCTGGACACCCA CTGCTGAACAAATATGATGATGTGGAGAACTCTGGAGGCTATGGAGGCAACCCTGGACAAGACAACAGGG TGAATGTGGGGATGGACTACAAGCAGACCCAACTTTGTATGGTGGGCTGTGCCCCTCCACTGGGAGAACAC TGGGGCAAGGGCACCCAGTGTAGCAACACCTCTGTCCAGAATGGAGACTGTCCTCCATTGGAACTGATTAC CTCTGTGATTCAGGATGGAGATATGGTGGACACAGGCTTTGGAGCTATGAACTTTGCTGACCTCCAAACCA ACAAGTCTGATGTGCCACTGGACATCTGTGGCACAGTGTGTAAATACCCTGACTACCTCCAAATGGCTGCT GACCCATATGGAGACAGACTGTTCTTCTACCTGAGGAAGGAACAGATGTTTGCCAGACACTTCTTCAACAG GGCTGGCACAGTGGGAGAACCTGTGCCTGATGACCTGCTGGTGAAGGGAGGCAACAACAGGTCCTCTGTGG CATCCAGCATCTATGTGCATACACCATCTGGCTCCCTGGTGTCCTCTGAGGCTCAACTTTTCAACAAGCCAT ACTGGCTCCAAAAGGCTCAAGGACACAACAATGGCATCTGTTGGGGCAACCACCTGTTTGTGACAGTGGTG GACACCACCAGGAGCACCAATATGACCCTGTGTGCCTCTGTGAGCAAGTCTGCCACCTACACCAACTCTGA CTACAAGGAATATATGAGGCATGTGGAGGAGTTTGACCTCCAATTCATCTTCCAACTTTGTAGCATCACCCT GTCTGCTGAGGTGATGGCTTACATCCACACAATGAACCCATCTGTGTTGGAGGACTGGAACTTTGGACTGA GCCCTCCTCCAAATGGCACCTTGGAGGACACCTACAGATATGTCCAGAGCCAGGCTATCACTTGTCAGAAG CCAACACCTGAGAAGGAGAAGCAGGACCCATACAAGGATATGAGTTTCTGGGAGGTGAACCTGAAAGAGA AGTTCTCCTCTGAACTGGACCAGTTTCCACTGGGCAGGAAGTTCCTGCTCCAATCTGGCTACAAAGCCAAGC CAAAACTGAAAAGGGCTGCCCCAACCAGCACCAGGACCTCCTCTGCCAAGAGGAAGAAGGTGAAGAAGTA AA |
| SEQ ID No: 18 | 1105 Synthetic HPV11 L1 gene |
| | ctgggtaccATGTGGAGACCATCTGACAGCACAGTCTATGTGCCTCCTCCAAACCCTGTGAGCAAGGTGGTGGC TACAGATGCCTATGTGAAGAGGACCAACATCTTCTACCATGCCTCCTCCAGCAGACTGCTGGCTGTGGGAC ACCCATACTACAGCATCAAGAAGGTGAACAAGACAGTGGTGCCAAAGGTGTCTGGCTACCAATACAGGGT GTTCAAGGTGGTGCTGCCTGACCCAAACAAGTTTGCCCTGCCTGACTCCTCCCTGTTTGACCCAACCACCCA GAGACTGGTGTGGGCTTGTACTGGATTGGAGGTGGGCAGGGGACAACCACTGGGAGTGGGAGTGTCTGGA CACCCACTGCTGAACAAATATGATGATGTGGAGAACTCTGGAGGCTATGGAGGCAACCCTGGACAAGACA ACAGGGTGAATGTGGGGATGGACTACAAGCAGACCCAACTTTGTATGGTGGGCTGTGCCCCTCCACTGGGA GAACACTGGGGCAAGGGCACCCAGTGTAGCAACACCTCTGTCCAGAATGGAGACTGTCCTCCATTGGAACT GATTACCTCTGTGATTCAGGATGGAGATATGGTGGACACAGGCTTTGGAGCTATGAACTTTGCTGACCTCCA AACCAACAAGTCTGATGTGCCACTGGACATCTGTGGCACAGTGTGTAAATACCCTGACTACCTCCAAATGG CTGCTGACCCATATGGAGACAGACTGTTCTTCTACCTGAGGAAGGAACAGATGTTTGCCAGACACTTCTTCA ACAGGGCTGGCACAGTGGGAGAACCTGTGCCTGATGACCTGCTGGTGAAGGGAGGCAACAACAGGTCCTC TGTGGCATCCAGCATCTATGTGCATACACCATCTGGCTCCCTGGTGTCCTCTGAGGCTCAACTTTTCAACAA GCCATACTGGCTCCAAAAGGCTCAAGGACACAACAATGGCATCTGTTGGGGCAACCACCTGTTTGTGACAG TGGTGGACACCACCAGGAGCACCAATATGACCCTGTGTGCCTCTGTGAGCAAGTCTGCCACCTACACCAAC TCTGACTACAAGGAATATATGAGGCATGTGGAGGAGTTTGACCTCCAATTCATCTTCCAACTTTGTAGCATC ACCCTGTCTGCTGAGGTGATGGCTTACATCCACACAATGAACCCATCTGTGTTGGAGGACTGGAACTTTGG ACTGAGCCCTCCTCCAAATGGCACCTTGGAGGACACCTACAGATATGTCCAGAGCCAGGCTATCACTTGTC AGAAGCCAACACCTGAGAAGGAGAAGCAGGACCCATACAAGGATATGAGTTTCTGGGAGGTGAACCTGAA AGAGAAGTTCTCCTCTGAACTGGACCAGTTTCCACTGGGCAGGAAGTTCCTGCTCCAATCTGGCTACAGGG GCAGGACCTCTGCCAGGACAGGCATCAAGAGACCTGCTGTGAGCAAGCCAAGCACAGCCCCAAAGAGGAA GAGGACCAAGACCAAGAAGTAAActcgagctc |
| SEQ ID No: 19 | 1106 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG |
| | ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 20 | 1107HPV11 L1 F1 |
| | CTTGGTACCATGTGGAGACCATCTGACAGCACAGT |
| SEQ ID No: 21 | 1108HPV11 L1 R1 |
| | GCTTGGCTTTGTAGCCAGATTGGAGCAGGAACTTCC |
| SEQ ID No: 22 | 1109 HPV11 L1 amplified sequence 1 |
| | CTTGGTACC[1] ATGTGGAGACCATCTGACAGCACAGTCTATGTGCCTCCTCCAAACCCTGTGAGCAAGGTGGTGGCTACAGA TGCCTATGTGAAGAGGACCAACATCTTCTACCATGCCTCCTCCAGCAGACTGCTGGCTGTGGGACACCCAT ACTACAGCATCAAGAAGGTGAACAAGACAGTGGTGCCAAAGGTGTCTGGCTACCAATACAGGGTGTTCAA GGTGGTGCTGCCTGACCCAAACAAGTTTGCCCTGCCTGACTCCTCCCTGTTTGACCCAACCACCCAGAGACT GGTGTGGGCTTGTACTGGATTGGAGGTGGGCAGGGGACAACCACTGGGAGTGGGAGTGTCTGGACACCCA CTGCTGAACAAATATGATGATGTGGAGAACTCTGGAGGCTATGGAGGCAACCCTGGACAAGACAACAGGG TGAATGTGGGGATGGACTACAAGCAGACCCAACTTTGTATGGTGGGCTGTGCCCCTCCACTGGGAGAACAC TGGGGCAAGGGCACCCAGTGTAGCAACACCTCTGTCCAGAATGGAGACTGTCCTCCATTGGAACTGATTAC CTCTGTGATTCAGGATGGAGATATGGTGGACACAGGCTTTGGAGCTATGAACTTTGCTGACCTCCAAACCA ACAAGTCTGATGTGCCACTGGACATCTGTGGCACAGTGTGTAAATACCCTGACTACCTCCAAATGGCTGCT GACCCATATGGAGACAGACTGTTCTTCTACCTGAGGAAGGAACAGATGTTTGCCAGACACTTCTTCAACAG GGCTGGCACAGTGGGAGAACCTGTGCCTGATGACCTGCTGGTGAAGGGAGGCAACAACAGGTCCTCTGTGG CATCCAGCATCTATGTGCATACACCATCTGGCTCCCTGGTGTCCTCTGAGGCTCAACTTTTCAACAAGCCAT ACTGGCTCCAAAAGGCTCAAGGACACAACAATGGCATCTGTTGGGGCAACCACCTGTTTGTGACAGTGGTG GACACCACCAGGAGCACCAATATGACCCTGTGTGCCTCTGTGAGCAAGTCTGCCACCTACACCAACTCTGA CTACAAGGAATATATGAGGCATGTGGAGGAGTTTGACCTCCAATTCATCTTCCAACTTTGTAGCATCACCCT GTCTGCTGAGGTGATGGCTTACATCCACACAATGAACCCATCTGTGTTGGAGGACTGGAACTTTGGACTGA GCCCTCCTCCAAATGGCACCTTGGAGGACACCTACAGATATGTCCAGAGCCAGGCTATCACTTGTCAGAAG CCAACACCTGAGAAGGAGAAGCAGGACCCATACAAGGATATGAGTTTCTGGGAGGTGAACCTGAAAGAGA AGTTCTCCTCTGAACTGGACCAGTTTCCACTGGGCAGGAAGTTCCTGCTCCAATCTGGCTAC[2]AAAGCCAA GC[3] |
| | [1]: Kpn 1 enzymatic cleavage site; |
| | [2]: the gene fragment encoding of HPV11 L1 aa 1-470; |
| | [3]: 10 bases overlapping with the gene fragment encoding HPV33L1 aa 474-499. |
| SEQ ID No: 23 | 1110HPV11 L1 F2 |
| | ATCTGGCTACAAAGCCAAGCCAAAACTGAAAAGGG |
| SEQ ID No: 24 | 1111HPV11 L1 R2 |
| | CTGTCTAGATTTACTTCTTCACCTTCTTCCTCTTGGC |
| SEQ ID No: 25 | 1112 HPV11 L1 amplified sequence 2 |
| | ATCTGGCTAC[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV11 L1 aa 1-470; |
| | [2]: the gene fragment encoding the C-terminal 26 amino acids of HPV33L1 aa 474-499; |
| | [3]: Xbal enzymatic cleavage site. |
| SEQ ID No: 26 | 1113 The amino acid sequence of HPV type 59 L1 protein aa 471-508 |
| | LQLGARPKPTIGPRKRAAPAPTSTPSPKRVKRRKSSRK |

### Appendix 3 : SEQUENCE LISTING -CHIMERIC HUMAN PAPILLOMAVIRUS TYPE 16 L1 PROTEIN

| | |
|---|---|
| SEQ ID No: 27 | 1601 The amino acid sequence of HPV type 16 L1 protein aa 1-474 |
| | MSLWLPSEATVYLPPVPVSKVVSTDEYVARTNIYYHAGTSRLLAVGHPYFPIKK PNNNKILVPKVSGLQYRVFRIHLPDPNKFGFPDTSFYNPDTQRLVWACVGVEVG RGQPLGVGISGHPLLNKLDDTENASAYAANAGVDNRECISMDYKQTQLCLIGC KPPIGEHWGKGSPCTNVAVNPGDCPPLELINTVIQDGDMVDTGFGAMDFTTLQ ANKSEVPLDICTSICKYPDYIKMVSEPYGDSLFFYLRREQMFVRHLFNRAGAVG ENVPDDLYIKGSGSTANLASSNYFPTPSGSMVTSDAQIFNKPYWLQRAQGHNN GICWGNQLFVTVVDTTRSTNMSLCAAISTSETTYKNTNFKEYLRHGEEYDLQFI FQLCKITLTADVMTYIHSMNSTILEDWNFGLQPPPGGTLEDTYRFVTSQAIACQ KHTPPAPKEDPLKKYTFWEVNLKEKFSADLDQFPLGRKFLLQAGL |
| SEQ ID No: 28 | 1602 The amino acid sequence of HPV type 33 L1 protein aa 474-499 |
| | KAKPKLKRAAPTSTRTSSAKRKKVKK |
| SEQ ID No: 29 | 1603 Amino acid sequence of chimeric HPV type 16 L1 protein |
| | MSLWLPSEATVYLPPVPVSKVVSTDEYVARTNIYYHAGTSRLLAVGHPYFPIKK PNNNKILVPKVSGLQYRVFRIHLPDPNKFGFPDTSFYNPDTQRLVWACVGVEVG RGQPLGVGISGHPLLNKLDDTENASAYAANAGVDNRECISMDYKQTQLCLIGC KPPIGEHWGKGSPCTNVAVNPGDCPPLELINTVIQDGDMVDTGFGAMDFTTLQ ANKSEVPLDICTSICKYPDYIKMVSEPYGDSLFFYLRREQMFVRHLFNRAGAVG ENVPDDLYIKGSGSTANLASSNYFPTPSGSMVTSDAQIFNKPYWLQRAQGHNN GICWGNQLFVTVVDTTRSTNMSLCAAISTSETTYKNTNFKEYLRHGEEYDLQFI FQLCKITLTADVMTYIHSMNSTILEDWNFGLQPPPGGTLEDTYRFVTSQAIACQ KHTPPAPKEDPLKKYTFWEVNLKEKFSADLDQFPLGRKFLLQAGLKAKPKLKR AAPTSTRTSSAKRKKVKK |
| SEQ ID No: 30 | 1604 Nucleotide sequence of chimeric HPV type 16 L1 protein |
| | ATGAGTCTGTGGCTGCCATCTGAGGCTACAGTCTACCTGCCTCCTGTGCCTGTGAGCAAGGTGGTGAGCAC AGATGAATATGTGGCAAGGACCAACATCTACTACCATGCTGGCACCAGCAGACTGCTGGCTGTGGGACACC CATACTTTCCAATCAAGAAGCCAAACAACAACAAGATTCTGGTGCCAAAGGTGTCTGGACTCCAATACAGG GTGTTCAGGATTCACCTGCCTGACCCAAACAAGTTTGGCTTTCCTGACACCTCCTTCTACAACCCTGACACC CAGAGACTGGTGTGGGCTTGTGTGGGAGTGGAGGTGGGCAGGGGACAACCACTGGGAGTGGGCATCTCTG GACACCCACTGCTGAACAAACTGGATGACACAGAGAATGCCTCTGCCTATGCTGCCAATGCTGGAGTGGAC AACAGGGAGTGTATCAGTATGGACTACAAGCAGACCCAACTTTGTCTGATTGGCTGTAAGCCTCCAATTGG AGAACACTGGGGCAAGGGCAGCCCATGTACCAATGTGGCTGTGAACCCTGGAGACTGTCCTCCATTGGAAC TGATAAACACAGTGATTCAGGATGGAGATATGGTGGACACAGGCTTTGGAGCTATGGACTTCACCACCCTC CAAGCCAACAAGTCTGAGGTGCCACTGGACATCTGTACCAGCATCTGTAAATACCCTGACTACATCAAGAT GGTGTCTGAACCATATGGAGACTCCCTGTTCTTCTACCTGAGGAGGGAACAGATGTTTGTGAGACACCTGTT CAACAGGGCTGGAGCAGTGGGAGAGAATGTGCCTGATGACCTCTACATCAAGGGCTCTGGCAGCACAGCC AACCTGGCATCCAGCAACTACTTTCCAACACCATCTGGCAGTATGGTGACCTCTGATGCCCAGATTTTCAAC AAGCCATACTGGCTCCAAAGGGCTCAAGGACACAACAATGGCATCTGTTGGGGCAACCAACTTTTTGTGAC AGTGGTGGACACCACCAGGAGCACCAATATGAGTCTGTGTGCTGCCATCAGCACCTCTGAGACCACCTACA AGAACACCAACTTCAAGGAATACCTGAGACATGGAGAGGAATATGACCTCCAATTCATCTTCCAACTTTGT AAGATTACCCTGACAGCAGATGTGATGACCTACATCCACAGTATGAACAGCACCATCTTGGAGGACTGGAA CTTTGGACTCCAACCTCCTCCTGGAGGCACCTTGGAGGACACCTACAGGTTTGTGACCAGCCAGGCTATTGC CTGTCAGAAACACACACCTCCTGCCCCAAAGGAGGACCCACTGAAAAAATACACCTTCTGGGAGGTGAACC TGAAAGAGAAGTTCTCTGCTGACCTGGACCAGTTTCCACTGGGCAGGAAGTTCCTGCTCCAAGCAGGACTG AAAGCCAAGCCAAAACTGAAAAGGGCTGCCCCAACCAGCACCAGGACCTCCTCTGCCAAGAGGAAGAAGG TGAAGAAGTAAA |
| SEQ ID No: 31 | 1605 Synthetic HPV16 L1 gene |
| | ctgggtaccATGAGTCTGTGGCTGCCATCTGAGGCTACAGTCTACCTGCCTCCTGTGCCTGTGAGCAAGGTGGTG AGCACAGATGAATATGTGGCAAGGACCAACATCTACTACCATGCTGGCACCAGCAGACTGCTGGCTGTGGG ACACCCATACTTTCCAATCAAGAAGCCAAACAACAACAAGATTCTGGTGCCAAAGGTGTCTGGACTCCAAT ACAGGGTGTTCAGGATTCACCTGCCTGACCCAAACAAGTTTGGCTTTCCTGACACCTCCTTCTACAACCCTG ACACCCAGAGACTGGTGTGGGCTTGTGTGGGAGTGGAGGTGGGCAGGGGACAACCACTGGGAGTGGGCAT CTCTGGACACCCACTGCTGAACAAACTGGATGACACAGAGAATGCCTCTGCCTATGCTGCCAATGCTGGAG TGGACAACAGGGAGTGTATCAGTATGGACTACAAGCAGACCCAACTTTGTCTGATTGGCTGTAAGCCTCCA ATTGGAGAACACTGGGGCAAGGGCAGCCCATGTACCAATGTGGCTGTGAACCCTGGAGACTGTCCTCCATT GGAACTGATAAACACAGTGATTCAGGATGGAGATATGGTGGACACAGGCTTTGGAGCTATGGACTTCACCA CCCTCCAAGCCAACAAGTCTGAGGTGCCACTGGACATCTGTACCAGCATCTGTAAATACCCTGACTACATC AAGATGGTGTCTGAACCATATGGAGACTCCCTGTTCTTCTACCTGAGGAGGGAACAGATGTTTGTGAGACA CCTGTTCAACAGGGCTGGAGCAGTGGGAGAGAATGTGCCTGATGACCTCTACATCAAGGGCTCTGGCAGCA CAGCCAACCTGGCATCCAGCAACTACTTTCCAACACCATCTGGCAGTATGGTGACCTCTGATGCCCAGATTT TCAACAAGCCATACTGGCTCCAAAGGGCTCAAGGACACAACAATGGCATCTGTTGGGGCAACCAACTTTTT GTGACAGTGGTGGACACCACCAGGAGCACCAATATGAGTCTGTGTGCTGCCATCAGCACCTCTGAGACCAC CTACAAGAACACCAACTTCAAGGAATACCTGAGACATGGAGAGGAATATGACCTCCAATTCATCTTCCAAC TTTGTAAGATTACCCTGACAGCAGATGTGATGACCTACATCCACAGTATGAACAGCACCATCTTGGAGGAC TGGAACTTTGGACTCCAACCTCCTCCTGGAGGCACCTTGGAGGACACCTACAGGTTTGTGACCAGCCAGGC TATTGCCTGTCAGAAACACACACCTCCTGCCCCAAAGGAGGACCCACTGAAAAAATACACCTTCTGGGAGG TGAACCTGAAAGAGAAGTTCTCTGCTGACCTGGACCAGTTTCCACTGGGCAGGAAGTTCCTGCTCCAAGCA GGACTGAAAGCCAAGCCAAAGTTCACCCTGGGCAAGAGGAAGGCTACACCAACCACCTCCAGCACCAGCA CCACAGCCAAGAGGAAGAAGAGGAAACTGTAAActcgagctc |
| SEQ ID No: 32 | 1606 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG |
| | ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 33 | 1607HPV16 L1 F1 |
| | CTTGGTACCATGAGTCTGTGGCTGCCATCTGAGG |
| SEQ ID No: 34 | 1608HPV16 L1 R1 |
| | GCTTGGCTTTCAGTCCTGCTTGGAGCAGGAACTTCC |
| SEQ ID No: 35 | 1609 HPV16 L1 amplified sequence 1 |
| | CTTGGTACC[1] ATGAGTCTGTGGCTGCCATCTGAGGCTACAGTCTACCTGCCTCCTGTGCCTGTGAGCAAGGTGGTGAGCAC AGATGAATATGTGGCAAGGACCAACATCTACTACCATGCTGGCACCAGCAGACTGCTGGCTGTGGGACACC CATACTTTCCAATCAAGAAGCCAAACAACAACAAGATTCTGGTGCCAAAGGTGTCTGGACTCCAATACAGG GTGTTCAGGATTCACCTGCCTGACCCAAACAAGTTTGGCTTTCCTGACACCTCCTTCTACAACCCTGACACC CAGAGACTGGTGTGGGCTTGTGTGGGAGTGGAGGTGGGCAGGGGACAACCACTGGGAGTGGGCATCTCTG GACACCCACTGCTGAACAAACTGGATGACACAGAGAATGCCTCTGCCTATGCTGCCAATGCTGGAGTGGAC AACAGGGAGTGTATCAGTATGGACTACAAGCAGACCCAACTTTGTCTGATTGGCTGTAAGCCTCCAATTGG AGAACACTGGGGCAAGGGCAGCCCATGTACCAATGTGGCTGTGAACCCTGGAGACTGTCCTCCATTGGAAC TGATAAACACAGTGATTCAGGATGGAGATATGGTGGACACAGGCTTTGGAGCTATGGACTTCACCACCCTC CAAGCCAACAAGTCTGAGGTGCCACTGGACATCTGTACCAGCATCTGTAAATACCCTGACTACATCAAGAT GGTGTCTGAACCATATGGAGACTCCCTGTTCTTCTACCTGAGGAGGGAACAGATGTTTGTGAGACACCTGTT CAACAGGGCTGGAGCAGTGGGAGAGAATGTGCCTGATGACCTCTACATCAAGGGCTCTGGCAGCACAGCC AACCTGGCATCCAGCAACTACTTTCCAACACCATCTGGCAGTATGGTGACCTCTGATGCCCAGATTTTCAAC AAGCCATACTGGCTCCAAAGGGCTCAAGGACACAACAATGGCATCTGTTGGGGCAACCAACTTTTTGTGAC AGTGGTGGACACCACCAGGAGCACCAATATGAGTCTGTGTGCTGCCATCAGCACCTCTGAGACCACCTACA AGAACACCAACTTCAAGGAATACCTGAGACATGGAGAGGAATATGACCTCCAATTCATCTTCCAACTTTGT AAGATTACCCTGACAGCAGATGTGATGACCTACATCCACAGTATGAACAGCACCATCTTGGAGGACTGGAA CTTTGGACTCCAACCTCCTCCTGGAGGCACCTTGGAGGACACCTACAGGTTTGTGACCAGCCAGGCTATTGC CTGTCAGAAACACACACCTCCTGCCCCAAAGGAGGACCCACTGAAAAAATACACCTTCTGGGAGGTGAACC TGAAAGAGAAGTTCTCTGCTGACCTGGACCAGTTTCCACTGGGCAGGAAGTTCCTGCTCCAAGCAGGACTG[ 2]AAAGCCAAGC[3] |
| | [1]: Kpn \| enzymatic cleavage site; |
| | [2]: the gene fragment encoding of HPV16 L1 aa 1-474; |
| | [3]: 10 bases overlapping with the gene fragment encoding HPV33L1 aa 474-499. |
| SEQ ID No: 36 | 1610HPV16 L1 F2 |
| | AGCAGGACTGAAAGCCAAGCCAAAACTGAAAAGGG |
| SEQ ID No: 37 | 1611HPV16 L1 R2 |
| | CTGTCTAGATTTACTTCTTCACCTTCTTCCTCTTGG |
| SEQ ID No: 38 | 1612 HPV16 L1 amplified sequence 2 |
| | AGCAGGACTG [1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV16 L1 aa 1-474; |
| | [2]: the gene fragment encoding the C-terminal 26 amino acids of HPV33L1 aa 474-499; |
| | [3]: Xbal enzymatic cleavage site. |
| SEQ ID No: 39 | 1613 The amino acid sequence of HPV type 59 L1 protein aa 471-508 |
| | LQLGARPKPTIGPRKRAAPAPTSTPSPKRVKRRKSSRK |

### Appendix 4 : SEQUENCE LISTING -CHIMERIC HUMAN PAPILLOMAVIRUS TYPE 18 L1 PROTEIN

| | |
|---|---|
| SEQ ID No: 40 | 1801 The amino acid sequence of HPV type 18 L1 protein aa 1-470 |
| | MALWRPSDNTVYLPPPSVARVVNTDDYVTRTSIFYHAGSSRLLTVGNPYFRVP AGGGNKQDIPKVSAYQYRVFRVQLPDPNKFGLPDTSIYNPETQRLVWACAGVE IGRGQPLGVGLSGHPFYNKLDDTESSHAATSNVSEDVRDNVSVDYKQTQLCILG CAPAIGEHWAKGTACKSRPLSQGDCPPLELKNTVLEDGDMVDTGYGAMDFST LQDTKCEVPLDICQSICKYPDYLQMSADPYGDSMFFCLRREQLFARHFWNRAG TMGDTVPQSLYIKGTGMRASPGSCVYSPSPSGSIVTSDSQLFNKPYWLHKAQGH NNGVCWHNQLFVTVVDTTRSTNLTICASTQSPVPGQYDATKFKQYSRHVEEYD LQFIFQLCTITLTADVMSYIHSMNSSILEDWNFGVPPPPTTSLVDTYRFVQSVAIT CQKDAAPAENKDPYDKLKFWNVDLKEKFSLDLDQYPLGRKFL |
| SEQ ID No: 41 | 1802 The amino acid sequence of HPV type 33 L1 protein aa 474-499 |
| | KAKPKLKRAAPTSTRTSSAKRKKVKK |
| SEQ ID No: 42 | 1803 Amino acid sequence of chimeric HPV type 18 L1 protein |
| | MALWRPSDNTVYLPPPSVARVVNTDDYVTRTSIFYHAGSSRLLTVGNPYFRVP AGGGNKQDIPKVSAYQYRVFRVQLPDPNKFGLPDTSIYNPETQRLVWACAGVE IGRGQPLGVGLSGHPFYNKLDDTESSHAATSNVSEDVRDNVSVDYKQTQLCILG CAPAIGEHWAKGTACKSRPLSQGDCPPLELKNTVLEDGDMVDTGYGAMDFST LQDTKCEVPLDICQSICKYPDYLQMSADPYGDSMFFCLRREQLFARHFWNRAG TMGDTVPQSLYIKGTGMRASPGSCVYSPSPSGSIVTSDSQLFNKPYWLHKAQGH NNGVCWHNQLFVTVVDTTRSTNLTICASTQSPVPGQYDATKFKQYSRHVEEYD LQFIFQLCTITLTADVMSYIHSMNSSILEDWNFGVPPPPTTSLVDTYRFVQSVAIT CQKDAAPAENKDPYDKLKFWNVDLKEKFSLDLDQYPLGRKFLKAKPKLKRAA PTSTRTSSAKRKKVKK |
| SEQ ID No: 43 | 1804 Nucleotide sequence of chimeric HPV type 18 L1 protein |
| | ATGGCCCTCTGGAGACCATCCGATAACACAGTGTACTTGCCCCCACCCAGCGTCGCCCGGGTGGTGAACAC AGACGACTACGTCACCAGAACCTCAATCTTCTACCACGCCGGGTCCAGCCGGCTGCTGACCGTGGGCAACC CCTACTTCCGCGTGCCCGCCGGCGGCGGAAACAAACAAGACATCCCCAAAGTCAGCGCCTATCAGTACCGG GTGTTCCGCGTCCAACTGCCCGATCCCAACAAGTTCGGCCTGCCCGACACCTCCATCTACAACCCCGAGACC CAGAGGCTGGTCTGGGCTTGCGCCGGCGTCGAGATCGGGAGGGGCCAACCCCTGGGCGTGGGGTTGTCCGG CCACCCCTTCTACAACAAGCTGGACGATACCGAGTCCAGCCACGCAGCAACCAGCAACGTCTCCGAAGATG TGCGCGATAACGTCAGCGTGGACTACAAACAAACCCAACTGTGCATCCTGGGATGCGCACCCGCCATCGGC GAGCATTGGGCCAAGGGGACCGCCTGCAAGAGCAGGCCCCTGAGCCAAGGGGACTGTCCACCCCTGGAGT TGAAGAATACCGTGCTCGAGGACGGCGACATGGTGGACACCGGCTACGGCGCTATGGATTTCTCCACCCTC CAGGACACCAAGTGCGAAGTGCCCCTCGACATCTGCCAAAGCATCTGCAAGTACCCCGACTACCTCCAGAT GAGCGCCGACCCCTACGGCGACAGCATGTTCTTCTGTCTCAGAAGGGAACAATTGTTCGCCCGCCACTTCTG GAACCGGGCCGGCACAATGGGAGATACAGTCCCCCAGAGCCTGTACATCAAGGGGACCGGAATGAGGGCC AGCCCCGGGTCCTGCGTCTACAGCCCAAGCCCCTCCGGGAGCATCGTCACAAGCGATAGCCAACTCTTCAA CAAGCCCTACTGGCTCCACAAAGCCCAAGGCCACAATAACGGGGTGTGTTGGCACAACCAGCTGTTCGTGA CCGTCGTGGACACAACCAGGTCCACAAACCTGACCATCTGCGCCAGCACCCAAAGCCCCGTGCCCGGCCAG TACGACGCCACAAAGTTCAAACAATACTCTCGGCACGTGGAAGAGTACGACCTCCAATTCATCTTCCAACT CTGCACCATCACCCTCACCGCCGACGTGATGAGCTACATCCACTCCATGAACTCCTCCATCCTGGAAGACTG GAATTTCGGCGTGCCACCACCCCCTACCACCTCCCTCGTCGACACCTACAGATTCGTGCAGAGCGTGGCCAT CACATGCCAGAAAGACGCCGCCCCCGCCGAGAACAAAGACCCATACGACAAACTGAAATTCTGGAACGTC GACCTGAAAGAGAAATTCAGCCTGGATCTGGACCAGTACCCATTGGGCAGGAAGTTCCTCAAAGCCAAGCC AAAACTGAAAAGGGCTGCCCCAACCAGCACCAGGACCTCCTCTGCCAAGAGGAAGAAGGTGAAGAAGTAA A |
| SEQ ID No: 44 | 1805 Synthetic HPV18 L1 gene |
| | ctgggtaccATGGCCCTCTGGAGACCATCCGATAACACAGTGTACTTGCCCCCACCCAGCGTCGCCCGGGTGGT GAACACAGACGACTACGTCACCAGAACCTCAATCTTCTACCACGCCGGGTCCAGCCGGCTGCTGACCGTGG GCAACCCCTACTTCCGCGTGCCCGCCGGCGGCGGAAACAAACAAGACATCCCCAAAGTCAGCGCCTATCAG TACCGGGTGTTCCGCGTCCAACTGCCCGATCCCAACAAGTTCGGCCTGCCCGACACCTCCATCTACAACCCC GAGACCCAGAGGCTGGTCTGGGCTTGCGCCGGCGTCGAGATCGGGAGGGGCCAACCCCTGGGCGTGGGGT TGTCCGGCCACCCCTTCTACAACAAGCTGGACGATACCGAGTCCAGCCACGCAGCAACCAGCAACGTCTCC GAAGATGTGCGCGATAACGTCAGCGTGGACTACAAACAAACCCAACTGTGCATCCTGGGATGCGCACCCGC CATCGGCGAGCATTGGGCCAAGGGGACCGCCTGCAAGAGCAGGCCCCTGAGCCAAGGGGACTGTCCACCC CTGGAGTTGAAGAATACCGTGCTCGAGGACGGCGACATGGTGGACACCGGCTACGGCGCTATGGATTTCTC CACCCTCCAGGACACCAAGTGCGAAGTGCCCCTCGACATCTGCCAAAGCATCTGCAAGTACCCCGACTACC TCCAGATGAGCGCCGACCCCTACGGCGACAGCATGTTCTTCTGTCTCAGAAGGGAACAATTGTTCGCCCGC CACTTCTGGAACCGGGCCGGCACAATGGGAGATACAGTCCCCCAGAGCCTGTACATCAAGGGGACCGGAA TGAGGGCCAGCCCCGGGTCCTGCGTCTACAGCCCAAGCCCCTCCGGGAGCATCGTCACAAGCGATAGCCAA CTCTTCAACAAGCCCTACTGGCTCCACAAAGCCCAAGGCCACAATAACGGGGTGTGTTGGCACAACCAGCT GTTCGTGACCGTCGTGGACACAACCAGGTCCACAAACCTGACCATCTGCGCCAGCACCCAAAGCCCCGTGC CCGGCCAGTACGACGCCACAAAGTTCAAACAATACTCTCGGCACGTGGAAGAGTACGACCTCCAATTCATC TTCCAACTCTGCACCATCACCCTCACCGCCGACGTGATGAGCTACATCCACTCCATGAACTCCTCCATCCTG GAAGACTGGAATTTCGGCGTGCCACCACCCCCTACCACCTCCCTCGTCGACACCTACAGATTCGTGCAGAG CGTGGCCATCACATGCCAGAAAGACGCCGCCCCCGCCGAGAACAAAGACCCATACGACAAACTGAAATTC TGGAACGTCGACCTGAAAGAGAAATTCAGCCTGGATCTGGACCAGTACCCATTGGGCAGGAAGTTCCTCGT GCAAGCCGGCCTCAGGAGAAAACCAACAATCGGGCCCAGGAAGAGGAGCGCCCCCAGCGCAACCACCAGC AGCAAGCCCGCAAAAAGGGTCAGAGTGAGGGCACGCAAATAAActcgagctc |
| SEQ ID No: 45 | 1806 Synthetic HPV33 L1 gene |
| | ctgggtaccATGAGTGTGTGGAGACCATCTGAGGCTACAGTCTACCTGCCTCCTGTGCCTGTGAGCAAGGTGGTG |
| | AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 46 | 1807HPV18 L1 F1 |
| | CTTGGTACCATGGCCCTCTGGAGACCATCCGATA |
| SEQ ID No: 47 | 1808HPV18 L1 R1 |
| | GCTTGGCTTTGAGGAACTTCCTGCCCAATGGGTAC |
| SEQ ID No: 48 | 1809 HPV18 L1 amplified sequence 1 |
| | CTTGGTACC[1] ATGGCCCTCTGGAGACCATCCGATAACACAGTGTACTTGCCCCCACCCAGCGTCGCCCGGGTGGTGAACAC AGACGACTACGTCACCAGAACCTCAATCTTCTACCACGCCGGGTCCAGCCGGCTGCTGACCGTGGGCAACC CCTACTTCCGCGTGCCCGCCGGCGGCGGAAACAAACAAGACATCCCCAAAGTCAGCGCCTATCAGTACCGG GTGTTCCGCGTCCAACTGCCCGATCCCAACAAGTTCGGCCTGCCCGACACCTCCATCTACAACCCCGAGACC CAGAGGCTGGTCTGGGCTTGCGCCGGCGTCGAGATCGGGAGGGGCCAACCCCTGGGCGTGGGGTTGTCCGG CCACCCCTTCTACAACAAGCTGGACGATACCGAGTCCAGCCACGCAGCAACCAGCAACGTCTCCGAAGATG TGCGCGATAACGTCAGCGTGGACTACAAACAAACCCAACTGTGCATCCTGGGATGCGCACCCGCCATCGGC GAGCATTGGGCCAAGGGGACCGCCTGCAAGAGCAGGCCCCTGAGCCAAGGGGACTGTCCACCCCTGGAGT TGAAGAATACCGTGCTCGAGGACGGCGACATGGTGGACACCGGCTACGGCGCTATGGATTTCTCCACCCTC CAGGACACCAAGTGCGAAGTGCCCCTCGACATCTGCCAAAGCATCTGCAAGTACCCCGACTACCTCCAGAT GAGCGCCGACCCCTACGGCGACAGCATGTTCTTCTGTCTCAGAAGGGAACAATTGTTCGCCCGCCACTTCTG GAACCGGGCCGGCACAATGGGAGATACAGTCCCCCAGAGCCTGTACATCAAGGGGACCGGAATGAGGGCC AGCCCCGGGTCCTGCGTCTACAGCCCAAGCCCCTCCGGGAGCATCGTCACAAGCGATAGCCAACTCTTCAA CAAGCCCTACTGGCTCCACAAAGCCCAAGGCCACAATAACGGGGTGTGTTGGCACAACCAGCTGTTCGTGA CCGTCGTGGACACAACCAGGTCCACAAACCTGACCATCTGCGCCAGCACCCAAAGCCCCGTGCCCGGCCAG TACGACGCCACAAAGTTCAAACAATACTCTCGGCACGTGGAAGAGTACGACCTCCAATTCATCTTCCAACT CTGCACCATCACCCTCACCGCCGACGTGATGAGCTACATCCACTCCATGAACTCCTCCATCCTGGAAGACTG GAATTTCGGCGTGCCACCACCCCCTACCACCTCCCTCGTCGACACCTACAGATTCGTGCAGAGCGTGGCCAT CACATGCCAGAAAGACGCCGCCCCCGCCGAGAACAAAGACCCATACGACAAACTGAAATTCTGGAACGTC GACCTGAAAGAGAAATTCAGCCTGGATCTGGACCAGTACCCATTGGGCAGGAAGTTCCTC[2]AAAGCCAAG C[3] |
| | [1]: Kpn I enzymatic cleavage site; |
| | [2]: the gene fragment encoding of HPV18 L1 aa 1-470; |
| | [3]: 10 bases overlapping with the gene fragment encoding HPV33L1 aa 474-499. |
| SEQ ID No: 49 | 1810HPV18 L1 F2 |
| | GAAGTTCCTCAAAGCCAAGCCAAAACTGAAAAGGG |
| SEQ ID No: 50 | 1811HPV18 L1 R2 |
| | CTGTCTAGATTTACTTCTTCACCTTCTTCCTCTTGG |
| SEQ ID No: 51 | 1812 HPV18 L1 amplified sequence 2 |
| | GAAGTTCCTC [1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV18 L1 aa 1-470; |
| | [2]: the gene fragment encoding the C-terminal 26 amino acids of HPV33L1 aa 474-499; |
| | [3]: XbaI enzymatic cleavage site. |
| SEQ ID No: 52 | 1813 The amino acid sequence of HPV type 59 L1 protein aa 471-508 |
| | LQLGARPKPTIGPRKRAAPAPTSTPSPKRVKRRKSSRK |

### Appendix 5 : SEQUENCE LISTING -CHIMERIC HUMAN PAPILLOMAVIRUS TYPE 31 L1 PROTEIN

| | |
|---|---|
| SEQ ID No: 53 | 3101 The amino acid sequence of HPV type 31 L1 protein aa 1-475 |
| | MSLWRPSEATVYLPPVPVSKVVSTDEYVTRTNIYYHAGSARLLTVGHPYYSIPK SDNPKKIVVPKVSGLQYRVFRVRLPDPNKFGFPDTSFYNPETQRLVWACVGLE VGRGQPLGVGISGHPLLNKFDDTENSNRYAGGPGTDNRECISMDYKQTQLCLL GCKPPIGEHWGKGSPCSNNAITPGDCPPLELKNSVIQDGDMVDTGFGAMDFTA LQDTKSNVPLDICNSICKYPDYLKMVAEPYGDTLFFYLRREQMFVRHFFNRSGT VGESVPTDLYIKGSGSTATLANSTYFPTPSGSMVTSDAQIFNKPYWMQRAQGH NNGICWGNQLFVTVVDTTRSTNMSVCAAIANSDTTFKSSNFKEYLRHGEEFDL QFIFQLCKITLSADIMTYIHSMNPAILEDWNFGLTTPPSGSLEDTYRFVTSQAITC QKSAPQKPKEDPFKDYVFWEVNLKEKFSADLDQFPLGRKFLLQAGY |
| SEQ ID No: 54 | 3102 The amino acid sequence of HPV type 33 L1 protein aa 474-499 |
| | KAKPKLKRAAPTSTRTSSAKRKKVKK |
| SEQ ID No: 55 | 3103 Amino acid sequence of chimeric HPV type 31 L1 protein |
| | MSLWRPSEATVYLPPVPVSKVVSTDEYVTRTNIYYHAGSARLLTVGHPYYSIPK SDNPKKIVVPKVSGLQYRVFRVRLPDPNKFGFPDTSFYNPETQRLVWACVGLE VGRGQPLGVGISGHPLLNKFDDTENSNRYAGGPGTDNRECISMDYKQTQLCLL GCKPPIGEHWGKGSPCSNNAITPGDCPPLELKNSVIQDGDMVDTGFGAMDFTA LQDTKSNVPLDICNSICKYPDYLKMVAEPYGDTLFFYLRREQMFVRHFFNRSGT VGESVPTDLYIKGSGSTATLANSTYFPTPSGSMVTSDAQIFNKPYWMQRAQGH NNGICWGNQLFVTVVDTTRSTNMSVCAAIANSDTTFKSSNFKEYLRHGEEFDL QFIFQLCKITLSADIMTYIHSMNPAILEDWNFGLTTPPSGSLEDTYRFVTSQAITC QKSAPQKPKEDPFKDYVFWEVNLKEKFSADLDQFPLGRKFLLQAGYKAKPKLK RAAPTSTRTSSAKRKKVKK |
| SEQ ID No: 56 | 3104 Nucleotide sequence of chimeric HPV type 31 L1 protein |
| | ATGAGCCTGTGGAGGCCCAGCGAGGCCACCGTGTACCTGCCCCCCGTGCCCGTGAGCAAGGTGGTGAGCAC CGACGAGTACGTGACCAGGACCAACATCTACTACCACGCCGGCAGCGCCAGGCTGCTGACCGTGGGCCACC CCTACTACAGCATCCCCAAGAGCGACAACCCCAAGAAGATCGTGGTGCCCAAGGTGAGCGGCCTGCAGTAC AGGGTGTTCAGGGTGAGGCTGCCCGACCCCAACAAGTTCGGCTTCCCCGACACCAGCTTCTACAACCCCGA GACCCAGAGGCTGGTGTGGGCCTGCGTGGGCCTGGAGGTGGGCAGGGGCCAGCCCCTGGGCGTGGGCATC AGCGGCCACCCCCTGCTGAACAAGTTCGACGACACCGAGAACAGCAACAGGTACGCCGGCGGCCCCGGCA CCGACAACAGGGAGTGCATCAGCATGGACTACAAGCAGACCCAGCTGTGCCTGCTGGGCTGCAAGCCCCCC ATCGGCGAGCACTGGGGCAAGGGCAGCCCCTGCAGCAACAACGCCATCACCCCCGGCGACTGCCCCCCCCT GGAGCTGAAGAACAGCGTGATCCAGGACGGCGACATGGTGGACACCGGCTTCGGCGCCATGGACTTCACC GCCCTGCAGGACACCAAGAGCAACGTGCCCCTGGACATCTGCAACAGCATCTGCAAGTACCCCGACTACCT GAAGATGGTGGCCGAGCCCTACGGCGACACCCTGTTCTTCTACCTGAGGAGGGAGCAGATGTTCGTGAGGC ACTTCTTCAACAGGAGCGGCACCGTGGGCGAGAGCGTGCCCACCGACCTGTACATCAAGGGCAGCGGCAG CACCGCCACCCTGGCCAACAGCACCTACTTCCCCACCCCCAGCGGCAGCATGGTGACCAGCGACGCCCAGA TCTTCAACAAGCCCTACTGGATGCAGAGGGCCCAGGGCCACAACAACGGCATCTGCTGGGGCAACCAGCTG TTCGTGACCGTGGTGGACACCACCAGGAGCACCAACATGAGCGTGTGCGCCGCCATCGCCAACAGCGACAC CACCTTCAAGAGCAGCAACTTCAAGGAGTACCTGAGGCACGGCGAGGAGTTCGACCTGCAGTTCATCTTCC AGCTGTGCAAGATCACCCTGAGCGCCGACATCATGACCTACATCCACAGCATGAACCCCGCCATCCTGGAG GACTGGAACTTCGGCCTGACCACCCCCCCCAGCGGCAGCCTGGAGGACACCTACAGGTTCGTGACCAGCCA GGCCATCACCTGCCAGAAGTCCGCCCCCCAGAAGCCCAAGGAGGACCCCTTCAAGGACTACGTGTTCTGGG AGGTGAACCTGAAGGAGAAGTTCAGCGCCGACCTGGACCAGTTCCCCCTGGGCAGGAAGTTCCTGCTGCAG GCCGGCTACAAAGCCAAGCCAAAACTGAAAAGGGCTGCCCCAACCAGCACCAGGACCTCCTCTGCCAAGA GGAAGAAGGTGAAGAAGTAAA |
| SEQ ID No: 57 | 3105 Synthetic HPV31 L1 gene |
| | ctgggtaccATGAGCCTGTGGAGGCCCAGCGAGGCCACCGTGTACCTGCCCCCCGTGCCCGTGAGCAAGGTGGT GAGCACCGACGAGTACGTGACCAGGACCAACATCTACTACCACGCCGGCAGCGCCAGGCTGCTGACCGTG GGCCACCCCTACTACAGCATCCCCAAGAGCGACAACCCCAAGAAGATCGTGGTGCCCAAGGTGAGCGGCCT GCAGTACAGGGTGTTCAGGGTGAGGCTGCCCGACCCCAACAAGTTCGGCTTCCCCGACACCAGCTTCTACA ACCCCGAGACCCAGAGGCTGGTGTGGGCCTGCGTGGGCCTGGAGGTGGGCAGGGGCCAGCCCCTGGGCGT GGGCATCAGCGGCCACCCCCTGCTGAACAAGTTCGACGACACCGAGAACAGCAACAGGTACGCCGGCGGC CCCGGCACCGACAACAGGGAGTGCATCAGCATGGACTACAAGCAGACCCAGCTGTGCCTGCTGGGCTGCA AGCCCCCCATCGGCGAGCACTGGGGCAAGGGCAGCCCCTGCAGCAACAACGCCATCACCCCCGGCGACTG CCCCCCCCTGGAGCTGAAGAACAGCGTGATCCAGGACGGCGACATGGTGGACACCGGCTTCGGCGCCATGG ACTTCACCGCCCTGCAGGACACCAAGAGCAACGTGCCCCTGGACATCTGCAACAGCATCTGCAAGTACCCC GACTACCTGAAGATGGTGGCCGAGCCCTACGGCGACACCCTGTTCTTCTACCTGAGGAGGGAGCAGATGTT CGTGAGGCACTTCTTCAACAGGAGCGGCACCGTGGGCGAGAGCGTGCCCACCGACCTGTACATCAAGGGCA GCGGCAGCACCGCCACCCTGGCCAACAGCACCTACTTCCCCACCCCCAGCGGCAGCATGGTGACCAGCGAC GCCCAGATCTTCAACAAGCCCTACTGGATGCAGAGGGCCCAGGGCCACAACAACGGCATCTGCTGGGGCA ACCAGCTGTTCGTGACCGTGGTGGACACCACCAGGAGCACCAACATGAGCGTGTGCGCCGCCATCGCCAAC AGCGACACCACCTTCAAGAGCAGCAACTTCAAGGAGTACCTGAGGCACGGCGAGGAGTTCGACCTGCAGTT CATCTTCCAGCTGTGCAAGATCACCCTGAGCGCCGACATCATGACCTACATCCACAGCATGAACCCCGCCA TCCTGGAGGACTGGAACTTCGGCCTGACCACCCCCCCCAGCGGCAGCCTGGAGGACACCTACAGGTTCGTG ACCAGCCAGGCCATCACCTGCCAGAAGTCCGCCCCCCAGAAGCCCAAGGAGGACCCCTTCAAGGACTACGT GTTCTGGGAGGTGAACCTGAAGGAGAAGTTCAGCGCCGACCTGGACCAGTTCCCCCTGGGCAGGAAGTTCC TGCTGCAGGCCGGCTACAGGGCCAGGCCCAAGTTCAAGGCCGGCAAGAGGAGCGCCCCCAGCGCCAGCAC CACCACCCCCGCCAAGAGGAAGAAGACCAAGAAGTAAActcgagctc |
| SEQ ID No: 58 | 3106 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 59 | 3107HPV31 L1 F1 |
| | CTTGGTACCATGAGCCTGTGGAGGCCCAGCGAG |
| SEQ ID No: 60 | 3108HPV31 L1 R1 |
| | GCTTGGCTTTGTAGCCGGCCTGCAGCAGGAACTTCCTG |
| SEQ ID No: 61 | 3109 HPV31 L1 amplified sequence 1 |
| | CTTGGTACC[1] ATGAGCCTGTGGAGGCCCAGCGAGGCCACCGTGTACCTGCCCCCCGTGCCCGTGAGCAAGGTGGTGAGCAC CGACGAGTACGTGACCAGGACCAACATCTACTACCACGCCGGCAGCGCCAGGCTGCTGACCGTGGGCCACC CCTACTACAGCATCCCCAAGAGCGACAACCCCAAGAAGATCGTGGTGCCCAAGGTGAGCGGCCTGCAGTAC AGGGTGTTCAGGGTGAGGCTGCCCGACCCCAACAAGTTCGGCTTCCCCGACACCAGCTTCTACAACCCCGA GACCCAGAGGCTGGTGTGGGCCTGCGTGGGCCTGGAGGTGGGCAGGGGCCAGCCCCTGGGCGTGGGCATC AGCGGCCACCCCCTGCTGAACAAGTTCGACGACACCGAGAACAGCAACAGGTACGCCGGCGGCCCCGGCA CCGACAACAGGGAGTGCATCAGCATGGACTACAAGCAGACCCAGCTGTGCCTGCTGGGCTGCAAGCCCCCC ATCGGCGAGCACTGGGGCAAGGGCAGCCCCTGCAGCAACAACGCCATCACCCCCGGCGACTGCCCCCCCCT GGAGCTGAAGAACAGCGTGATCCAGGACGGCGACATGGTGGACACCGGCTTCGGCGCCATGGACTTCACC GCCCTGCAGGACACCAAGAGCAACGTGCCCCTGGACATCTGCAACAGCATCTGCAAGTACCCCGACTACCT GAAGATGGTGGCCGAGCCCTACGGCGACACCCTGTTCTTCTACCTGAGGAGGGAGCAGATGTTCGTGAGGC ACTTCTTCAACAGGAGCGGCACCGTGGGCGAGAGCGTGCCCACCGACCTGTACATCAAGGGCAGCGGCAG CACCGCCACCCTGGCCAACAGCACCTACTTCCCCACCCCCAGCGGCAGCATGGTGACCAGCGACGCCCAGA TCTTCAACAAGCCCTACTGGATGCAGAGGGCCCAGGGCCACAACAACGGCATCTGCTGGGGCAACCAGCTG TTCGTGACCGTGGTGGACACCACCAGGAGCACCAACATGAGCGTGTGCGCCGCCATCGCCAACAGCGACAC CACCTTCAAGAGCAGCAACTTCAAGGAGTACCTGAGGCACGGCGAGGAGTTCGACCTGCAGTTCATCTTCC AGCTGTGCAAGATCACCCTGAGCGCCGACATCATGACCTACATCCACAGCATGAACCCCGCCATCCTGGAG GACTGGAACTTCGGCCTGACCACCCCCCCCAGCGGCAGCCTGGAGGACACCTACAGGTTCGTGACCAGCCA GGCCATCACCTGCCAGAAGTCCGCCCCCCAGAAGCCCAAGGAGGACCCCTTCAAGGACTACGTGTTCTGGG AGGTGAACCTGAAGGAGAAGTTCAGCGCCGACCTGGACCAGTTCCCCCTGGGCAGGAAGTTCCTGCTGCAG GCCGGCTAC[2]AAAGCCAAGC[3] |
| | [1]: Kpn I enzymatic cleavage site; |
| | [2]: the gene fragment encoding of HPV31 L1 aa 1-475; |
| | [3]: 10 bases overlapping with the gene fragment encoding HPV33L1 aa 474-499. |
| SEQ ID No: 62 | 3110HPV31 L1 F2 |
| | GGCCGGCTACAAAGCCAAGCCAAAACTGAAAAGGG |
| SEQ ID No: 63 | 3111HPV31 L1 R2 |
| | CTGTCTAGATTTACTTCTTCACCTTCTTCCTCTTGGCAGAG |
| SEQ ID No: 64 | 3112 HPV31 L1 amplified sequence 2 |
| | GGCCGGCTAC[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV31 L1 aa 1-475; |
| | [2]: the gene fragment encoding the C-terminal 26 amino acids of HPV33L1 aa 474-499; |
| | [3]: Xbal enzymatic cleavage site. |
| SEQ ID No: 65 | 3113 The amino acid sequence of HPV type 59 L1 protein aa 471-508 |
| | LQLGARPKPTIGPRKRAAPAPTSTPSPKRVKRRKSSRK |

### Appendix 6: SEQUENCE LISTING - HUMAN PAPILLOMAVIRUS TYPE 33 L1 PROTEIN

| | |
|---|---|
| SEQ ID No: 66 | 3301 The amino acid sequence of HPV type 33 L1 protein |
| | MSVWRPSEATVYLPPVPVSKVVSTDEYVSRTSIYYYAGSSRLLAVGHPYFSIKN PTNAKKLLVPKVSGLQYRVFRVRLPDPNKFGFPDTSFYNPDTQRLVWACVGLEI GRGQPLGVGISGHPLLNKFDDTETGNKYPGQPGADNRECLSMDYKQTQLCLLG CKPPTGEHWGKGVACTNAAPANDCPPLELINTIIEDGDMVDTGFGCMDFKTLQ ANKSDVPIDICGSTCKYPDYLKMTSEPYGDSLFFFLRREQMFVRHFFNRAGTLG EAVPDDLYIKGSGTTASIQSSAFFPTPSGSMVTSESQLFNKPYWLQRAQGHNNGI CWGNQVFVTVVDTTRSTNMTLCTQVTSDSTYKNENFKEYIRHVEEYDLQFVFQ LCKVTLTAEVMTYIHAMNPDILEDWQFGLTPPPSASLQDTYRFVTSQAITCQKT VPPKEKEDPLGKYTFWEVDLKEKFSADLDQFPLGRKFLLQAGLKAKPKLKRAA PTSTRTSSAKRKKVKK |
| SEQ ID No: 67 | 3302 Nucleotide sequence of HPV type 33 L1 protein |
| | ATGAGTGTGTGGAGACCATCTGAGGCTACAGTCTACCTGCCTCCTGTGCCTGTGAGCAAGGTGGTGAGCAC AGATGAATATGTGAGCAGGACCAGCATCTACTACTATGCTGGCTCCAGCAGACTGCTGGCTGTGGGACACC CATACTTCAGCATCAAGAACCCAACCAATGCCAAGAAACTGCTGGTGCCAAAGGTGTCTGGACTCCAATAC AGGGTGTTCAGGGTGAGACTGCCTGACCCAAACAAGTTTGGCTTTCCTGACACCTCCTTCTACAACCCTGAC ACCCAGAGACTGGTGTGGGCTTGTGTGGGATTGGAGATTGGCAGGGGACAACCACTGGGAGTGGGCATCTC TGGACACCCACTGCTGAACAAGTTTGATGACACAGAGACAGGCAACAAATACCCTGGACAACCTGGAGCA GACAACAGGGAGTGTCTGAGTATGGACTACAAGCAGACCCAACTTTGTCTGCTGGGCTGTAAGCCTCCAAC AGGAGAACACTGGGGCAAGGGAGTGGCTTGTACCAATGCTGCCCCTGCCAATGACTGTCCTCCATTGGAAC TGATAAACACCATCATTGAGGATGGAGATATGGTGGACACAGGCTTTGGCTGTATGGACTTCAAGACCCTC CAAGCCAACAAGTCTGATGTGCCAATTGACATCTGTGGCAGCACTTGTAAATACCCTGACTACCTGAAAAT GACCTCTGAACCATATGGAGACTCCCTGTTCTTCTTCCTGAGGAGGGAACAGATGTTTGTGAGACACTTCTT CAACAGGGCTGGCACCCTGGGAGAGGCTGTGCCTGATGACCTCTACATCAAGGGCTCTGGCACCACAGCCA GCATCCAGTCCTCTGCCTTCTTTCCAACACCATCTGGCAGTATGGTGACCTCTGAGAGCCAACTTTTCAACA AGCCATACTGGCTCCAAAGGGCTCAAGGACACAACAATGGCATCTGTTGGGGCAACCAGGTGTTTGTGACA GTGGTGGACACCACCAGGAGCACCAATATGACCCTGTGTACCCAGGTGACCTCTGACAGCACCTACAAGAA TGAGAACTTCAAGGAATACATCAGGCATGTGGAGGAATATGACCTCCAATTTGTGTTCCAACTTTGTAAGG TGACCCTGACAGCAGAGGTGATGACCTACATCCATGCTATGAACCCTGACATCTTGGAGGACTGGCAGTTT GGACTGACACCTCCTCCATCTGCCTCCCTCCAAGACACCTACAGGTTTGTGACCAGCCAGGCTATCACTTGT CAGAAGACAGTGCCTCCAAAGGAGAAGGAGGACCCACTGGGCAAATACACCTTCTGGGAGGTGGACCTGA AAGAGAAGTTCTCTGCTGACCTGGACCAGTTTCCACTGGGCAGGAAGTTCCTGCTCCAAGCAGGACTGAAA GCCAAGCCAAAACTGAAAAGGGCTGCCCCAACCAGCACCAGGACCTCCTCTGCCAAGAGGAAGAAGGTGA AGAAGTAAA |
| SEQ ID No: 68 | 3303 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |

### Appendix 7 : SEQUENCE LISTING -CHIMERIC HUMAN PAPILLOMAVIRUS TYPE 35 L1 PROTEIN

| | |
|---|---|
| SEQ ID No: 69 | 3501 The amino acid sequence of HPV type 35 L1 protein aa 1-472 |
| | MSLWRSNEATVYLPPVSVSKVVSTDEYVTRTNIYYHAGSSRLLAVGHPYYAIK KQDSNKIAVPKVSGLQYRVFRVKLPDPNKFGFPDTSFYDPASQRLVWACTGVE VGRGQPLGVGISGHPLLNKLDDTENSNKYVGNSGTDNRECISMDYKQTQLCLI GCRPPIGEHWGKGTPCNANQVKAGECPPLELLNTVLQDGDMVDTGFGAMDFT TLQANKSDVPLDICSSICKYPDYLKMVSEPYGDMLFFYLRREQMFVRHLFNRA GTVGETVPADLYIKGTTGTLPSTSYFPTPSGSMVTSDAQIFNKPYWLQRAQGHN NGICWSNQLFVTVVDTTRSTNMSVCSAVSSSDSTYKNDNFKEYLRHGEEYDLQ FIFQLCKITLTADVMTYIHSMNPSILEDWNFGLTPPPSGTLEDTYRYVTSQAVTC QKPSAPKPKDDPLKNYTFWEVDLKEKFSADLDQFPLGRKFLLQAGL |
| SEQ ID No: 70 | 3502 The amino acid sequence of HPV type 33 L1 protein aa 474-499 |
| | KAKPKLKRAAPTSTRTSSAKRKKVKK |
| SEQ ID No: 71 | 3503 Amino acid sequence of chimeric HPV type 35 L1 protein |
| | MSLWRSNEATVYLPPVSVSKVVSTDEYVTRTNIYYHAGSSRLLAVGHPYYAIK KQDSNKIAVPKVSGLQYRVFRVKLPDPNKFGFPDTSFYDPASQRLVWACTGVE VGRGQPLGVGISGHPLLNKLDDTENSNKYVGNSGTDNRECISMDYKQTQLCLI GCRPPIGEHWGKGTPCNANQVKAGECPPLELLNTVLQDGDMVDTGFGAMDFT TLQANKSDVPLDICSSICKYPDYLKMVSEPYGDMLFFYLRREQMFVRHLFNRA GTVGETVPADLYIKGTTGTLPSTSYFPTPSGSMVTSDAQIFNKPYWLQRAQGHN NGICWSNQLFVTVVDTTRSTNMSVCSAVSSSDSTYKNDNFKEYLRHGEEYDLQ FIFQLCKITLTADVMTYIHSMNPSILEDWNFGLTPPPSGTLEDTYRYVTSQAVTC QKPSAPKPKDDPLKNYTFWEVDLKEKFSADLDQFPLGRKFLLQAGLKAKPKLK RAAPTSTRTSSAKRKKVKK |
| SEQ ID No: 72 | 3504 Nucleotide sequence of chimeric HPV type 35 L1 protein |
| | ATGAGTCTGTGGAGGAGCAATGAGGCTACAGTCTACCTGCCTCCTGTGTCTGTGAGCAAGGTGGTGAGCAC AGATGAATATGTGACCAGGACCAACATCTACTACCATGCTGGCTCCAGCAGACTGCTGGCTGTGGGACACC CATACTATGCCATCAAGAAGCAGGACAGCAACAAGATTGCTGTGCCAAAGGTGTCTGGACTCCAATACAGG GTGTTCAGGGTGAAACTGCCTGACCCAAACAAGTTTGGCTTTCCTGACACCTCCTTCTATGACCCTGCCAGC CAGAGACTGGTGTGGGCTTGTACTGGAGTGGAGGTGGGCAGGGGACAACCACTGGGAGTGGGCATCTCTG GACACCCACTGCTGAACAAACTGGATGACACAGAGAACAGCAACAAATATGTGGGCAACTCTGGCACAGA CAACAGGGAGTGTATCAGTATGGACTACAAGCAGACCCAACTTTGTCTGATTGGCTGTAGACCTCCAATTG GAGAACACTGGGGCAAGGGCACACCATGTAATGCCAACCAGGTGAAGGCTGGAGAGTGTCCTCCATTGGA ACTGCTGAACACAGTGCTCCAAGATGGAGATATGGTGGACACAGGCTTTGGAGCTATGGACTTCACCACCC TCCAAGCCAACAAGTCTGATGTGCCACTGGACATCTGTTCCAGCATCTGTAAATACCCTGACTACCTGAAA ATGGTGTCTGAACCATATGGAGATATGCTGTTCTTCTACCTGAGGAGGGAACAGATGTTTGTGAGACACCT GTTCAACAGGGCTGGCACAGTGGGAGAGACAGTGCCTGCTGACCTCTACATCAAGGGCACCACAGGCACCC TGCCAAGCACCTCCTACTTTCCAACACCATCTGGCAGTATGGTGACCTCTGATGCCCAGATTTTCAACAAGC CATACTGGCTCCAAAGGGCTCAAGGACACAACAATGGCATCTGTTGGAGCAACCAACTTTTTGTGACAGTG GTGGACACCACCAGGAGCACCAATATGAGTGTGTGTTCTGCTGTGTCCTCCTCTGACAGCACCTACAAGAA TGACAACTTCAAGGAATACCTGAGACATGGAGAGGAATATGACCTCCAATTCATCTTCCAACTTTGTAAGA TTACCCTGACAGCAGATGTGATGACCTACATCCACAGTATGAACCCAAGCATCTTGGAGGACTGGAACTTT GGACTGACACCTCCTCCATCTGGCACCTTGGAGGACACCTACAGATATGTGACCAGCCAGGCTGTGACTTG TCAGAAGCCATCTGCCCCAAAGCCAAAGGATGACCCACTGAAAAACTACACCTTCTGGGAGGTGGACCTGA AAGAGAAGTTCTCTGCTGACCTGGACCAGTTTCCACTGGGCAGGAAGTTCCTGCTCCAAGCAGGACTGAAA GCCAAGCCAAAACTGAAAAGGGCTGCCCCAACCAGCACCAGGACCTCCTCTGCCAAGAGGAAGAAGGTGA AGAAGTAAA |
| | |
| SEQ ID No: 73 | 3505 Synthetic HPV35 L1 gene |
| | ctgggtaccATGAGTCTGTGGAGGAGCAATGAGGCTACAGTCTACCTGCCTCCTGTGTCTGTGAGCAAGGTGGT GAGCACAGATGAATATGTGACCAGGACCAACATCTACTACCATGCTGGCTCCAGCAGACTGCTGGCTGTGG GACACCCATACTATGCCATCAAGAAGCAGGACAGCAACAAGATTGCTGTGCCAAAGGTGTCTGGACTCCAA TACAGGGTGTTCAGGGTGAAACTGCCTGACCCAAACAAGTTTGGCTTTCCTGACACCTCCTTCTATGACCCT GCCAGCCAGAGACTGGTGTGGGCTTGTACTGGAGTGGAGGTGGGCAGGGGACAACCACTGGGAGTGGGCA TCTCTGGACACCCACTGCTGAACAAACTGGATGACACAGAGAACAGCAACAAATATGTGGGCAACTCTGGC ACAGACAACAGGGAGTGTATCAGTATGGACTACAAGCAGACCCAACTTTGTCTGATTGGCTGTAGACCTCC AATTGGAGAACACTGGGGCAAGGGCACACCATGTAATGCCAACCAGGTGAAGGCTGGAGAGTGTCCTCCA TTGGAACTGCTGAACACAGTGCTCCAAGATGGAGATATGGTGGACACAGGCTTTGGAGCTATGGACTTCAC CACCCTCCAAGCCAACAAGTCTGATGTGCCACTGGACATCTGTTCCAGCATCTGTAAATACCCTGACTACCT GAAAATGGTGTCTGAACCATATGGAGATATGCTGTTCTTCTACCTGAGGAGGGAACAGATGTTTGTGAGAC ACCTGTTCAACAGGGCTGGCACAGTGGGAGAGACAGTGCCTGCTGACCTCTACATCAAGGGCACCACAGGC ACCCTGCCAAGCACCTCCTACTTTCCAACACCATCTGGCAGTATGGTGACCTCTGATGCCCAGATTTTCAAC AAGCCATACTGGCTCCAAAGGGCTCAAGGACACAACAATGGCATCTGTTGGAGCAACCAACTTTTTGTGAC AGTGGTGGACACCACCAGGAGCACCAATATGAGTGTGTGTTCTGCTGTGTCCTCCTCTGACAGCACCTACA AGAATGACAACTTCAAGGAATACCTGAGACATGGAGAGGAATATGACCTCCAATTCATCTTCCAACTTTGT AAGATTACCCTGACAGCAGATGTGATGACCTACATCCACAGTATGAACCCAAGCATCTTGGAGGACTGGAA CTTTGGACTGACACCTCCTCCATCTGGCACCTTGGAGGACACCTACAGATATGTGACCAGCCAGGCTGTGA CTTGTCAGAAGCCATCTGCCCCAAAGCCAAAGGATGACCCACTGAAAAACTACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAGACCAAACTTCAGACTGGGCAAGAGGGCTGCCCCTGCCAGCACCAGCAAGAAGTCCAGCACC AAGAGGAGGAAGGTGAAGAGCTAAActcgagctc |
| SEQ ID No: 74 | 3506 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 75 | 3507HPV35 L1 F1 |
| | CTTGGTACCATGAGTCTGTGGAGGAGCAATGAGG |
| SEQ ID No: 76 | 3508HPV35 L1 R1 |
| | GCTTGGCTTTCAGTCCTGCTTGGAGCAGGAACTTCC |
| SEQ ID No: 77 | 3509 HPV35 L1 amplified sequence 1 |
| | CTTGGTACC[1] ATGAGTCTGTGGAGGAGCAATGAGGCTACAGTCTACCTGCCTCCTGTGTCTGTGAGCAAGGTGGTGAGCAC AGATGAATATGTGACCAGGACCAACATCTACTACCATGCTGGCTCCAGCAGACTGCTGGCTGTGGGACACC CATACTATGCCATCAAGAAGCAGGACAGCAACAAGATTGCTGTGCCAAAGGTGTCTGGACTCCAATACAGG GTGTTCAGGGTGAAACTGCCTGACCCAAACAAGTTTGGCTTTCCTGACACCTCCTTCTATGACCCTGCCAGC CAGAGACTGGTGTGGGCTTGTACTGGAGTGGAGGTGGGCAGGGGACAACCACTGGGAGTGGGCATCTCTG GACACCCACTGCTGAACAAACTGGATGACACAGAGAACAGCAACAAATATGTGGGCAACTCTGGCACAGA CAACAGGGAGTGTATCAGTATGGACTACAAGCAGACCCAACTTTGTCTGATTGGCTGTAGACCTCCAATTG GAGAACACTGGGGCAAGGGCACACCATGTAATGCCAACCAGGTGAAGGCTGGAGAGTGTCCTCCATTGGA ACTGCTGAACACAGTGCTCCAAGATGGAGATATGGTGGACACAGGCTTTGGAGCTATGGACTTCACCACCC TCCAAGCCAACAAGTCTGATGTGCCACTGGACATCTGTTCCAGCATCTGTAAATACCCTGACTACCTGAAA ATGGTGTCTGAACCATATGGAGATATGCTGTTCTTCTACCTGAGGAGGGAACAGATGTTTGTGAGACACCT GTTCAACAGGGCTGGCACAGTGGGAGAGACAGTGCCTGCTGACCTCTACATCAAGGGCACCACAGGCACCC TGCCAAGCACCTCCTACTTTCCAACACCATCTGGCAGTATGGTGACCTCTGATGCCCAGATTTTCAACAAGC CATACTGGCTCCAAAGGGCTCAAGGACACAACAATGGCATCTGTTGGAGCAACCAACTTTTTGTGACAGTG GTGGACACCACCAGGAGCACCAATATGAGTGTGTGTTCTGCTGTGTCCTCCTCTGACAGCACCTACAAGAA TGACAACTTCAAGGAATACCTGAGACATGGAGAGGAATATGACCTCCAATTCATCTTCCAACTTTGTAAGA TTACCCTGACAGCAGATGTGATGACCTACATCCACAGTATGAACCCAAGCATCTTGGAGGACTGGAACTTT GGACTGACACCTCCTCCATCTGGCACCTTGGAGGACACCTACAGATATGTGACCAGCCAGGCTGTGACTTG TCAGAAGCCATCTGCCCCAAAGCCAAAGGATGACCCACTGAAAAACTACACCTTCTGGGAGGTGGACCTGA AAGAGAAGTTCTCTGCTGACCTGGACCAGTTTCCACTGGGCAGGAAGTTCCTGCTCCAAGCAGGACTG[2]AA AGCCAAGC[3] |
| | [1]: Kpn I enzymatic cleavage site; |
| | [2]: the gene fragment encoding of HPV35 L1 aa 1-472; |
| | [3]: 10 bases overlapping with the gene fragment encoding HPV33L1 aa 474-499. |
| SEQ ID No: 78 | 3510HPV35 L1 F2 |
| | AGCAGGACTGAAAGCCAAGCCAAAACTGAAAAGGG |
| SEQ ID No: 79 | 3511HPV35 L1 R2 |
| | CTGTCTAGATTTACTTCTTCACCTTCTTCCTCTTGG |
| SEQ ID No: 80 | 3512 HPV35 L1 amplified sequence 2 |
| | AGCAGGACTG[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV35 L1 aa 1-472; |
| | [2]: the gene fragment encoding the C-terminal 26 amino acids of HPV33L1 aa 474-499; |
| | [3]: Xbal enzymatic cleavage site. |
| SEQ ID No: 81 | 3513 The amino acid sequence of HPV type 59 L1 protein aa 471-508 |
| | LQLGARPKPTIGPRKRAAPAPTSTPSPKRVKRRKSSRK |

### Appendix 8 : SEQUENCE LISTING -CHIMERIC HUMAN PAPILLOMAVIRUS TYPE 39 L1 PROTEIN

| | |
|---|---|
| SEQ ID No: 82 | 3901 The amino acid sequence of HPV type 39 L1 protein aa 1-469 |
| | MAMWRSSDSMVYLPPPSVAKVVNTDDYVTRTGIYYYAGSSRLLTVGHPYFKV GMNGGRKQDIPKVSAYQYRVFRVTLPDPNKFSIPDASLYNPETQRLVWACVGV EVGRGQPLGVGISGHPLYNRQDDTENSPFSSTTNKDSRDNVSVDYKQTQLCIIG CVPAIGEHWGKGKACKPNNVSTGDCPPLELVNTPIEDGDMIDTGYGAMDFGAL QETKSEVPLDICQSICKYPDYLQMSADVYGDSMFFCLRREQLFARHFWNRGGM VGDAIPAQLYIKGTDIRANPGSSVYCPSPSGSMVTSDSQLFNKPYWLHKAQGHN NGICWHNQLFLTVVDTTRSTNFTLSTSIESSIPSTYDPSKFKEYTRHVEEYDLQFI FQLCTVTLTTDVMSYIHTMNSSILDNWNFAVAPPPSASLVDTYRYLQSAAITCQ KDAPAPEKKDPYDGLKFWNVDLREKFSLELDQFPLGRKFL |
| SEQ ID No: 83 | 3902 The amino acid sequence of HPV type 59 L1 protein aa 471-508 |
| | LQLGARPKPTIGPRKRAAPAPTSTPSPKRVKRRKSSRK |
| SEQ ID No: 84 | 3903 Amino acid sequence of chimeric HPV type 39 L1 protein |
| | MAMWRSSDSMVYLPPPSVAKVVNTDDYVTRTGIYYYAGSSRLLTVGHPYFKV GMNGGRKQDIPKVSAYQYRVFRVTLPDPNKFSIPDASLYNPETQRLVWACVGV EVGRGQPLGVGISGHPLYNRQDDTENSPFSSTTNKDSRDNVSVDYKQTQLCIIG CVPAIGEHWGKGKACKPNNVSTGDCPPLELVNTPIEDGDMIDTGYGAMDFGAL QETKSEVPLDICQSICKYPDYLQMSADVYGDSMFFCLRREQLFARHFWNRGGM VGDAIPAQLYIKGTDIRANPGSSVYCPSPSGSMVTSDSQLFNKPYWLHKAQGHN NGICWHNQLFLTVVDTTRSTNFTLSTSIESSIPSTYDPSKFKEYTRHVEEYDLQFI FQLCTVTLTTDVMSYIHTMNSSILDNWNFAVAPPPSASLVDTYRYLQSAAITCQ KDAPAPEKKDPYDGLKFWNVDLREKFSLELDQFPLGRKFLLQLGARPKPTIGPR KRAAPAPTSTPSPKRVKRRKSSRK |
| SEQ ID No: 85 | 3904 Nucleotide sequence of chimeric HPV type 39 L1 protein |
| | ATGGCTATGTGGAGGTCCTCTGACAGTATGGTCTACCTGCCTCCTCCATCTGTGGCTAAGGTGGTGAACACA GATGACTATGTGACCAGGACAGGCATCTACTACTATGCTGGCTCCAGCAGACTGCTGACAGTGGGACACCC ATACTTCAAGGTGGGGATGAATGGAGGCAGGAAGCAGGACATCCCAAAGGTGTCTGCCTACCAATACAGG GTGTTCAGGGTGACCCTGCCTGACCCAAACAAGTTCAGCATCCCTGATGCCTCCCTCTACAACCCTGAGACC CAGAGACTGGTGTGGGCTTGTGTGGGAGTGGAGGTGGGCAGGGGACAACCACTGGGAGTGGGCATCTCTG GACACCCACTCTACAACAGACAGGATGACACAGAGAACAGCCCATTCTCCAGCACCACCAACAAGGACAG CAGGGACAATGTGTCTGTGGACTACAAGCAGACCCAACTTTGTATCATTGGCTGTGTGCCTGCCATTGGAG AACACTGGGGCAAGGGCAAGGCTTGTAAGCCAAACAATGTGAGCACAGGAGACTGTCCTCCATTGGAACT GGTGAACACACCAATTGAGGATGGAGATATGATTGACACAGGCTATGGAGCTATGGACTTTGGAGCCCTCC AAGAGACCAAGTCTGAGGTGCCACTGGACATCTGTCAGAGCATCTGTAAATACCCTGACTACCTCCAAATG AGTGCTGATGTCTATGGAGACAGTATGTTCTTCTGTCTGAGGAGGGAACAACTTTTTGCCAGACACTTCTGG AACAGGGGAGGGATGGTGGGAGATGCCATCCCTGCCCAACTCTACATCAAGGGCACAGACATCAGGGCTA ACCCTGGCTCCTCTGTCTACTGTCCAAGCCCATCTGGCAGTATGGTGACCTCTGACAGCCAACTTTTCAACA AGCCATACTGGCTGCACAAGGCTCAAGGACACAACAATGGCATCTGTTGGCACAACCAACTTTTCCTGACA GTGGTGGACACCACCAGGAGCACCAACTTCACCCTGAGCACCAGCATTGAGTCCAGCATCCCAAGCACCTA TGACCCAAGCAAGTTCAAGGAATACACCAGGCATGTGGAGGAATATGACCTCCAATTCATCTTCCAACTTT GTACTGTGACCCTGACCACAGATGTGATGAGTTACATCCACACAATGAACTCCAGCATCCTGGACAACTGG AACTTTGCTGTGGCTCCTCCTCCATCTGCCTCCCTGGTGGACACCTACAGATACCTCCAATCTGCTGCCATC ACTTGTCAGAAGGATGCCCCTGCCCCTGAGAAGAAGGACCCATATGATGGACTGAAGTTCTGGAATGTGGA CCTGAGGGAGAAGTTCTCCTTGGAACTGGACCAGTTTCCACTGGGCAGGAAGTTCCTGCTCCAACTTGGAG CCAGACCAAAGCCAACCATTGGACCAAGGAAGAGGGCTGCCCCTGCCCCAACCAGCACACCAAGCCCAAA GAGGGTGAAGAGGAGGAAGTCCAGCAGGAAGTAAA |
| | |
| SEQ ID No: 86 | 3905 Synthetic HPV39 L1 gene |
| | ctgggtaccATGGCTATGTGGAGGTCCTCTGACAGTATGGTCTACCTGCCTCCTCCATCTGTGGCTAAGGTGGTG AACACAGATGACTATGTGACCAGGACAGGCATCTACTACTATGCTGGCTCCAGCAGACTGCTGACAGTGGG ACACCCATACTTCAAGGTGGGGATGAATGGAGGCAGGAAGCAGGACATCCCAAAGGTGTCTGCCTACCAA TACAGGGTGTTCAGGGTGACCCTGCCTGACCCAAACAAGTTCAGCATCCCTGATGCCTCCCTCTACAACCCT GAGACCCAGAGACTGGTGTGGGCTTGTGTGGGAGTGGAGGTGGGCAGGGGACAACCACTGGGAGTGGGCA TCTCTGGACACCCACTCTACAACAGACAGGATGACACAGAGAACAGCCCATTCTCCAGCACCACCAACAAG GACAGCAGGGACAATGTGTCTGTGGACTACAAGCAGACCCAACTTTGTATCATTGGCTGTGTGCCTGCCAT TGGAGAACACTGGGGCAAGGGCAAGGCTTGTAAGCCAAACAATGTGAGCACAGGAGACTGTCCTCCATTG GAACTGGTGAACACACCAATTGAGGATGGAGATATGATTGACACAGGCTATGGAGCTATGGACTTTGGAGC CCTCCAAGAGACCAAGTCTGAGGTGCCACTGGACATCTGTCAGAGCATCTGTAAATACCCTGACTACCTCC AAATGAGTGCTGATGTCTATGGAGACAGTATGTTCTTCTGTCTGAGGAGGGAACAACTTTTTGCCAGACACT TCTGGAACAGGGGAGGGATGGTGGGAGATGCCATCCCTGCCCAACTCTACATCAAGGGCACAGACATCAG GGCTAACCCTGGCTCCTCTGTCTACTGTCCAAGCCCATCTGGCAGTATGGTGACCTCTGACAGCCAACTTTT CAACAAGCCATACTGGCTGCACAAGGCTCAAGGACACAACAATGGCATCTGTTGGCACAACCAACTTTTCC TGACAGTGGTGGACACCACCAGGAGCACCAACTTCACCCTGAGCACCAGCATTGAGTCCAGCATCCCAAGC ACCTATGACCCAAGCAAGTTCAAGGAATACACCAGGCATGTGGAGGAATATGACCTCCAATTCATCTTCCA ACTTTGTACTGTGACCCTGACCACAGATGTGATGAGTTACATCCACACAATGAACTCCAGCATCCTGGACA ACTGGAACTTTGCTGTGGCTCCTCCTCCATCTGCCTCCCTGGTGGACACCTACAGATACCTCCAATCTGCTG CCATCACTTGTCAGAAGGATGCCCCTGCCCCTGAGAAGAAGGACCCATATGATGGACTGAAGTTCTGGAAT GTGGACCTGAGGGAGAAGTTCTCCTTGGAACTGGACCAGTTTCCACTGGGCAGGAAGTTCCTGCTCCAAGC CAGGGTGAGGAGGAGACCAACCATTGGACCAAGGAAGAGACCTGCTGCCAGCACCTCCTCCTCCTCTGCCA CCAAACACAAGAGGAAGAGGGTGAGCAAGTAAActcgagctc |
| SEQ ID No: 87 | 3906 Synthetic HPV59 L1 gene |
| | ctgggtaccATGGCTCTGTGGAGGTCCTCTGACAACAAGGTCTACCTGCCTCCTCCATCTGTGGCTAAGGTGGTG AGCACAGATGAATATGTGACCAGGACCAGCATCTTCTACCATGCTGGCTCCAGCAGACTGCTGACAGTGGG ACACCCATACTTCAAGGTGCCAAAGGGAGGCAATGGCAGACAGGATGTGCCAAAGGTGTCTGCCTACCAAT ACAGGGTGTTCAGGGTGAAACTGCCTGACCCAAACAAGTTTGGACTGCCTGACAACACAGTCTATGACCCA AACAGCCAGAGACTGGTGTGGGCTTGTGTGGGAGTGGAGATTGGCAGGGGACAACCACTGGGAGTGGGAC TGTCTGGACACCCACTCTACAACAAACTGGATGACACAGAGAACTCTCATGTGGCATCTGCTGTGGACACC AAGGACACCAGGGACAATGTGTCTGTGGACTACAAGCAGACCCAACTTTGTATCATTGGCTGTGTGCCTGC CATTGGAGAACACTGGACCAAGGGCACAGCCTGTAAGCCAACCACAGTGGTCCAGGGAGACTGTCCTCCAT TGGAACTGATAAACACACCAATTGAGGATGGAGATATGGTGGACACAGGCTATGGAGCTATGGACTTCAA ACTGCTCCAAGACAACAAGTCTGAGGTGCCACTGGACATCTGTCAGAGCATCTGTAAATACCCTGACTACC TCCAAATGAGTGCTGATGCCTATGGAGACAGTATGTTCTTCTGTCTGAGGAGGGAACAGGTGTTTGCCAGA CACTTCTGGAACAGGTCTGGCACAATGGGAGACCAACTTCCTGAGTCCCTCTACATCAAGGGCACAGACAT CAGGGCTAACCCTGGCTCCTACCTCTACAGCCCAAGCCCATCTGGCTCTGTGGTGACCTCTGACAGCCAACT TTTCAACAAGCCATACTGGCTGCACAAGGCTCAAGGACTGAACAATGGCATCTGTTGGCACAACCAACTTT TCCTGACAGTGGTGGACACCACCAGGAGCACCAACCTGTCTGTGTGTGCCAGCACCACCTCCAGCATCCCA AATGTCTACACACCAACCTCCTTCAAGGAATATGCCAGGCATGTGGAGGAGTTTGACCTCCAATTCATCTTC CAACTTTGTAAGATTACCCTGACCACAGAGGTGATGAGTTACATCCACAATATGAACACCACCATCTTGGA GGACTGGAACTTTGGAGTGACACCTCCTCCAACAGCCTCCCTGGTGGACACCTACAGGTTTGTCCAGTCTGC TGCTGTGACTTGTCAGAAGGACACAGCCCCTCCTGTGAAGCAGGACCCATATGACAAACTGAAGTTCTGGC CTGTGGACCTGAAAGAGAGGTTCTCTGCTGACCTGGACCAGTTTCCACTGGGCAGGAAGTTCCTGCTCCAA CTTGGAGCCAGACCAAAGCCAACCATTGGACCAAGGAAGAGGGCTGCCCCTGCCCCAACCAGCACACCAA GCCCAAAGAGGGTGAAGAGGAGGAAGTCCAGCAGGAAGTAAActcgagctc |
| SEQ ID No: 88 | 3907HPV39 L1 F1 |
| | CTTGGTACCATGGCTATGTGGAGGTCCTCTGACAGTAT |
| SEQ ID No: 89 | 3908HPV39 L1 R1 |
| | TCCAAGTTGGAGCAGGAACTTCCTGCCCAGTGGAAACT |
| SEQ ID No: 90 | 3909 HPV39 L1 amplified sequence 1 |
| | CTTGGTACC[1] ATGGCTATGTGGAGGTCCTCTGACAGTATGGTCTACCTGCCTCCTCCATCTGTGGCTAAGGTGGTGAACACA GATGACTATGTGACCAGGACAGGCATCTACTACTATGCTGGCTCCAGCAGACTGCTGACAGTGGGACACCC ATACTTCAAGGTGGGGATGAATGGAGGCAGGAAGCAGGACATCCCAAAGGTGTCTGCCTACCAATACAGG GTGTTCAGGGTGACCCTGCCTGACCCAAACAAGTTCAGCATCCCTGATGCCTCCCTCTACAACCCTGAGACC CAGAGACTGGTGTGGGCTTGTGTGGGAGTGGAGGTGGGCAGGGGACAACCACTGGGAGTGGGCATCTCTG GACACCCACTCTACAACAGACAGGATGACACAGAGAACAGCCCATTCTCCAGCACCACCAACAAGGACAG CAGGGACAATGTGTCTGTGGACTACAAGCAGACCCAACTTTGTATCATTGGCTGTGTGCCTGCCATTGGAG AACACTGGGGCAAGGGCAAGGCTTGTAAGCCAAACAATGTGAGCACAGGAGACTGTCCTCCATTGGAACT GGTGAACACACCAATTGAGGATGGAGATATGATTGACACAGGCTATGGAGCTATGGACTTTGGAGCCCTCC AAGAGACCAAGTCTGAGGTGCCACTGGACATCTGTCAGAGCATCTGTAAATACCCTGACTACCTCCAAATG AGTGCTGATGTCTATGGAGACAGTATGTTCTTCTGTCTGAGGAGGGAACAACTTTTTGCCAGACACTTCTGG AACAGGGGAGGGATGGTGGGAGATGCCATCCCTGCCCAACTCTACATCAAGGGCACAGACATCAGGGCTA ACCCTGGCTCCTCTGTCTACTGTCCAAGCCCATCTGGCAGTATGGTGACCTCTGACAGCCAACTTTTCAACA AGCCATACTGGCTGCACAAGGCTCAAGGACACAACAATGGCATCTGTTGGCACAACCAACTTTTCCTGACA GTGGTGGACACCACCAGGAGCACCAACTTCACCCTGAGCACCAGCATTGAGTCCAGCATCCCAAGCACCTA TGACCCAAGCAAGTTCAAGGAATACACCAGGCATGTGGAGGAATATGACCTCCAATTCATCTTCCAACTTT GTACTGTGACCCTGACCACAGATGTGATGAGTTACATCCACACAATGAACTCCAGCATCCTGGACAACTGG AACTTTGCTGTGGCTCCTCCTCCATCTGCCTCCCTGGTGGACACCTACAGATACCTCCAATCTGCTGCCATC ACTTGTCAGAAGGATGCCCCTGCCCCTGAGAAGAAGGACCCATATGATGGACTGAAGTTCTGGAATGTGGA CCTGAGGGAGAAGTTCTCCTTGGAACTGGACCAGTTTCCACTGGGCAGGAAGTTCCTG[2] CTCCAACTTGGA[3] |
| | [1]: Kpn I enzymatic cleavage site; |
| | [2]: the gene fragment encoding of HPV39 L1 aa 1-469; |
| | [3]: 12 bases overlapping with the gene fragment encoding HPV59L1 aa 471-508. |
| SEQ ID No: 91 | 3910HPV39 L1 F2 |
| | AGGAAGTTCCTGCTCCAACTTGGAGCCAGACCAAAGC |
| SEQ ID No: 92 | 3911HPV39 L1 R2 |
| | CTGTCTAGATTTACTTCCTGCTGGACTTCCTCCTCTTCAC |
| SEQ ID No: 93 | 3912 HPV39 L1 amplified sequence 2 |
| | AGGAAGTTCCTG[1] CTCCAACTTGGAGCCAGACCAAAGCCAACCATTGGACCAAGGAAGAGGGCTGCCCCTGCCCCAACCAGCA CACCAAGCCCAAAGAGGGTGAAGAGGAGGAAGTCCAGCAGGAAG[2] TAA ATCTAGA[3]CAG. |
| | [1]: 12bp overlapping with the gene fragment encoding the C-terminus of HPV39 L1 aa 1-469; |
| | [2]: the gene fragment encoding the C-terminal 38 amino acids of HPV59L1 aa 471-508; |
| | [3]: Xbal enzymatic cleavage site. |
| SEQ ID No: 94 | 3913 The amino acid sequence of HPV type 33 L1 protein aa 474-499 |
| | KAKPKLKRAAPTSTRTSSAKRKKVKK |

### Appendix 9 : SEQUENCE LISTING -CHIMERIC HUMAN PAPILLOMAVIRUS TYPE 45 L1 PROTEIN

| | |
|---|---|
| SEQ ID No: 95 | 4501 The amino acid sequence of HPV type 45 L1 protein aa 1-478 |
| | MALWRPSDSTVYLPPPSVARVVNTDDYVSRTSIFYHAGSSRLLTVGNPYFRVVP SGAGNKQAVPKVSAYQYRVFRVALPDPNKFGLPDSTIYNPETQRLVWACVGM EIGRGQPLGIGLSGHPFYNKLDDTESAHAATAVITQDVRDNVSVDYKQTQLCIL GCVPAIGEHWAKGTLCKPAQLQPGDCPPLELKNTIIEDGDMVDTGYGAMDFST LQDTKCEVPLDICQSICKYPDYLQMSADPYGDSMFFCLRREQLFARHFWNRAG VMGDTVPTDLYIKGTSANMRETPGSCVYSPSPSGSITTSDSQLFNKPYWLHKAQ GHNNGICWHNQLFVTVVDTTRSTNLTLCASTQNPVPNTYDPTKFKHYSRHVEE YDLQFIFQLCTITLTAEVMSYIHSMNSSILENWNFGVPPPPTTSLVDTYRFVQSV AVTCQKDTTPPEKQDPYDKLKFWTVDLKEKFSSDLDQYPLGRKFLVQAGL |
| SEQ ID No: 96 | 4502 The amino acid sequence of HPV type 33 L1 protein aa 474-499 |
| | KAKPKLKRAAPTSTRTSSAKRKKVKK |
| SEQ ID No: 97 | 4503 Amino acid sequence of chimeric HPV type 45 L1 protein |
| | MALWRPSDSTVYLPPPSVARVVNTDDYVSRTSIFYHAGSSRLLTVGNPYFRVVP SGAGNKQAVPKVSAYQYRVFRVALPDPNKFGLPDSTIYNPETQRLVWACVGM EIGRGQPLGIGLSGHPFYNKLDDTESAHAATAVITQDVRDNVSVDYKQTQLCIL GCVPAIGEHWAKGTLCKPAQLQPGDCPPLELKNTIIEDGDMVDTGYGAMDFST LQDTKCEVPLDICQSICKYPDYLQMSADPYGDSMFFCLRREQLFARHFWNRAG VMGDTVPTDLYIKGTSANMRETPGSCVYSPSPSGSITTSDSQLFNKPYWLHKAQ GHNNGICWHNQLFVTVVDTTRSTNLTLCASTQNPVPNTYDPTKFKHYSRHVEE YDLQFIFQLCTITLTAEVMSYIHSMNSSILENWNFGVPPPPTTSLVDTYRFVQSV AVTCQKDTTPPEKQDPYDKLKFWTVDLKEKFSSDLDQYPLGRKFLVQAGLKA KPKLKRAAPTSTRTSSAKRKKVKK |
| SEQ ID No: 98 | 4504 Nucleotide sequence of chimeric HPV type 45 L1 protein |
| | ATGGCTCTGTGGAGACCATCTGACAGCACAGTCTACCTGCCTCCTCCATCTGTGGCAAGGGTGGTGAACAC AGATGACTATGTGAGCAGGACCAGCATCTTCTACCATGCTGGCTCCAGCAGACTGCTGACAGTGGGCAACC CATACTTCAGGGTGGTGCCAAGTGGAGCAGGCAACAAGCAGGCTGTGCCAAAGGTGTCTGCCTACCAATAC AGGGTGTTCAGGGTGGCTCTGCCTGACCCAAACAAGTTTGGACTGCCTGACAGCACCATCTACAACCCTGA GACCCAGAGACTGGTGTGGGCTTGTGTGGGGATGGAGATTGGCAGGGGACAACCACTGGGCATTGGACTGT CTGGACACCCATTCTACAACAAACTGGATGACACAGAGTCTGCCCATGCTGCCACAGCAGTGATTACCCAG GATGTGAGGGACAATGTGTCTGTGGACTACAAGCAGACCCAACTTTGTATCCTGGGCTGTGTGCCTGCCATT GGAGAACACTGGGCTAAGGGCACCCTGTGTAAGCCTGCCCAACTCCAACCTGGAGACTGTCCTCCATTGGA ACTGAAAAACACCATCATTGAGGATGGAGATATGGTGGACACAGGCTATGGAGCTATGGACTTCAGCACCC TCCAAGACACCAAGTGTGAGGTGCCACTGGACATCTGTCAGAGCATCTGTAAATACCCTGACTACCTCCAA ATGAGTGCTGACCCATATGGAGACAGTATGTTCTTCTGTCTGAGGAGGGAACAACTTTTTGCCAGACACTTC TGGAACAGGGCTGGAGTGATGGGAGACACAGTGCCAACAGACCTCTACATCAAGGGCACCTCTGCCAATAT GAGGGAGACACCTGGCTCCTGTGTCTACAGCCCAAGCCCATCTGGCAGCATCACCACCTCTGACAGCCAAC TTTTCAACAAGCCATACTGGCTGCACAAGGCTCAAGGACACAACAATGGCATCTGTTGGCACAACCAACTT TTTGTGACAGTGGTGGACACCACCAGGAGCACCAACCTGACCCTGTGTGCCAGCACCCAGAACCCTGTGCC AAACACCTATGACCCAACCAAGTTCAAGCACTACAGCAGGCATGTGGAGGAATATGACCTCCAATTCATCT TCCAACTTTGTACCATCACCCTGACAGCAGAGGTGATGAGTTACATCCACAGTATGAACTCCAGCATCTTGG AGAACTGGAACTTTGGAGTGCCTCCTCCTCCAACCACCTCCCTGGTGGACACCTACAGGTTTGTCCAGTCTG TGGCTGTGACTTGTCAGAAGGACACCACACCTCCTGAGAAGCAGGACCCATATGACAAACTGAAGTTCTGG ACAGTGGACCTGAAAGAGAAGTTCTCCTCTGACCTGGACCAATACCCACTGGGCAGGAAGTTCCTGGTCCA GGCTGGACTGAAAGCCAAGCCAAAACTGAAAAGGGCTGCCCCAACCAGCACCAGGACCTCCTCTGCCAAG AGGAAGAAGGTGAAGAAGTAAA |
| SEQ ID No: 99 | 4505 Synthetic HPV45 L1 gene |
| | ctgggtaccATGGCTCTGTGGAGACCATCTGACAGCACAGTCTACCTGCCTCCTCCATCTGTGGCAAGGGTGGTG AACACAGATGACTATGTGAGCAGGACCAGCATCTTCTACCATGCTGGCTCCAGCAGACTGCTGACAGTGGG CAACCCATACTTCAGGGTGGTGCCAAGTGGAGCAGGCAACAAGCAGGCTGTGCCAAAGGTGTCTGCCTACC AATACAGGGTGTTCAGGGTGGCTCTGCCTGACCCAAACAAGTTTGGACTGCCTGACAGCACCATCTACAAC CCTGAGACCCAGAGACTGGTGTGGGCTTGTGTGGGGATGGAGATTGGCAGGGGACAACCACTGGGCATTG GACTGTCTGGACACCCATTCTACAACAAACTGGATGACACAGAGTCTGCCCATGCTGCCACAGCAGTGATT ACCCAGGATGTGAGGGACAATGTGTCTGTGGACTACAAGCAGACCCAACTTTGTATCCTGGGCTGTGTGCC TGCCATTGGAGAACACTGGGCTAAGGGCACCCTGTGTAAGCCTGCCCAACTCCAACCTGGAGACTGTCCTC CATTGGAACTGAAAAACACCATCATTGAGGATGGAGATATGGTGGACACAGGCTATGGAGCTATGGACTTC AGCACCCTCCAAGACACCAAGTGTGAGGTGCCACTGGACATCTGTCAGAGCATCTGTAAATACCCTGACTA CCTCCAAATGAGTGCTGACCCATATGGAGACAGTATGTTCTTCTGTCTGAGGAGGGAACAACTTTTTGCCAG ACACTTCTGGAACAGGGCTGGAGTGATGGGAGACACAGTGCCAACAGACCTCTACATCAAGGGCACCTCTG CCAATATGAGGGAGACACCTGGCTCCTGTGTCTACAGCCCAAGCCCATCTGGCAGCATCACCACCTCTGAC AGCCAACTTTTCAACAAGCCATACTGGCTGCACAAGGCTCAAGGACACAACAATGGCATCTGTTGGCACAA CCAACTTTTTGTGACAGTGGTGGACACCACCAGGAGCACCAACCTGACCCTGTGTGCCAGCACCCAGAACC CTGTGCCAAACACCTATGACCCAACCAAGTTCAAGCACTACAGCAGGCATGTGGAGGAATATGACCTCCAA TTCATCTTCCAACTTTGTACCATCACCCTGACAGCAGAGGTGATGAGTTACATCCACAGTATGAACTCCAGC ATCTTGGAGAACTGGAACTTTGGAGTGCCTCCTCCTCCAACCACCTCCCTGGTGGACACCTACAGGTTTGTC CAGTCTGTGGCTGTGACTTGTCAGAAGGACACCACACCTCCTGAGAAGCAGGACCCATATGACAAACTGAA GTTCTGGACAGTGGACCTGAAAGAGAAGTTCTCCTCTGACCTGGACCAATACCCACTGGGCAGGAAGTTCC TGGTCCAGGCTGGACTGAGGAGGAGACCAACCATTGGACCAAGGAAGAGACCTGCTGCCAGCACCAGCAC AGCCAGCAGACCTGCCAAGAGGGTGAGGATTAGGAGCAAGAAGTAAA ctcgagctc |
| SEQ ID No: 100 | 4506 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 101 | 4507HPV45 L1 F1 |
| | CTTGGTACCATGGCTCTGTGGAGACCATCTGAC |
| SEQ ID No: 102 | 4508HPV45 L1 R1 |
| | GCTTGGCTTTCAGTCCAGCCTGGACCAGGAAC |
| SEQ ID No: 103 | 4509 HPV45 L1 amplified sequence 1 |
| | CTTGGTACC[1] ATGGCTCTGTGGAGACCATCTGACAGCACAGTCTACCTGCCTCCTCCATCTGTGGCAAGGGTGGTGAACAC AGATGACTATGTGAGCAGGACCAGCATCTTCTACCATGCTGGCTCCAGCAGACTGCTGACAGTGGGCAACC CATACTTCAGGGTGGTGCCAAGTGGAGCAGGCAACAAGCAGGCTGTGCCAAAGGTGTCTGCCTACCAATAC AGGGTGTTCAGGGTGGCTCTGCCTGACCCAAACAAGTTTGGACTGCCTGACAGCACCATCTACAACCCTGA GACCCAGAGACTGGTGTGGGCTTGTGTGGGGATGGAGATTGGCAGGGGACAACCACTGGGCATTGGACTGT CTGGACACCCATTCTACAACAAACTGGATGACACAGAGTCTGCCCATGCTGCCACAGCAGTGATTACCCAG GATGTGAGGGACAATGTGTCTGTGGACTACAAGCAGACCCAACTTTGTATCCTGGGCTGTGTGCCTGCCATT GGAGAACACTGGGCTAAGGGCACCCTGTGTAAGCCTGCCCAACTCCAACCTGGAGACTGTCCTCCATTGGA ACTGAAAAACACCATCATTGAGGATGGAGATATGGTGGACACAGGCTATGGAGCTATGGACTTCAGCACCC TCCAAGACACCAAGTGTGAGGTGCCACTGGACATCTGTCAGAGCATCTGTAAATACCCTGACTACCTCCAA ATGAGTGCTGACCCATATGGAGACAGTATGTTCTTCTGTCTGAGGAGGGAACAACTTTTTGCCAGACACTTC TGGAACAGGGCTGGAGTGATGGGAGACACAGTGCCAACAGACCTCTACATCAAGGGCACCTCTGCCAATAT GAGGGAGACACCTGGCTCCTGTGTCTACAGCCCAAGCCCATCTGGCAGCATCACCACCTCTGACAGCCAAC TTTTCAACAAGCCATACTGGCTGCACAAGGCTCAAGGACACAACAATGGCATCTGTTGGCACAACCAACTT TTTGTGACAGTGGTGGACACCACCAGGAGCACCAACCTGACCCTGTGTGCCAGCACCCAGAACCCTGTGCC AAACACCTATGACCCAACCAAGTTCAAGCACTACAGCAGGCATGTGGAGGAATATGACCTCCAATTCATCT TCCAACTTTGTACCATCACCCTGACAGCAGAGGTGATGAGTTACATCCACAGTATGAACTCCAGCATCTTGG AGAACTGGAACTTTGGAGTGCCTCCTCCTCCAACCACCTCCCTGGTGGACACCTACAGGTTTGTCCAGTCTG TGGCTGTGACTTGTCAGAAGGACACCACACCTCCTGAGAAGCAGGACCCATATGACAAACTGAAGTTCTGG ACAGTGGACCTGAAAGAGAAGTTCTCCTCTGACCTGGACCAATACCCACTGGGCAGGAAGTTCCTGGTCCA GGCTGGACTG[2]AAAGCCAAGC[3] |
| | [1]: Kpn I enzymatic cleavage site; |
| | [2]: the gene fragment encoding of HPV45 L1 aa 1-478; |
| | [3]: 10 bases overlapping with the gene fragment encoding HPV33L1 aa 474-499. |
| SEQ ID No: 104 | 4510HPV45 L1 F2 |
| | GGCTGGACTGAAAGCCAAGCCAAAACTGAAAAGGG |
| SEQ ID No: 105 | 45 11 HPV45 L1 R2 |
| | CTGTCTAGATTTACTTCTTCACCTTCTTCCTCTTGGC |
| SEQ ID No: 106 | 4512 HPV45 L1 amplified sequence 2 |
| | GGCTGGACTG[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV45 L1 aa 1-478; |
| | [2]: the gene fragment encoding the C-terminal 26 amino acids of HPV33L1 aa 474-499; |
| | [3]: Xbal enzymatic cleavage site. |
| SEQ ID No: 107 | 4513 The amino acid sequence of HPV type 59 L1 protein aa 471-508 |
| | LQLGARPKPTIGPRKRAAPAPTSTPSPKRVKRRKSSRK |

### Appendix 10 : SEQUENCE LISTING -CHIMERIC HUMAN PAPILLOMAVIRUS TYPE 51 L1 PROTEIN

| | |
|---|---|
| SEQ ID No: 108 | 5101 The amino acid sequence of HPV type 51 L1 protein aa 1-474 |
| | MALWRTNDSKVYLPPAPVSRIVNTEEYITRTGIYYYAGSSRLITLGHPYFPIPKTS TRAAIPKVSAFQYRVFRVQLPDPNKFGLPDPNLYNPDTDRLVWGCVGVEVGRG QPLGVGLSGHPLFNKYDDTENSRIANGNAQQDVRDNTSVDNKQTQLCIIGCAPP IGEHWGIGTTCKNTPVPPGDCPPLELVSSVIQDGDMIDTGFGAMDFAALQATKS DVPLDISQSVCKYPDYLKMSADTYGNSMFFHLRREQIFARHYYNKLVGVGEDI PNDYYIKGSGNGRDPIESYIYSATPSGSMITSDSQIFNKPYWLHRAQGHNNGICW NNQLFITCVDTTRSTNLTISTATAAVSPTFTPSNFKQYIRHGEEYELQFIFQLCKIT LTTEVMAYLHTMDPTILEQWNFGLTLPPSASLEDAYRFVRNAATSCQKDTPPQ AKPDPLAKYKFWDVDLKERFSLDLDQFALGRKFLLQVGV |
| SEQ ID No: 109 | 5102 The amino acid sequence of HPV type 33 L1 protein aa 474-499 |
| | KAKPKLKRAAPTSTRTSSAKRKKVKK |
| SEQ ID No: 110 | 5103 Amino acid sequence of chimeric HPV type 51 L1 protein |
| | MALWRTNDSKVYLPPAPVSRIVNTEEYITRTGIYYYAGSSRLITLGHPYFPIPKTS TRAAIPKVSAFQYRVFRVQLPDPNKFGLPDPNLYNPDTDRLVWGCVGVEVGRG QPLGVGLSGHPLFNKYDDTENSRIANGNAQQDVRDNTSVDNKQTQLCIIGCAPP IGEHWGIGTTCKNTPVPPGDCPPLELVSSVIQDGDMIDTGFGAMDFAALQATKS DVPLDISQSVCKYPDYLKMSADTYGNSMFFHLRREQIFARHYYNKLVGVGEDI PNDYYIKGSGNGRDPIESYIYSATPSGSMITSDSQIFNKPYWLHRAQGHNNGICW NNQLFITCVDTTRSTNLTISTATAAVSPTFTPSNFKQYIRHGEEYELQFIFQLCKIT LTTEVMAYLHTMDPTILEQWNFGLTLPPSASLEDAYRFVRNAATSCQKDTPPQ AKPDPLAKYKFWDVDLKERFSLDLDQFALGRKFLLQVGVKAKPKLKRAAPTST RTSSAKRKKVKK |
| SEQ ID No: 111 | 5104 Nucleotide sequence of chimeric HPV type 51 L1 protein |
| | ATGGCTCTGTGGAGGACCAATGACAGCAAGGTCTACCTGCCTCCTGCCCCTGTGAGCAGGATTGTGAACAC AGAGGAATACATCACCAGGACAGGCATCTACTACTATGCTGGCTCCAGCAGACTGATTACCCTGGGACACC CATACTTTCCAATCCCAAAGACCAGCACCAGGGCTGCCATCCCAAAGGTGTCTGCCTTCCAATACAGGGTG TTCAGGGTCCAACTTCCTGACCCAAACAAGTTTGGACTGCCTGACCCAAACCTCTACAACCCTGACACAGA CAGACTGGTGTGGGGCTGTGTGGGAGTGGAGGTGGGCAGGGGACAACCACTGGGAGTGGGACTGTCTGGA CACCCACTGTTCAACAAATATGATGACACAGAGAACAGCAGGATTGCCAATGGCAATGCCCAACAGGATGT GAGGGACAACACCTCTGTGGACAACAAGCAGACCCAACTTTGTATCATTGGCTGTGCCCCTCCAATTGGAG AACACTGGGGCATTGGCACCACTTGTAAGAACACACCTGTGCCTCCTGGAGACTGTCCTCCATTGGAACTG GTGTCCTCTGTGATTCAGGATGGAGATATGATTGACACAGGCTTTGGAGCTATGGACTTTGCTGCCCTCCAA GCCACCAAGTCTGATGTGCCACTGGACATCAGCCAGTCTGTGTGTAAATACCCTGACTACCTGAAAATGAG TGCTGACACCTATGGCAACAGTATGTTCTTCCACCTGAGGAGGGAACAGATTTTTGCCAGACACTACTACA ACAAACTGGTGGGAGTGGGAGAGGACATCCCAAATGACTACTACATCAAGGGCTCTGGCAATGGCAGGGA CCCAATTGAGTCCTACATCTACTCTGCCACACCATCTGGCAGTATGATTACCTCTGACAGCCAGATTTTCAA CAAGCCATACTGGCTGCACAGGGCTCAAGGACACAACAATGGCATCTGTTGGAACAACCAACTTTTCATCA CTTGTGTGGACACCACCAGGAGCACCAACCTGACCATCAGCACAGCCACAGCAGCAGTGAGCCCAACCTTC ACACCAAGCAACTTCAAGCAATACATCAGACATGGAGAGGAATATGAACTCCAATTCATCTTCCAACTTTG TAAGATTACCCTGACCACAGAGGTGATGGCTTACCTGCACACAATGGACCCAACCATCTTGGAACAGTGGA ACTTTGGACTGACCCTGCCTCCATCTGCCTCCTTGGAGGATGCCTACAGGTTTGTGAGGAATGCTGCCACCT CCTGTCAGAAGGACACACCTCCACAGGCTAAGCCTGACCCACTGGCTAAATACAAGTTCTGGGATGTGGAC CTGAAAGAGAGGTTCTCCCTGGACCTGGACCAGTTTGCCCTGGGCAGGAAGTTCCTGCTCCAAGTGGGAGT CAAAGCCAAGCCAAAACTGAAAAGGGCTGCCCCAACCAGCACCAGGACCTCCTCTGCCAAGAGGAAGAAG GTGAAGAAGTAAA |
| | |
| SEQ ID No: 112 | 5105 Synthetic HPV51 L1 gene |
| | ctgggtaccATGGCTCTGTGGAGGACCAATGACAGCAAGGTCTACCTGCCTCCTGCCCCTGTGAGCAGGATTGT GAACACAGAGGAATACATCACCAGGACAGGCATCTACTACTATGCTGGCTCCAGCAGACTGATTACCCTGG GACACCCATACTTTCCAATCCCAAAGACCAGCACCAGGGCTGCCATCCCAAAGGTGTCTGCCTTCCAATAC AGGGTGTTCAGGGTCCAACTTCCTGACCCAAACAAGTTTGGACTGCCTGACCCAAACCTCTACAACCCTGA CACAGACAGACTGGTGTGGGGCTGTGTGGGAGTGGAGGTGGGCAGGGGACAACCACTGGGAGTGGGACTG TCTGGACACCCACTGTTCAACAAATATGATGACACAGAGAACAGCAGGATTGCCAATGGCAATGCCCAACA GGATGTGAGGGACAACACCTCTGTGGACAACAAGCAGACCCAACTTTGTATCATTGGCTGTGCCCCTCCAA TTGGAGAACACTGGGGCATTGGCACCACTTGTAAGAACACACCTGTGCCTCCTGGAGACTGTCCTCCATTG GAACTGGTGTCCTCTGTGATTCAGGATGGAGATATGATTGACACAGGCTTTGGAGCTATGGACTTTGCTGCC CTCCAAGCCACCAAGTCTGATGTGCCACTGGACATCAGCCAGTCTGTGTGTAAATACCCTGACTACCTGAA AATGAGTGCTGACACCTATGGCAACAGTATGTTCTTCCACCTGAGGAGGGAACAGATTTTTGCCAGACACT ACTACAACAAACTGGTGGGAGTGGGAGAGGACATCCCAAATGACTACTACATCAAGGGCTCTGGCAATGG CAGGGACCCAATTGAGTCCTACATCTACTCTGCCACACCATCTGGCAGTATGATTACCTCTGACAGCCAGAT TTTCAACAAGCCATACTGGCTGCACAGGGCTCAAGGACACAACAATGGCATCTGTTGGAACAACCAACTTT TCATCACTTGTGTGGACACCACCAGGAGCACCAACCTGACCATCAGCACAGCCACAGCAGCAGTGAGCCCA ACCTTCACACCAAGCAACTTCAAGCAATACATCAGACATGGAGAGGAATATGAACTCCAATTCATCTTCCA ACTTTGTAAGATTACCCTGACCACAGAGGTGATGGCTTACCTGCACACAATGGACCCAACCATCTTGGAAC AGTGGAACTTTGGACTGACCCTGCCTCCATCTGCCTCCTTGGAGGATGCCTACAGGTTTGTGAGGAATGCTG CCACCTCCTGTCAGAAGGACACACCTCCACAGGCTAAGCCTGACCCACTGGCTAAATACAAGTTCTGGGAT GTGGACCTGAAAGAGAGGTTCTCCCTGGACCTGGACCAGTTTGCCCTGGGCAGGAAGTTCCTGCTCCAAGT GGGAGTCCAGAGGAAGCCAAGACCTGGACTGAAAAGACCTGCCTCCTCTGCCTCCTCCTCCTCCTCCTCCTC TGCCAAGAGGAAGAGGGTGAAGAAGTAAActcgagctc |
| SEQ ID No: 113 | 5106 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 114 | 5107HPV51 L1 F1 |
| | CTTGGTACCATGGCTCTGTGGAGGACCAATGACA |
| SEQ ID No: 115 | 5108HPV51 L1 R1 |
| | GCTTGGCTTTGACTCCCACTTGGAGCAGGAAC |
| SEQ ID No: 116 | 5109 HPV51 L1 amplified sequence 1 |
| | CTTGGTACC[1] ATGGCTCTGTGGAGGACCAATGACAGCAAGGTCTACCTGCCTCCTGCCCCTGTGAGCAGGATTGTGAACAC AGAGGAATACATCACCAGGACAGGCATCTACTACTATGCTGGCTCCAGCAGACTGATTACCCTGGGACACC CATACTTTCCAATCCCAAAGACCAGCACCAGGGCTGCCATCCCAAAGGTGTCTGCCTTCCAATACAGGGTG TTCAGGGTCCAACTTCCTGACCCAAACAAGTTTGGACTGCCTGACCCAAACCTCTACAACCCTGACACAGA CAGACTGGTGTGGGGCTGTGTGGGAGTGGAGGTGGGCAGGGGACAACCACTGGGAGTGGGACTGTCTGGA CACCCACTGTTCAACAAATATGATGACACAGAGAACAGCAGGATTGCCAATGGCAATGCCCAACAGGATGT GAGGGACAACACCTCTGTGGACAACAAGCAGACCCAACTTTGTATCATTGGCTGTGCCCCTCCAATTGGAG AACACTGGGGCATTGGCACCACTTGTAAGAACACACCTGTGCCTCCTGGAGACTGTCCTCCATTGGAACTG GTGTCCTCTGTGATTCAGGATGGAGATATGATTGACACAGGCTTTGGAGCTATGGACTTTGCTGCCCTCCAA GCCACCAAGTCTGATGTGCCACTGGACATCAGCCAGTCTGTGTGTAAATACCCTGACTACCTGAAAATGAG TGCTGACACCTATGGCAACAGTATGTTCTTCCACCTGAGGAGGGAACAGATTTTTGCCAGACACTACTACA ACAAACTGGTGGGAGTGGGAGAGGACATCCCAAATGACTACTACATCAAGGGCTCTGGCAATGGCAGGGA CCCAATTGAGTCCTACATCTACTCTGCCACACCATCTGGCAGTATGATTACCTCTGACAGCCAGATTTTCAA CAAGCCATACTGGCTGCACAGGGCTCAAGGACACAACAATGGCATCTGTTGGAACAACCAACTTTTCATCA CTTGTGTGGACACCACCAGGAGCACCAACCTGACCATCAGCACAGCCACAGCAGCAGTGAGCCCAACCTTC ACACCAAGCAACTTCAAGCAATACATCAGACATGGAGAGGAATATGAACTCCAATTCATCTTCCAACTTTG TAAGATTACCCTGACCACAGAGGTGATGGCTTACCTGCACACAATGGACCCAACCATCTTGGAACAGTGGA ACTTTGGACTGACCCTGCCTCCATCTGCCTCCTTGGAGGATGCCTACAGGTTTGTGAGGAATGCTGCCACCT CCTGTCAGAAGGACACACCTCCACAGGCTAAGCCTGACCCACTGGCTAAATACAAGTTCTGGGATGTGGAC CTGAAAGAGAGGTTCTCCCTGGACCTGGACCAGTTTGCCCTGGGCAGGAAGTTCCTGCTCCAAGTGGGAGT C [2]AAAGCCAAGC[3] |
| | [1]: Kpn I enzymatic cleavage site; |
| | [2]: the gene fragment encoding of HPV51 L1 aa 1-474; |
| | [3]: 10 bases overlapping with the gene fragment encoding HPV33L1 aa 474-499 |
| SEQ ID No: 117 | 5110HPV51 L1 F2 |
| | AGTGGGAGTCAAAGCCAAGCCAAAACTGAAAAGGG |
| SEQ ID No: 118 | 5111HPV51 L1 R2 |
| | CTGTCTAGATTTACTTCTTCACCTTCTTCCTCTTGG |
| SEQ ID No: 119 | 5112 HPV51 L1 amplified sequence 2 |
| | AGTGGGAGTC[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV51 L1 aa 1-474; |
| | [2]: the gene fragment encoding the C-terminal 26 amino acids of HPV33L1 aa 474-499; |
| | [3]: Xbal enzymatic cleavage site. |
| SEQ ID No: 120 | 5113 The amino acid sequence of HPV type 59 L1 protein aa 471-508 |
| | LQLGARPKPTIGPRKRAAPAPTSTPSPKRVKRRKSSRK |

### Appendix 11 : SEQUENCE LISTING -CHIMERIC HUMAN PAPILLOMAVIRUS TYPE 52 L1 PROTEIN

| | |
|---|---|
| SEQ ID No: 121 | 5201 The amino acid sequence of HPV type 52 L1 protein aa 1-478 |
| | MSVWRPSEATVYLPPVPVSKVVSTDEYVSRTSIYYYAGSSRLLTVGHPYFSIKN TSSGNGKKVLVPKVSGLQYRVFRIKLPDPNKFGFPDTSFYNPETQRLVWACTGL EIGRGQPLGVGISGHPLLNKFDDTETSNKYAGKPGIDNRECLSMDYKQTQLCIL GCKPPIGEHWGKGTPCNNNSGNPGDCPPLQLINSVIQDGDMVDTGFGCMDFNT LQASKSDVPIDICSSVCKYPDYLQMASEPYGDSLFFFLRREQMFVRHFFNRAGT LGDPVPGDLYIQGSNSGNTATVQSSAFFPTPSGSMVTSESQLFNKPYWLQRAQG HNNGICWGNQLFVTVVDTTRSTNMTLCAEVKKESTYKNENFKEYLRHGEEFDL QFIFQLCKITLTADVMTYIHKMDATILEDWQFGLTPPPSASLEDTYRFVTSTAIT CQKNTPPKGKEDPLKDYMFWEVDLKEKFSADLDQFPLGRKFLLQAGL |
| SEQ ID No: 122 | 5202 The amino acid sequence of HPV type 33 L1 protein aa 474-499 |
| | KAKPKLKRAAPTSTRTSSAKRKKVKK |
| SEQ ID No: 123 | 5203 Amino acid sequence of chimeric HPV type 52 L1 protein |
| | MSVWRPSEATVYLPPVPVSKVVSTDEYVSRTSIYYYAGSSRLLTVGHPYFSIKN TSSGNGKKVLVPKVSGLQYRVFRIKLPDPNKFGFPDTSFYNPETQRLVWACTGL EIGRGQPLGVGISGHPLLNKFDDTETSNKYAGKPGIDNRECLSMDYKQTQLCIL GCKPPIGEHWGKGTPCNNNSGNPGDCPPLQLINSVIQDGDMVDTGFGCMDFNT LQASKSDVPIDICSSVCKYPDYLQMASEPYGDSLFFFLRREQMFVRHFFNRAGT LGDPVPGDLYIQGSNSGNTATVQSSAFFPTPSGSMVTSESQLFNKPYWLQRAQG HNNGICWGNQLFVTVVDTTRSTNMTLCAEVKKESTYKNENFKEYLRHGEEFDL QFIFQLCKITLTADVMTYIHKMDATILEDWQFGLTPPPSASLEDTYRFVTSTAIT CQKNTPPKGKEDPLKDYMFWEVDLKEKFSADLDQFPLGRKFLLQAGLKAKPK LKRAAPTSTRTSSAKRKKVKK |
| SEQ ID No: 124 | 5204 Nucleotide sequence of chimeric HPV type 52 L1 protein |
| | ATGAGCGTGTGGAGGCCCAGCGAGGCCACCGTGTACCTGCCCCCCGTGCCCGTGAGCAAGGTGGTGAGCAC CGACGAGTACGTGAGCAGGACCAGCATCTACTACTACGCCGGCAGCAGCAGGCTGCTGACCGTGGGCCACC CCTACTTCAGCATCAAGAACACCAGCAGCGGCAACGGCAAGAAGGTGCTGGTGCCCAAGGTGAGCGGCCT GCAGTACAGGGTGTTCAGGATCAAGCTGCCCGACCCCAACAAGTTCGGCTTCCCCGACACCAGCTTCTACA ACCCCGAGACCCAGAGGCTGGTGTGGGCCTGCACCGGCCTGGAGATCGGCAGGGGCCAGCCCCTGGGCGT GGGCATCAGCGGCCACCCCCTGCTGAACAAGTTCGACGACACCGAGACCAGCAACAAGTACGCCGGCAAG CCCGGCATCGACAACAGGGAGTGCCTGAGCATGGACTACAAGCAGACCCAGCTGTGCATCCTGGGCTGCAA GCCCCCCATCGGCGAGCACTGGGGCAAGGGCACCCCCTGCAACAACAACAGCGGCAACCCCGGCGACTGC CCCCCCCTGCAGCTGATCAACAGCGTGATCCAGGACGGCGACATGGTGGACACCGGCTTCGGCTGCATGGA CTTCAACACCCTGCAGGCCAGCAAGAGCGACGTGCCCATCGACATCTGCAGCAGCGTGTGCAAGTACCCCG ACTACCTGCAGATGGCCAGCGAGCCCTACGGCGACAGCCTGTTCTTCTTCCTGAGGAGGGAGCAGATGTTC GTGAGGCACTTCTTCAACAGGGCCGGCACCCTGGGCGACCCCGTGCCCGGCGACCTGTACATCCAGGGCAG CAACAGCGGCAACACCGCCACCGTGCAGAGCAGCGCCTTCTTCCCCACCCCCAGCGGCAGCATGGTGACCA GCGAGAGCCAGCTGTTCAACAAGCCCTACTGGCTGCAGAGGGCCCAGGGCCACAACAACGGCATCTGCTG GGGCAACCAGCTGTTCGTGACCGTGGTGGACACCACCAGGAGCACCAACATGACCCTGTGCGCCGAGGTGA AGAAGGAGAGCACCTACAAGAACGAGAACTTCAAGGAGTACCTGAGGCACGGCGAGGAGTTCGACCTGCA GTTCATCTTCCAGCTGTGCAAGATCACCCTGACCGCCGACGTGATGACCTACATCCACAAGATGGACGCCA CCATCCTGGAGGACTGGCAGTTCGGCCTGACCCCCCCCCCCAGCGCCAGCCTGGAGGACACCTACAGGTTC GTGACCAGCACCGCCATCACCTGCCAGAAGAACACCCCCCCCAAGGGCAAGGAGGACCCCCTGAAGGACT ACATGTTCTGGGAGGTGGACCTGAAGGAGAAGTTCAGCGCCGACCTGGACCAGTTCCCCCTGGGCAGGAAG TTCCTGCTGCAGGCCGGCCTGAAAGCCAAGCCAAAACTGAAAAGGGCTGCCCCAACCAGCACCAGGACCTC CTCTGCCAAGAGGAAGAAGGTGAAGAAGTAAA |
| | |
| SEQ ID No: 125 | 5205 Synthetic HPV52 L1 gene |
| | ctgggtaccATGAGCGTGTGGAGGCCCAGCGAGGCCACCGTGTACCTGCCCCCCGTGCCCGTGAGCAAGGTGGT GAGCACCGACGAGTACGTGAGCAGGACCAGCATCTACTACTACGCCGGCAGCAGCAGGCTGCTGACCGTG GGCCACCCCTACTTCAGCATCAAGAACACCAGCAGCGGCAACGGCAAGAAGGTGCTGGTGCCCAAGGTGA GCGGCCTGCAGTACAGGGTGTTCAGGATCAAGCTGCCCGACCCCAACAAGTTCGGCTTCCCCGACACCAGC TTCTACAACCCCGAGACCCAGAGGCTGGTGTGGGCCTGCACCGGCCTGGAGATCGGCAGGGGCCAGCCCCT GGGCGTGGGCATCAGCGGCCACCCCCTGCTGAACAAGTTCGACGACACCGAGACCAGCAACAAGTACGCC GGCAAGCCCGGCATCGACAACAGGGAGTGCCTGAGCATGGACTACAAGCAGACCCAGCTGTGCATCCTGG GCTGCAAGCCCCCCATCGGCGAGCACTGGGGCAAGGGCACCCCCTGCAACAACAACAGCGGCAACCCCGG CGACTGCCCCCCCCTGCAGCTGATCAACAGCGTGATCCAGGACGGCGACATGGTGGACACCGGCTTCGGCT GCATGGACTTCAACACCCTGCAGGCCAGCAAGAGCGACGTGCCCATCGACATCTGCAGCAGCGTGTGCAAG TACCCCGACTACCTGCAGATGGCCAGCGAGCCCTACGGCGACAGCCTGTTCTTCTTCCTGAGGAGGGAGCA GATGTTCGTGAGGCACTTCTTCAACAGGGCCGGCACCCTGGGCGACCCCGTGCCCGGCGACCTGTACATCC AGGGCAGCAACAGCGGCAACACCGCCACCGTGCAGAGCAGCGCCTTCTTCCCCACCCCCAGCGGCAGCATG GTGACCAGCGAGAGCCAGCTGTTCAACAAGCCCTACTGGCTGCAGAGGGCCCAGGGCCACAACAACGGCA TCTGCTGGGGCAACCAGCTGTTCGTGACCGTGGTGGACACCACCAGGAGCACCAACATGACCCTGTGCGCC GAGGTGAAGAAGGAGAGCACCTACAAGAACGAGAACTTCAAGGAGTACCTGAGGCACGGCGAGGAGTTCG ACCTGCAGTTCATCTTCCAGCTGTGCAAGATCACCCTGACCGCCGACGTGATGACCTACATCCACAAGATG GACGCCACCATCCTGGAGGACTGGCAGTTCGGCCTGACCCCCCCCCCCAGCGCCAGCCTGGAGGACACCTA CAGGTTCGTGACCAGCACCGCCATCACCTGCCAGAAGAACACCCCCCCCAAGGGCAAGGAGGACCCCCTG AAGGACTACATGTTCTGGGAGGTGGACCTGAAGGAGAAGTTCAGCGCCGACCTGGACCAGTTCCCCCTGGG CAGGAAGTTCCTGCTGCAGGCCGGCCTGCAGGCCAGGCCCAAGCTGAAGAGGCCCGCCAGCAGCGCCCCC AGGACCAGCACCAAGAAGAAGAAGGTGAAGAGGTAAActcgagctc |
| SEQ ID No: 126 | 5206 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 127 | 5207HPV52 L1 F1 |
| | CTTGGTACCATGAGCGTGTGGAGGCCCAGCGAGG |
| SEQ ID No: 128 | 5208HPV52 L1 R1 |
| | GCTTGGCTTTCAGGCCGGCCTGCAGCAGGAACTTC |
| SEQ ID No: 129 | 5209 HPV52 L1 amplified sequence 1 |
| | CTTGGTACC[1] ATGAGCGTGTGGAGGCCCAGCGAGGCCACCGTGTACCTGCCCCCCGTGCCCGTGAGCAAGGTGGTGAGCAC CGACGAGTACGTGAGCAGGACCAGCATCTACTACTACGCCGGCAGCAGCAGGCTGCTGACCGTGGGCCACC CCTACTTCAGCATCAAGAACACCAGCAGCGGCAACGGCAAGAAGGTGCTGGTGCCCAAGGTGAGCGGCCT GCAGTACAGGGTGTTCAGGATCAAGCTGCCCGACCCCAACAAGTTCGGCTTCCCCGACACCAGCTTCTACA ACCCCGAGACCCAGAGGCTGGTGTGGGCCTGCACCGGCCTGGAGATCGGCAGGGGCCAGCCCCTGGGCGT GGGCATCAGCGGCCACCCCCTGCTGAACAAGTTCGACGACACCGAGACCAGCAACAAGTACGCCGGCAAG CCCGGCATCGACAACAGGGAGTGCCTGAGCATGGACTACAAGCAGACCCAGCTGTGCATCCTGGGCTGCAA GCCCCCCATCGGCGAGCACTGGGGCAAGGGCACCCCCTGCAACAACAACAGCGGCAACCCCGGCGACTGC CCCCCCCTGCAGCTGATCAACAGCGTGATCCAGGACGGCGACATGGTGGACACCGGCTTCGGCTGCATGGA CTTCAACACCCTGCAGGCCAGCAAGAGCGACGTGCCCATCGACATCTGCAGCAGCGTGTGCAAGTACCCCG ACTACCTGCAGATGGCCAGCGAGCCCTACGGCGACAGCCTGTTCTTCTTCCTGAGGAGGGAGCAGATGTTC GTGAGGCACTTCTTCAACAGGGCCGGCACCCTGGGCGACCCCGTGCCCGGCGACCTGTACATCCAGGGCAG CAACAGCGGCAACACCGCCACCGTGCAGAGCAGCGCCTTCTTCCCCACCCCCAGCGGCAGCATGGTGACCA GCGAGAGCCAGCTGTTCAACAAGCCCTACTGGCTGCAGAGGGCCCAGGGCCACAACAACGGCATCTGCTG GGGCAACCAGCTGTTCGTGACCGTGGTGGACACCACCAGGAGCACCAACATGACCCTGTGCGCCGAGGTGA AGAAGGAGAGCACCTACAAGAACGAGAACTTCAAGGAGTACCTGAGGCACGGCGAGGAGTTCGACCTGCA GTTCATCTTCCAGCTGTGCAAGATCACCCTGACCGCCGACGTGATGACCTACATCCACAAGATGGACGCCA CCATCCTGGAGGACTGGCAGTTCGGCCTGACCCCCCCCCCCAGCGCCAGCCTGGAGGACACCTACAGGTTC GTGACCAGCACCGCCATCACCTGCCAGAAGAACACCCCCCCCAAGGGCAAGGAGGACCCCCTGAAGGACT ACATGTTCTGGGAGGTGGACCTGAAGGAGAAGTTCAGCGCCGACCTGGACCAGTTCCCCCTGGGCAGGAAG TTCCTGCTGCAGGCCGGCCTG [2]AAAGCCAAGC[3] |
| | [1]: Kpn I enzymatic cleavage site; |
| | [2]: the gene fragment encoding of HPV52 L1 aa 1-478; |
| | [3]: 10 bases overlapping with the gene fragment encoding HPV33L1 aa 474-499. |
| SEQ ID No: 130 | 5210HPV52 L1 F2 |
| | GGCCGGCCTGAAAGCCAAGCCAAAACTGAAAAGGG |
| SEQ ID No: 131 | 5211HPV52 L1 R2 |
| | CTGTCTAGATTTACTTCTTCACCTTCTTCCTCTTGG |
| SEQ ID No: 132 | 5212 HPV52 L1 amplified sequence 2 |
| | GGCCGGCCTG[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV52 L1 aa 1-478; |
| | [2]: the gene fragment encoding the C-terminal 26 amino acids of HPV33L1 aa 474-499; |
| | [3]: Xbal enzymatic cleavage site |
| SEQ ID No: 133 | 5213 The amino acid sequence of HPV type 59 L1 protein aa 471-508 |
| | LQLGARPKPTIGPRKRAAPAPTSTPSPKRVKRRKSSRK |

### Appendix 12 : SEQUENCE LISTING -CHIMERIC HUMAN PAPILLOMAVIRUS TYPE 56 L1 PROTEIN

| | |
|---|---|
| SEQ ID No: 134 | 5601 The amino acid sequence of HPV type 56 L1 protein aa 1-467 |
| | MATWRPSENKVYLPPTPVSKVVATDSYVKRTSIFYHAGSSRLLAVGHPYYSVT KDNTKTNIPKVSAYQYRVFRVRLPDPNKFGLPDTNIYNPDQERLVWACVGLEV GRGQPLGAGLSGHPLFNRLDDTESSNLANNNVIEDSRDNISVDGKQTQLCIVGC TPAMGEHWTKGAVCKSTQVTTGDCPPLALINTPIEDGDMIDTGFGAMDFKVLQ ESKAEVPLDIVQSTCKYPDYLKMSADAYGDSMWFYLRREQLFARHYFNRAGK VGETIPAELYLKGSNGREPPPSSVYVATPSGSMITSEAQLFNKPYWLQRAQGHN NGICWGNQLFVTVVDTTRSTNMTISTATEQLSKYDARKINQYLRHVEEYELQF VFQLCKITLSAEVMAYLHNMNANLLEDWNIGLSPPVATSLEDKYRYVRSTAITC QREQPPTEKQDPLAKYKFWDVNLQDSFSTDLDQFPLGRKFL |
| SEQ ID No: 135 | 5602 The amino acid sequence of HPV type 33 L1 protein aa 469-499 |
| | LQAGLKAKPKLKRAAPTSTRTSSAKRKKVKK |
| SEQ ID No: 136 | 5603 Amino acid sequence of chimeric HPV type 56 L1 protein |
| | MATWRPSENKVYLPPTPVSKVVATDSYVKRTSIFYHAGSSRLLAVGHPYYSVT KDNTKTNIPKVSAYQYRVFRVRLPDPNKFGLPDTNIYNPDQERLVWACVGLEV GRGQPLGAGLSGHPLFNRLDDTESSNLANNNVIEDSRDNISVDGKQTQLCIVGC TPAMGEHWTKGAVCKSTQVTTGDCPPLALINTPIEDGDMIDTGFGAMDFKVLQ ESKAEVPLDIVQSTCKYPDYLKMSADAYGDSMWFYLRREQLFARHYFNRAGK VGETIPAELYLKGSNGREPPPSSVYVATPSGSMITSEAQLFNKPYWLQRAQGHN NGICWGNQLFVTVVDTTRSTNMTISTATEQLSKYDARKINQYLRHVEEYELQF VFQLCKITLSAEVMAYLHNMNANLLEDWNIGLSPPVATSLEDKYRYVRSTAITC QREQPPTEKQDPLAKYKFWDVNLQDSFSTDLDQFPLGRKFLLQAGLKAKPKLK RAAPTSTRTSSAKRKKVKK |
| SEQ ID No: 137 | 5604 Nucleotide sequence of chimeric HPV type 56 L1 protein |
| | ATGGCTACCTGGAGACCATCTGAGAACAAGGTCTACCTGCCTCCAACACCTGTGAGCAAGGTGGTGGCTAC AGACTCCTATGTGAAGAGGACCAGCATCTTCTACCATGCTGGCTCCAGCAGACTGCTGGCTGTGGGACACC CATACTACTCTGTGACCAAGGACAACACCAAGACCAACATCCCAAAGGTGTCTGCCTACCAATACAGGGTG TTCAGGGTGAGACTGCCTGACCCAAACAAGTTTGGACTGCCTGACACCAACATCTACAACCCTGACCAGGA GAGACTGGTGTGGGCTTGTGTGGGATTGGAGGTGGGCAGGGGACAACCACTGGGAGCAGGACTGTCTGGA CACCCACTGTTCAACAGACTGGATGACACAGAGTCCAGCAACCTGGCTAACAACAATGTGATTGAGGACAG CAGGGACAACATCTCTGTGGATGGCAAGCAGACCCAACTTTGTATTGTGGGCTGTACTCCTGCTATGGGAG AACACTGGACCAAGGGAGCAGTGTGTAAGAGCACCCAGGTGACCACAGGAGACTGTCCTCCACTGGCTCTG ATAAACACACCAATTGAGGATGGAGATATGATTGACACAGGCTTTGGAGCTATGGACTTCAAGGTGCTCCA AGAGAGCAAGGCTGAGGTGCCACTGGACATTGTCCAGAGCACTTGTAAATACCCTGACTACCTGAAAATGA GTGCTGATGCCTATGGAGACAGTATGTGGTTCTACCTGAGGAGGGAACAACTTTTTGCCAGACACTACTTC AACAGGGCTGGCAAGGTGGGAGAGACCATCCCTGCTGAACTCTACCTGAAAGGCAGCAATGGCAGGGAAC CTCCTCCATCCTCTGTCTATGTGGCTACACCATCTGGCAGTATGATTACCTCTGAGGCTCAACTTTTCAACAA GCCATACTGGCTCCAAAGGGCTCAAGGACACAACAATGGCATCTGTTGGGGCAACCAACTTTTTGTGACAG TGGTGGACACCACCAGGAGCACCAATATGACCATCAGCACAGCCACAGAACAACTTAGCAAATATGATGC CAGGAAGATAAACCAATACCTGAGGCATGTGGAGGAATATGAACTCCAATTTGTGTTCCAACTTTGTAAGA TTACCCTGTCTGCTGAGGTGATGGCTTACCTGCACAATATGAATGCCAACCTGTTGGAGGACTGGAACATTG GACTGAGCCCTCCTGTGGCTACCTCCTTGGAGGACAAATACAGATATGTGAGGAGCACAGCCATCACTTGT CAGAGGGAACAACCTCCAACAGAGAAGCAGGACCCACTGGCTAAATACAAGTTCTGGGATGTGAACCTCC AAGACTCCTTCAGCACAGACCTGGACCAGTTTCCACTGGGCAGGAAGTTCCTGCTCCAAGCAGGACTGAAA GCCAAGCCAAAACTGAAAAGGGCTGCCCCAACCAGCACCAGGACCTCCTCTGCCAAGAGGAAGAAGGTGA AGAAGTAAA |
| SEQ ID No: 138 | 5605 Synthetic HPV56 L1 gene |
| | ctgggtaccATGGCTACCTGGAGACCATCTGAGAACAAGGTCTACCTGCCTCCAACACCTGTGAGCAAGGTGGT GGCTACAGACTCCTATGTGAAGAGGACCAGCATCTTCTACCATGCTGGCTCCAGCAGACTGCTGGCTGTGG GACACCCATACTACTCTGTGACCAAGGACAACACCAAGACCAACATCCCAAAGGTGTCTGCCTACCAATAC AGGGTGTTCAGGGTGAGACTGCCTGACCCAAACAAGTTTGGACTGCCTGACACCAACATCTACAACCCTGA CCAGGAGAGACTGGTGTGGGCTTGTGTGGGATTGGAGGTGGGCAGGGGACAACCACTGGGAGCAGGACTG TCTGGACACCCACTGTTCAACAGACTGGATGACACAGAGTCCAGCAACCTGGCTAACAACAATGTGATTGA GGACAGCAGGGACAACATCTCTGTGGATGGCAAGCAGACCCAACTTTGTATTGTGGGCTGTACTCCTGCTA TGGGAGAACACTGGACCAAGGGAGCAGTGTGTAAGAGCACCCAGGTGACCACAGGAGACTGTCCTCCACT GGCTCTGATAAACACACCAATTGAGGATGGAGATATGATTGACACAGGCTTTGGAGCTATGGACTTCAAGG TGCTCCAAGAGAGCAAGGCTGAGGTGCCACTGGACATTGTCCAGAGCACTTGTAAATACCCTGACTACCTG AAAATGAGTGCTGATGCCTATGGAGACAGTATGTGGTTCTACCTGAGGAGGGAACAACTTTTTGCCAGACA CTACTTCAACAGGGCTGGCAAGGTGGGAGAGACCATCCCTGCTGAACTCTACCTGAAAGGCAGCAATGGCA GGGAACCTCCTCCATCCTCTGTCTATGTGGCTACACCATCTGGCAGTATGATTACCTCTGAGGCTCAACTTT TCAACAAGCCATACTGGCTCCAAAGGGCTCAAGGACACAACAATGGCATCTGTTGGGGCAACCAACTTTTT GTGACAGTGGTGGACACCACCAGGAGCACCAATATGACCATCAGCACAGCCACAGAACAACTTAGCAAAT ATGATGCCAGGAAGATAAACCAATACCTGAGGCATGTGGAGGAATATGAACTCCAATTTGTGTTCCAACTT TGTAAGATTACCCTGTCTGCTGAGGTGATGGCTTACCTGCACAATATGAATGCCAACCTGTTGGAGGACTG GAACATTGGACTGAGCCCTCCTGTGGCTACCTCCTTGGAGGACAAATACAGATATGTGAGGAGCACAGCCA TCACTTGTCAGAGGGAACAACCTCCAACAGAGAAGCAGGACCCACTGGCTAAATACAAGTTCTGGGATGTG AACCTCCAAGACTCCTTCAGCACAGACCTGGACCAGTTTCCACTGGGCAGGAAGTTCCTGATGCAACTTGG CACCAGGAGCAAGCCTGCTGTGGCTACCAGCAAGAAGAGGTCTGCCCCAACCAGCACCAGCACACCTGCC AAGAGGAAGAGGAGGTAAActcgagctc |
| SEQ ID No: 139 | 5606 Synthetic HPV33 L1 gene |
| | ctgggtaccATGAGTGTGTGGAGACCATCTGAGGCTACAGTCTACCTGCCTCCTGTGCCTGTGAGCAAGGTGGTG |
| | AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 140 | 5607HPV56 L1 F1 |
| | CTTGGTACCATGGCTACCTGGAGACCATCTGAG |
| SEQ ID No: 141 | 5608HPV56 L1 R1 |
| | CTGCTTGGAGCAGGAACTTCCTGCCCAGTGG |
| | |
| SEQ ID No: 142 | 5609 HPV56 L1 amplified sequence 1 |
| | CTTGGTACC[1] ATGGCTACCTGGAGACCATCTGAGAACAAGGTCTACCTGCCTCCAACACCTGTGAGCAAGGTGGTGGCTAC AGACTCCTATGTGAAGAGGACCAGCATCTTCTACCATGCTGGCTCCAGCAGACTGCTGGCTGTGGGACACC CATACTACTCTGTGACCAAGGACAACACCAAGACCAACATCCCAAAGGTGTCTGCCTACCAATACAGGGTG TTCAGGGTGAGACTGCCTGACCCAAACAAGTTTGGACTGCCTGACACCAACATCTACAACCCTGACCAGGA GAGACTGGTGTGGGCTTGTGTGGGATTGGAGGTGGGCAGGGGACAACCACTGGGAGCAGGACTGTCTGGA CACCCACTGTTCAACAGACTGGATGACACAGAGTCCAGCAACCTGGCTAACAACAATGTGATTGAGGACAG CAGGGACAACATCTCTGTGGATGGCAAGCAGACCCAACTTTGTATTGTGGGCTGTACTCCTGCTATGGGAG AACACTGGACCAAGGGAGCAGTGTGTAAGAGCACCCAGGTGACCACAGGAGACTGTCCTCCACTGGCTCTG ATAAACACACCAATTGAGGATGGAGATATGATTGACACAGGCTTTGGAGCTATGGACTTCAAGGTGCTCCA AGAGAGCAAGGCTGAGGTGCCACTGGACATTGTCCAGAGCACTTGTAAATACCCTGACTACCTGAAAATGA GTGCTGATGCCTATGGAGACAGTATGTGGTTCTACCTGAGGAGGGAACAACTTTTTGCCAGACACTACTTC AACAGGGCTGGCAAGGTGGGAGAGACCATCCCTGCTGAACTCTACCTGAAAGGCAGCAATGGCAGGGAAC CTCCTCCATCCTCTGTCTATGTGGCTACACCATCTGGCAGTATGATTACCTCTGAGGCTCAACTTTTCAACAA GCCATACTGGCTCCAAAGGGCTCAAGGACACAACAATGGCATCTGTTGGGGCAACCAACTTTTTGTGACAG TGGTGGACACCACCAGGAGCACCAATATGACCATCAGCACAGCCACAGAACAACTTAGCAAATATGATGC CAGGAAGATAAACCAATACCTGAGGCATGTGGAGGAATATGAACTCCAATTTGTGTTCCAACTTTGTAAGA TTACCCTGTCTGCTGAGGTGATGGCTTACCTGCACAATATGAATGCCAACCTGTTGGAGGACTGGAACATTG GACTGAGCCCTCCTGTGGCTACCTCCTTGGAGGACAAATACAGATATGTGAGGAGCACAGCCATCACTTGT CAGAGGGAACAACCTCCAACAGAGAAGCAGGACCCACTGGCTAAATACAAGTTCTGGGATGTGAACCTCC AAGACTCCTTCAGCACAGACCTGGACCAGTTTCCACTGGGCAGGAAGTTCCTG[2]CTCCAAGCAG[3] |
| | [1]: Kpn I enzymatic cleavage site; |
| | [2]: the gene fragment encoding of HPV56 L1 aa 1-467; |
| | [3]: 10 bases overlapping with the gene fragment encoding HPV33L1 aa 469-499. |
| SEQ ID No: 143 | 5610HPV56 L1 F2 |
| | GAAGTTCCTGCTCCAAGCAGGACTGAAAGCCAAGCC |
| SEQ ID No: 144 | 5611HPV56 L1 R2 |
| | CTGTCTAGATTTACTTCTTCACCTTCTTCCTCTTGG |
| SEQ ID No: 145 | 5612 HPV56 L1 amplified sequence 2 |
| | GAAGTTCCTG[1] CTCCAAGCAGGACTGAAAGCCAAGCCAAAACTGAAAAGGGCTGCCCCAACCAGCACCAGGACCTCCTCTG CCAAGAGGAAGAAGGTGAAGAAG[2] TAA ATCTAGA[3]CAG. |
| | [1]: 10bp overlapping with the gene fragment encoding the C-terminus of HPV56 L1 aa 1-467; |
| | [2]: the gene fragment encoding the C-terminal 31 amino acids of HPV33L1 aa 469-499; |
| | [3]: Xbal enzymatic cleavage site. |
| SEQ ID No: 146 | 5613 The amino acid sequence of HPV type 59 L1 protein aa 471-508 |
| | LQLGARPKPTIGPRKRAAPAPTSTPSPKRVKRRKSSRK |

### Appendix 13 : SEQUENCE LISTING -CHIMERIC HUMAN PAPILLOMAVIRUS TYPE 58 L1 PROTEIN

| | |
|---|---|
| SEQ ID No: 147 | 5801 The amino acid sequence of HPV type 58 L1 protein aa 1-473 |
| | MSVWRPSEATVYLPPVPVSKVVSTDEYVSRTSIYYYAGSSRLLAVGNPYFSIKS PNNNKKVLVPKVSGLQYRVFRVRLPDPNKFGFPDTSFYNPDTQRLVWACVGLE IGRGQPLGVGVSGHPYLNKFDDTETSNRYPAQPGSDNRECLSMDYKQTQLCLI GCKPPTGEHWGKGVACNNNAAATDCPPLELFNSIIEDGDMVDTGFGCMDFGTL QANKSDVPIDICNSTCKYPDYLKMASEPYGDSLFFFLRREQMFVRHFFNRAGKL GEAVPDDLYIKGSGNTAVIQSSAFFPTPSGSIVTSESQLFNKPYWLQRAQGHNN GICWGNQLFVTVVDTTRSTNMTLCTEVTKEGTYKNDNFKEYVRHVEEYDLQF VFQLCKITLTAEIMTYIHTMDSNILEDWQFGLTPPPSASLQDTYRFVTSQAITCQ KTAPPKEKEDPLNKYTFWEVNLKEKFSADLDQFPLGRKFLLQSGL |
| SEQ ID No: 148 | 5802 The amino acid sequence of HPV type 33 L1 protein aa 474-499 |
| | KAKPKLKRAAPTSTRTSSAKRKKVKK |
| SEQ ID No: 149 | 5803 Amino acid sequence of chimeric HPV type 58 L1 protein |
| | MSVWRPSEATVYLPPVPVSKVVSTDEYVSRTSIYYYAGSSRLLAVGNPYFSIKS PNNNKKVLVPKVSGLQYRVFRVRLPDPNKFGFPDTSFYNPDTQRLVWACVGLE IGRGQPLGVGVSGHPYLNKFDDTETSNRYPAQPGSDNRECLSMDYKQTQLCLI GCKPPTGEHWGKGVACNNNAAATDCPPLELFNSIIEDGDMVDTGFGCMDFGTL QANKSDVPIDICNSTCKYPDYLKMASEPYGDSLFFFLRREQMFVRHFFNRAGKL GEAVPDDLYIKGSGNTAVIQSSAFFPTPSGSIVTSESQLFNKPYWLQRAQGHNN GICWGNQLFVTVVDTTRSTNMTLCTEVTKEGTYKNDNFKEYVRHVEEYDLQF VFQLCKITLTAEIMTYIHTMDSNILEDWQFGLTPPPSASLQDTYRFVTSQAITCQ KTAPPKEKEDPLNKYTFWEVNLKEKFSADLDQFPLGRKFLLQSGLKAKPKLKR AAPTSTRTSSAKRKKVKK |
| SEQ ID No: 150 | 5804 Nucleotide sequence of chimeric HPV type 58 L1 protein |
| | ATGAGCGTGTGGAGGCCCAGCGAGGCCACCGTGTACCTGCCCCCCGTGCCCGTGAGCAAGGTGGTGAGCAC CGACGAGTACGTGAGCAGGACCAGCATCTACTACTACGCCGGCAGCAGCAGGCTGCTGGCCGTGGGCAAC CCCTACTTCAGCATCAAGAGCCCCAACAACAACAAGAAGGTGCTGGTGCCCAAGGTGAGCGGCCTGCAGTA CAGGGTGTTCAGGGTGAGGCTGCCCGACCCCAACAAGTTCGGCTTCCCCGACACCAGCTTCTACAACCCCG ACACCCAGAGGCTGGTGTGGGCCTGCGTGGGCCTGGAGATCGGCAGGGGCCAGCCCCTGGGCGTGGGCGT GAGCGGCCACCCCTACCTGAACAAGTTCGACGACACCGAGACCAGCAACAGGTACCCCGCCCAGCCCGGC AGCGACAACAGGGAGTGCCTGAGCATGGACTACAAGCAGACCCAGCTGTGCCTGATCGGCTGCAAGCCCC CCACCGGCGAGCACTGGGGCAAGGGCGTGGCCTGCAACAACAACGCCGCCGCCACCGACTGCCCCCCCCTG GAGCTGTTCAACAGCATCATCGAGGACGGCGACATGGTGGACACCGGCTTCGGCTGCATGGACTTCGGCAC CCTGCAGGCCAACAAGAGCGACGTGCCCATCGACATCTGCAACAGCACCTGCAAGTACCCCGACTACCTGA AGATGGCCAGCGAGCCCTACGGCGACAGCCTGTTCTTCTTCCTGAGGAGGGAGCAGATGTTCGTGAGGCAC TTCTTCAACAGGGCCGGCAAGCTGGGCGAGGCCGTGCCCGACGACCTGTACATCAAGGGCAGCGGCAACA CCGCCGTGATCCAGAGCAGCGCCTTCTTCCCCACCCCCAGCGGCAGCATCGTGACCAGCGAGAGCCAGCTG TTCAACAAGCCCTACTGGCTGCAGAGGGCCCAGGGCCACAACAACGGCATCTGCTGGGGCAACCAGCTGTT CGTGACCGTGGTGGACACCACCAGGAGCACCAACATGACCCTGTGCACCGAGGTGACCAAGGAGGGCACC TACAAGAACGACAACTTCAAGGAGTACGTGAGGCACGTGGAGGAGTACGACCTGCAGTTCGTGTTCCAGCT GTGCAAGATCACCCTGACCGCCGAGATCATGACCTACATCCACACCATGGACAGCAACATCCTGGAGGACT GGCAGTTCGGCCTGACCCCCCCCCCCAGCGCCAGCCTGCAGGACACCTACAGGTTCGTGACCAGCCAGGCC ATCACCTGCCAGAAGACCGCCCCCCCCAAGGAGAAGGAGGACCCCCTGAACAAGTACACCTTCTGGGAGG TGAACCTGAAGGAGAAGTTCAGCGCCGACCTGGACCAGTTCCCCCTGGGCAGGAAGTTCCTGCTGCAGAGC GGCCTGAAAGCCAAGCCAAAACTGAAAAGGGCTGCCCCAACCAGCACCAGGACCTCCTCTGCCAAGAGGA AGAAGGTGAAGAAGTAAA |
| SEQ ID No: 151 | 5805 Synthetic HPV58 L1 gene |
| | ctgggtaccATGAGCGTGTGGAGGCCCAGCGAGGCCACCGTGTACCTGCCCCCCGTGCCCGTGAGCAAGGTGGT GAGCACCGACGAGTACGTGAGCAGGACCAGCATCTACTACTACGCCGGCAGCAGCAGGCTGCTGGCCGTG GGCAACCCCTACTTCAGCATCAAGAGCCCCAACAACAACAAGAAGGTGCTGGTGCCCAAGGTGAGCGGCC TGCAGTACAGGGTGTTCAGGGTGAGGCTGCCCGACCCCAACAAGTTCGGCTTCCCCGACACCAGCTTCTAC AACCCCGACACCCAGAGGCTGGTGTGGGCCTGCGTGGGCCTGGAGATCGGCAGGGGCCAGCCCCTGGGCG TGGGCGTGAGCGGCCACCCCTACCTGAACAAGTTCGACGACACCGAGACCAGCAACAGGTACCCCGCCCA GCCCGGCAGCGACAACAGGGAGTGCCTGAGCATGGACTACAAGCAGACCCAGCTGTGCCTGATCGGCTGC AAGCCCCCCACCGGCGAGCACTGGGGCAAGGGCGTGGCCTGCAACAACAACGCCGCCGCCACCGACTGCC CCCCCCTGGAGCTGTTCAACAGCATCATCGAGGACGGCGACATGGTGGACACCGGCTTCGGCTGCATGGAC TTCGGCACCCTGCAGGCCAACAAGAGCGACGTGCCCATCGACATCTGCAACAGCACCTGCAAGTACCCCGA CTACCTGAAGATGGCCAGCGAGCCCTACGGCGACAGCCTGTTCTTCTTCCTGAGGAGGGAGCAGATGTTCG TGAGGCACTTCTTCAACAGGGCCGGCAAGCTGGGCGAGGCCGTGCCCGACGACCTGTACATCAAGGGCAGC GGCAACACCGCCGTGATCCAGAGCAGCGCCTTCTTCCCCACCCCCAGCGGCAGCATCGTGACCAGCGAGAG CCAGCTGTTCAACAAGCCCTACTGGCTGCAGAGGGCCCAGGGCCACAACAACGGCATCTGCTGGGGCAACC AGCTGTTCGTGACCGTGGTGGACACCACCAGGAGCACCAACATGACCCTGTGCACCGAGGTGACCAAGGA GGGCACCTACAAGAACGACAACTTCAAGGAGTACGTGAGGCACGTGGAGGAGTACGACCTGCAGTTCGTG TTCCAGCTGTGCAAGATCACCCTGACCGCCGAGATCATGACCTACATCCACACCATGGACAGCAACATCCT GGAGGACTGGCAGTTCGGCCTGACCCCCCCCCCCAGCGCCAGCCTGCAGGACACCTACAGGTTCGTGACCA GCCAGGCCATCACCTGCCAGAAGACCGCCCCCCCCAAGGAGAAGGAGGACCCCCTGAACAAGTACACCTT CTGGGAGGTGAACCTGAAGGAGAAGTTCAGCGCCGACCTGGACCAGTTCCCCCTGGGCAGGAAGTTCCTGC TGCAGAGCGGCCTGAAGGCCAAGCCCAGGCTGAAGAGGAGCGCCCCCACCACCAGGGCCCCCAGCACCAA GAGGAAGAAGGTGAAGAAGTAAA ctcgagctc |
| SEQ ID No: 152 | 5806 Synthetic HPV33 L1 gene |
| | ctgggtaccATGAGTGTGTGGAGACCATCTGAGGCTACAGTCTACCTGCCTCCTGTGCCTGTGAGCAAGGTGGTG AGCACAGATGAATATGTGAGCAGGACCAGCATCTACTACTATGCTGGCTCCAGCAGACTGCTGGCTGTGGG ACACCCATACTTCAGCATCAAGAACCCAACCAATGCCAAGAAACTGCTGGTGCCAAAGGTGTCTGGACTCC AATACAGGGTGTTCAGGGTGAGACTGCCTGACCCAAACAAGTTTGGCTTTCCTGACACCTCCTTCTACAACC CTGACACCCAGAGACTGGTGTGGGCTTGTGTGGGATTGGAGATTGGCAGGGGACAACCACTGGGAGTGGG CATCTCTGGACACCCACTGCTGAACAAGTTTGATGACACAGAGACAGGCAACAAATACCCTGGACAACCTG GAGCAGACAACAGGGAGTGTCTGAGTATGGACTACAAGCAGACCCAACTTTGTCTGCTGGGCTGTAAGCCT CCAACAGGAGAACACTGGGGCAAGGGAGTGGCTTGTACCAATGCTGCCCCTGCCAATGACTGTCCTCCATT GGAACTGATAAACACCATCATTGAGGATGGAGATATGGTGGACACAGGCTTTGGCTGTATGGACTTCAAGA CCCTCCAAGCCAACAAGTCTGATGTGCCAATTGACATCTGTGGCAGCACTTGTAAATACCCTGACTACCTGA AAATGACCTCTGAACCATATGGAGACTCCCTGTTCTTCTTCCTGAGGAGGGAACAGATGTTTGTGAGACACT TCTTCAACAGGGCTGGCACCCTGGGAGAGGCTGTGCCTGATGACCTCTACATCAAGGGCTCTGGCACCACA GCCAGCATCCAGTCCTCTGCCTTCTTTCCAACACCATCTGGCAGTATGGTGACCTCTGAGAGCCAACTTTTC AACAAGCCATACTGGCTCCAAAGGGCTCAAGGACACAACAATGGCATCTGTTGGGGCAACCAGGTGTTTGT GACAGTGGTGGACACCACCAGGAGCACCAATATGACCCTGTGTACCCAGGTGACCTCTGACAGCACCTACA AGAATGAGAACTTCAAGGAATACATCAGGCATGTGGAGGAATATGACCTCCAATTTGTGTTCCAACTTTGT AAGGTGACCCTGACAGCAGAGGTGATGACCTACATCCATGCTATGAACCCTGACATCTTGGAGGACTGGCA GTTTGGACTGACACCTCCTCCATCTGCCTCCCTCCAAGACACCTACAGGTTTGTGACCAGCCAGGCTATCAC TTGTCAGAAGACAGTGCCTCCAAAGGAGAAGGAGGACCCACTGGGCAAATACACCTTCTGGGAGGTGGAC CTGAAAGAGAAGTTCTCTGCTGACCTGGACCAGTTTCCACTGGGCAGGAAGTTCCTGCTCCAAGCAGGACT GAAAGCCAAGCCAAAACTGAAAAGGGCTGCCCCAACCAGCACCAGGACCTCCTCTGCCAAGAGGAAGAAG GTGAAGAAGTAAActcgagctc |
| SEQ ID No: 153 | 5807HPV58 L1 F1 |
| | CTTGGTACCATGAGCGTGTGGAGGCCCAGCGAGG |
| SEQ ID No: 154 | 5808HPV58 L1 R1 |
| | GCTTGGCTTTCAGGCCGCTCTGCAGCAGGAACTTCC |
| SEQ ID No: 155 | 5809 HPV58 L1 amplified sequence 1 |
| | CTTGGTACC[1] ATGAGCGTGTGGAGGCCCAGCGAGGCCACCGTGTACCTGCCCCCCGTGCCCGTGAGCAAGGTGGTGAGCAC CGACGAGTACGTGAGCAGGACCAGCATCTACTACTACGCCGGCAGCAGCAGGCTGCTGGCCGTGGGCAAC CCCTACTTCAGCATCAAGAGCCCCAACAACAACAAGAAGGTGCTGGTGCCCAAGGTGAGCGGCCTGCAGTA CAGGGTGTTCAGGGTGAGGCTGCCCGACCCCAACAAGTTCGGCTTCCCCGACACCAGCTTCTACAACCCCG ACACCCAGAGGCTGGTGTGGGCCTGCGTGGGCCTGGAGATCGGCAGGGGCCAGCCCCTGGGCGTGGGCGT GAGCGGCCACCCCTACCTGAACAAGTTCGACGACACCGAGACCAGCAACAGGTACCCCGCCCAGCCCGGC AGCGACAACAGGGAGTGCCTGAGCATGGACTACAAGCAGACCCAGCTGTGCCTGATCGGCTGCAAGCCCC CCACCGGCGAGCACTGGGGCAAGGGCGTGGCCTGCAACAACAACGCCGCCGCCACCGACTGCCCCCCCCTG GAGCTGTTCAACAGCATCATCGAGGACGGCGACATGGTGGACACCGGCTTCGGCTGCATGGACTTCGGCAC CCTGCAGGCCAACAAGAGCGACGTGCCCATCGACATCTGCAACAGCACCTGCAAGTACCCCGACTACCTGA AGATGGCCAGCGAGCCCTACGGCGACAGCCTGTTCTTCTTCCTGAGGAGGGAGCAGATGTTCGTGAGGCAC TTCTTCAACAGGGCCGGCAAGCTGGGCGAGGCCGTGCCCGACGACCTGTACATCAAGGGCAGCGGCAACA CCGCCGTGATCCAGAGCAGCGCCTTCTTCCCCACCCCCAGCGGCAGCATCGTGACCAGCGAGAGCCAGCTG TTCAACAAGCCCTACTGGCTGCAGAGGGCCCAGGGCCACAACAACGGCATCTGCTGGGGCAACCAGCTGTT CGTGACCGTGGTGGACACCACCAGGAGCACCAACATGACCCTGTGCACCGAGGTGACCAAGGAGGGCACC TACAAGAACGACAACTTCAAGGAGTACGTGAGGCACGTGGAGGAGTACGACCTGCAGTTCGTGTTCCAGCT GTGCAAGATCACCCTGACCGCCGAGATCATGACCTACATCCACACCATGGACAGCAACATCCTGGAGGACT GGCAGTTCGGCCTGACCCCCCCCCCCAGCGCCAGCCTGCAGGACACCTACAGGTTCGTGACCAGCCAGGCC ATCACCTGCCAGAAGACCGCCCCCCCCAAGGAGAAGGAGGACCCCCTGAACAAGTACACCTTCTGGGAGG TGAACCTGAAGGAGAAGTTCAGCGCCGACCTGGACCAGTTCCCCCTGGGCAGGAAGTTCCTGCTGCAGAGC GGCCTG [2]AAAGCCAAGC[3] |
| | [1]: Kpn I enzymatic cleavage site; |
| | [2]: the gene fragment encoding of HPV58 L1 aa 1-473; |
| | [3]: 10 bases overlapping with the gene fragment encoding HPV33L1 aa 474-499. |
| SEQ ID No: 156 | 5810HPV58 L1 F2 |
| | GAGCGGCCTGAAAGCCAAGCCAAAACTGAAAAGGG |
| SEQ ID No: 157 | 5811HPV58 L1 R2 |
| | CTGTCTAGATTTACTTCTTCACCTTCTTCCTCTTGG |
| SEQ ID No: 158 | 5812 HPV58 L1 amplified sequence 2 |
| | GAGCGGCCTG[1] AAAGCCAAGCCAAAACTGAAAAGGGCTGCCCCAACCAGCACCAGG ACCTCCTCTGCCAAGAGGAAGAAGGTGAAGAAG[2] TAA ATCTAGA[3]CAG. |
| | [1]: 10bp overlapping with the gene fragment encoding the C-terminus of HPV58 L1 aa 1-473; |
| | [2]: the gene fragment encoding the C-terminal 26 amino acids of HPV33L1 aa 474-499; |
| | [3]: Xbal enzymatic cleavage site |
| SEQ ID No: 159 | 5813 The amino acid sequence of HPV type 59 L1 protein aa 471-508 |
| | LQLGARPKPTIGPRKRAAPAPTSTPSPKRVKRRKSSRK |

### Appendix 14: SEQUENCE LISTING - HUMAN PAPILLOMAVIRUS TYPE 59 L1 PROTEIN

| | |
|---|---|
| SEQ ID No: 160 | 5901 The amino acid sequence of HPV type 59 L1 protein |
| | MALWRSSDNKVYLPPPSVAKVVSTDEYVTRTSIFYHAGSSRLLTVGHPYFKVP KGGNGRQDVPKVSAYQYRVFRVKLPDPNKFGLPDNTVYDPNSQRLVWACVG VEIGRGQPLGVGLSGHPLYNKLDDTENSHVASAVDTKDTRDNVSVDYKQTQL CIIGCVPAIGEHWTKGTACKPTTVVQGDCPPLELINTPIEDGDMVDTGYGAMDF KLLQDNKSEVPLDICQSICKYPDYLQMSADAYGDSMFFCLRREQVFARHFWNR SGTMGDQLPESLYIKGTDIRANPGSYLYSPSPSGSVVTSDSQLFNKPYWLHKAQ GLNNGICWHNQLFLTVVDTTRSTNLSVCASTTSSIPNVYTPTSFKEYARHVEEF DLQFIFQLCKITLTTEVMSYIHNMNTTILEDWNFGVTPPPTASLVDTYRFVQSAA VTCQKDTAPPVKQDPYDKLKFWPVDLKERFSADLDQFPLGRKFLLQLGARPKP TIGPRKRAAPAPTSTPSPKRVKRRKSSRK |
| SEQ ID No: 161 | 5902 Nucleotide sequence of HPV type 59 L1 protein |
| | ATGGCTCTGTGGAGGTCCTCTGACAACAAGGTCTACCTGCCTCCTCCATCTGTGGCTAAGGTGGTGAGCACA GATGAATATGTGACCAGGACCAGCATCTTCTACCATGCTGGCTCCAGCAGACTGCTGACAGTGGGACACCC ATACTTCAAGGTGCCAAAGGGAGGCAATGGCAGACAGGATGTGCCAAAGGTGTCTGCCTACCAATACAGG GTGTTCAGGGTGAAACTGCCTGACCCAAACAAGTTTGGACTGCCTGACAACACAGTCTATGACCCAAACAG CCAGAGACTGGTGTGGGCTTGTGTGGGAGTGGAGATTGGCAGGGGACAACCACTGGGAGTGGGACTGTCT GGACACCCACTCTACAACAAACTGGATGACACAGAGAACTCTCATGTGGCATCTGCTGTGGACACCAAGGA CACCAGGGACAATGTGTCTGTGGACTACAAGCAGACCCAACTTTGTATCATTGGCTGTGTGCCTGCCATTGG AGAACACTGGACCAAGGGCACAGCCTGTAAGCCAACCACAGTGGTCCAGGGAGACTGTCCTCCATTGGAA CTGATAAACACACCAATTGAGGATGGAGATATGGTGGACACAGGCTATGGAGCTATGGACTTCAAACTGCT CCAAGACAACAAGTCTGAGGTGCCACTGGACATCTGTCAGAGCATCTGTAAATACCCTGACTACCTCCAAA TGAGTGCTGATGCCTATGGAGACAGTATGTTCTTCTGTCTGAGGAGGGAACAGGTGTTTGCCAGACACTTCT GGAACAGGTCTGGCACAATGGGAGACCAACTTCCTGAGTCCCTCTACATCAAGGGCACAGACATCAGGGCT AACCCTGGCTCCTACCTCTACAGCCCAAGCCCATCTGGCTCTGTGGTGACCTCTGACAGCCAACTTTTCAAC AAGCCATACTGGCTGCACAAGGCTCAAGGACTGAACAATGGCATCTGTTGGCACAACCAACTTTTCCTGAC AGTGGTGGACACCACCAGGAGCACCAACCTGTCTGTGTGTGCCAGCACCACCTCCAGCATCCCAAATGTCT ACACACCAACCTCCTTCAAGGAATATGCCAGGCATGTGGAGGAGTTTGACCTCCAATTCATCTTCCAACTTT GTAAGATTACCCTGACCACAGAGGTGATGAGTTACATCCACAATATGAACACCACCATCTTGGAGGACTGG AACTTTGGAGTGACACCTCCTCCAACAGCCTCCCTGGTGGACACCTACAGGTTTGTCCAGTCTGCTGCTGTG ACTTGTCAGAAGGACACAGCCCCTCCTGTGAAGCAGGACCCATATGACAAACTGAAGTTCTGGCCTGTGGA CCTGAAAGAGAGGTTCTCTGCTGACCTGGACCAGTTTCCACTGGGCAGGAAGTTCCTGCTCCAACTTGGAG CCAGACCAAAGCCAACCATTGGACCAAGGAAGAGGGCTGCCCCTGCCCCAACCAGCACACCAAGCCCAAA GAGGGTGAAGAGGAGGAAGTCCAGCAGGAAGTAAA |
| SEQ ID No: 162 | 5903 Synthetic HPV59 L1 gene |
| | ctgggtaccATGGCTCTGTGGAGGTCCTCTGACAACAAGGTCTACCTGCCTCCTCCATCTGTGGCTAAGGTGGTG AGCACAGATGAATATGTGACCAGGACCAGCATCTTCTACCATGCTGGCTCCAGCAGACTGCTGACAGTGGG ACACCCATACTTCAAGGTGCCAAAGGGAGGCAATGGCAGACAGGATGTGCCAAAGGTGTCTGCCTACCAAT ACAGGGTGTTCAGGGTGAAACTGCCTGACCCAAACAAGTTTGGACTGCCTGACAACACAGTCTATGACCCA AACAGCCAGAGACTGGTGTGGGCTTGTGTGGGAGTGGAGATTGGCAGGGGACAACCACTGGGAGTGGGAC TGTCTGGACACCCACTCTACAACAAACTGGATGACACAGAGAACTCTCATGTGGCATCTGCTGTGGACACC AAGGACACCAGGGACAATGTGTCTGTGGACTACAAGCAGACCCAACTTTGTATCATTGGCTGTGTGCCTGC CATTGGAGAACACTGGACCAAGGGCACAGCCTGTAAGCCAACCACAGTGGTCCAGGGAGACTGTCCTCCAT TGGAACTGATAAACACACCAATTGAGGATGGAGATATGGTGGACACAGGCTATGGAGCTATGGACTTCAA ACTGCTCCAAGACAACAAGTCTGAGGTGCCACTGGACATCTGTCAGAGCATCTGTAAATACCCTGACTACC TCCAAATGAGTGCTGATGCCTATGGAGACAGTATGTTCTTCTGTCTGAGGAGGGAACAGGTGTTTGCCAGA CACTTCTGGAACAGGTCTGGCACAATGGGAGACCAACTTCCTGAGTCCCTCTACATCAAGGGCACAGACAT CAGGGCTAACCCTGGCTCCTACCTCTACAGCCCAAGCCCATCTGGCTCTGTGGTGACCTCTGACAGCCAACT TTTCAACAAGCCATACTGGCTGCACAAGGCTCAAGGACTGAACAATGGCATCTGTTGGCACAACCAACTTT TCCTGACAGTGGTGGACACCACCAGGAGCACCAACCTGTCTGTGTGTGCCAGCACCACCTCCAGCATCCCA AATGTCTACACACCAACCTCCTTCAAGGAATATGCCAGGCATGTGGAGGAGTTTGACCTCCAATTCATCTTC CAACTTTGTAAGATTACCCTGACCACAGAGGTGATGAGTTACATCCACAATATGAACACCACCATCTTGGA GGACTGGAACTTTGGAGTGACACCTCCTCCAACAGCCTCCCTGGTGGACACCTACAGGTTTGTCCAGTCTGC TGCTGTGACTTGTCAGAAGGACACAGCCCCTCCTGTGAAGCAGGACCCATATGACAAACTGAAGTTCTGGC CTGTGGACCTGAAAGAGAGGTTCTCTGCTGACCTGGACCAGTTTCCACTGGGCAGGAAGTTCCTGCTCCAA CTTGGAGCCAGACCAAAGCCAACCATTGGACCAAGGAAGAGGGCTGCCCCTGCCCCAACCAGCACACCAA GCCCAAAGAGGGTGAAGAGGAGGAAGTCCAGCAGGAAGTAAActcgagctc |

## Claims

1. A HPV immunogenic composition for use in preventing HPV-related diseases or infections, comprising:
chimeric HPV L1 protein of HPV types 6, 11, 16, 18, 31, 35, 39, 45, 51, 52, 56 and 58, HPV Type 33 L1 protein and HPV Type 59 L1 protein,
wherein said chimeric HPV L1 protein of HPV Types 6, 11, 16, 18, 31, 35, 39, 45, 51, 52, 56 and 58 is shown in SEQ ID No: 3, SEQ ID No: 16, SEQ ID No: 29, SEQ ID No: 42, SEQ ID No: 55, SEQ ID No: 71, SEQ ID No: 84, SEQ ID No: 97, SEQ ID No: 110, SEQ ID No: 123, SEQ ID No: 136 and SEQ ID No: 149, respectively, and
the HPV Type 33 L1 protein and HPV Type 59 L1 protein is shown in SEQ ID No: 66 and SEQ ID No: 160, respectively.

2. The HPV immunogenic composition for use according to claim 1,
wherein said chimeric HPV L1 protein of HPV Types 6 is encoded by SEQ ID No: 4,
wherein said chimeric HPV L1 protein of HPV Types 11 is encoded by SEQ ID No: 17,
wherein said chimeric HPV L1 protein of HPV Types 16 is encoded by SEQ ID No: 30,
wherein said chimeric HPV L1 protein of HPV Types 18 is encoded by SEQ ID No: 43,
wherein said chimeric HPV L1 protein of HPV Types 31 is encoded by SEQ ID No: 56,
wherein said chimeric HPV L1 protein of HPV Types 35 is encoded by SEQ ID No: 72,
wherein said chimeric HPV L1 protein of HPV Types 39 is encoded by SEQ ID No: 85,
wherein said chimeric HPV L1 protein of HPV Types 45 is encoded by SEQ ID No: 98,
wherein said chimeric HPV L1 protein of HPV Types 51 is encoded by SEQ ID No: 111,
wherein said chimeric HPV L1 protein of HPV Types 52 is encoded by SEQ ID No: 124,
wherein said chimeric HPV L1 protein of HPV Types 56 is encoded by SEQ ID No: 137,
wherein said chimeric HPV L1 protein of HPV Types 58 is encoded by SEQ ID No: 150,
wherein said HPV L1 protein of HPV Types 33 is encoded by SEQ ID No: 67, or
wherein said HPV L1 protein of HPV Types 59 is encoded by SEQ ID No: 161.

3. The HPV immunogenic composition for use according to claim 1 or 2, wherein said HPV immunogenic composition further comprises a physiologically acceptable carrier and, optionally, an adjuvant.

4. The HPV immunogenic composition for use according to claim 3, wherein said adjuvant is an aluminum phosphate adjuvant.

## Patentansprüche

1. HPV-immunogene Zusammensetzung zur Verwendung bei der Prävention von HPVbezogenen Krankheiten oder Infektionen, umfassend:
chimäres HPV-L1-Protein der HPV-Typen 6, 11, 16, 18, 31, 35, 39, 45, 51, 52, 56 und 58, HPV-L1-Protein Typ 33 und HPV-L1-Protein Typ 59,
wobei das chimäre HPV-L1-Protein der HPV-Typen 6, 11, 16, 18, 31, 35, 39, 45, 51, 52, 56 und 58 in SEQ ID NO: 3, SEQ ID NO: 16, SEQ ID NO: 29, SEQ ID NO: 42, SEQ ID NO: 55, SEQ ID NO: 71, SEQ ID NO: 84, SEQ ID NO: 97, SEQ ID NO: 110, SEQ ID NO: 123, SEQ ID NO: 136 bzw. SEQ ID NO: 149 gezeigt ist, und
das HPV-L1-Protein Typ 33 und das HPV-L1-Protein Typ 59 in SEQ ID NO: 66 bzw. SEQ ID NO: 160 gezeigt sind.

2. HPV-immunogene Zusammensetzung zur Verwendung nach Anspruch 1, wobei das chimäre HPV-L1-Protein der HPV-Typen 6 durch SEQ ID NO: 4 kodiert ist,
wobei das chimäre HPV-L1-Protein der HPV-Typen 11 durch SEQ ID NO: 17 kodiert ist,
wobei das chimäre HPV-L1-Protein der HPV-Typen 16 durch SEQ ID NO: 30 kodiert ist,
wobei das chimäre HPV-L1-Protein der HPV-Typen 18 durch SEQ ID NO: 43 kodiert ist,
wobei das chimäre HPV-L1-Protein der HPV-Typen 31 durch SEQ ID NO: 56 kodiert ist,
wobei das chimäre HPV-L1-Protein der HPV-Typen 35 durch SEQ ID NO: 72 kodiert ist,
wobei das chimäre HPV-L1-Protein der HPV-Typen 39 durch SEQ ID NO: 85 kodiert ist,
wobei das chimäre HPV-L1-Protein der HPV-Typen 45 durch SEQ ID NO: 98 kodiert ist,
wobei das chimäre HPV-L1-Protein der HPV-Typen 51 durch SEQ ID NO: 111 kodiert ist,
wobei das chimäre HPV-L1-Protein der HPV-Typen 52 durch SEQ ID NO: 124 kodiert ist,
wobei das chimäre HPV-L1-Protein der HPV-Typen 56 durch SEQ ID NO: 137 kodiert ist,
wobei das chimäre HPV-L1-Protein der HPV-Typen 58 durch SEQ ID NO: 150 kodiert ist,
wobei das HPV-L1-Protein der HPV-Typen 33 durch SEQ ID NO: 67 codiert wird, oder
wobei das HPV-L1-Protein der HPV-Typen 59 durch SEQ ID NO: 161 kodiert ist.

3. HPV-immunogene Zusammensetzung zur Verwendung nach Anspruch 1 oder 2, wobei die HPV-immunogene Zusammensetzung ferner einen physiologisch annehmbaren Träger und optional ein Adjuvans umfasst.

4. HPV-immunogene Zusammensetzung zur Verwendung nach Anspruch 3, wobei das Adjuvans ein Aluminiumphosphat-Adjuvans ist.

## Revendications

1. Composition immunogène de HPV destinée à être utilisée dans la prévention des maladies ou infections associées au HPV, comprenant :
une protéine L1 HPV chimère de HPV de type 6, 11, 16, 18, 31, 35, 39, 45, 51, 52, 56 et 58, une protéine L1 de HPV de type 33 et une protéine L1 de HPV de type 59,
ladite protéine L1 HPV chimère de HPV de type 6, 11, 16, 18, 31, 35, 39, 45, 51, 52, 56 et 58 étant représenté dans SEQ ID n° : 3, SEQ ID n° : 16, SEQ ID n° : 29, SEQ ID n° : 42, SEQ ID n° : 55, SEQ ID n° : 71, SEQ ID n° : 84, SEQ ID n° : 97, SEQ ID n° : 110, SEQ ID n° : 123, SEQ ID n° : 136 et SEQ ID n° : 149, respectivement, et
ladite protéine L1 de HPV de type 33 et ladite protéine L1 de HPV de type 59 étant représentée dans SEQ ID n° : 66 et SEQ ID n° : 160, respectivement.

2. Composition immunogène de HPV destinée à être utilisée selon la revendication 1,
ladite protéine L1 HPV chimère de HPV de type 6 étant codée par SEQ ID n° : 4,
ladite protéine L1 HPV chimère de HPV de type 11 étant codée par SEQ ID n° : 17,
ladite protéine L1 HPV chimère de HPV de type 16 étant codée par SEQ ID n° : 30,
ladite protéine L1 HPV chimère de HPV de type 18 étant codée par SEQ ID n° : 43,
ladite protéine L1 HPV chimère de HPV de type 31 étant codée par SEQ ID n° : 56,
ladite protéine L1 HPV chimère de HPV de type 35 étant codée par SEQ ID n° : 72,
ladite protéine L1 HPV chimère de HPV de type 39 étant codée par SEQ ID n° : 85,
ladite protéine L1 HPV chimère de HPV de type 45 étant codée par SEQ ID n° : 98,
ladite protéine L1 HPV chimère de HPV de type 51 étant codée par SEQ ID n° : 111,
ladite protéine L1 HPV chimère de HPV de type 52 étant codée par SEQ ID n° : 124,
ladite protéine L1 HPV chimère de HPV de type 56 étant codée par SEQ ID n° : 137,
ladite protéine L1 HPV chimère de HPV de type 58 étant codée par SEQ ID n° : 150,
ladite protéine L1 HPV chimère de HPV de type 33 étant codée par SEQ ID n° : 67, ou
ladite protéine L1 HPV chimère de HPV de type 59 étant codée par SEQ ID n° : 161.

3. Composition immunogène de HPV destinée à être utilisée selon la revendication 1 ou 2, ladite composition immunogène de HPV comprenant en outre un vecteur physiologiquement acceptable et, éventuellement, un adjuvant.

4. Composition immunogène de HPV destinée à être utilisée selon la revendication 3, ledit adjuvant étant un adjuvant de phosphate d'aluminium.
